# EUROPEAN PATENT SPECIFICATION

(11) **EP 3 087 187 B1**
(45) Date of publication and mention of the grant of the patent: **11.12.2024**
(21) Application number: 14827776.7
(22) Date of filing: 23.12.2014
(51) Int. Cl.: C12N 15/86

(54) **A METHOD OF MAKING ADENOVIRUS AND CORRESPONDING PLASMIDS**
VERFAHREN ZUR HERSTELLUNG VON ADENOVIRUS UND KORRESPONDIERENDE PLASMIDE
PROCÉDÉS DE FABRICATION D'ADÉNOVIRUS ET PLASMIDES CORRESPONDANTS

(30) Priority: 23.12.2013 GB 201322851; 24.10.2014 WO PCT/EP2014/072919
(43) Date of publication of application: 02.11.2016
(73) Proprietor: Akamis Bio Limited, Abingdon, Oxfordshire OX14 3YS (GB)
(72) Inventor: BROWN, Alice Claire Noel, Abingdon Oxfordshire OX14 4SD (GB); NICOLSON, Tamara, London EC1A 9JR (GB)
(74) Representative: Simmons & Simmons LLP (Munich)
(86) International application number: PCT/EP2014/079162
(87) International publication number: WO 2015/097220

(56) References cited:
- WO-A2-2006/060314
- WO-A2-2012/024351
- JOSEPHINE M. JANSSEN ET AL: "Development of an AdEasy-based system to produce first- and second-generation adenoviral vectors with tropism for CAR- or CD46-positive cells", THE JOURNAL OF GENE MEDICINE, vol. 15, no. 1, 1 January 2013 (2013-01-01), pages 1 - 11, XP055184188, ISSN: 1099-498X, DOI: 10.1002/jgm.2687
- HOFFMANN DENNIS ET AL: "Efficient generation of double heterologous promoter controlled oncolytic adenovirus vectors by a single homologous recombination step in Escherichia coli", BMC BIOTECHNOLOGY, vol. 6, no. 36, 3 August 2006 (2006-08-03), pages 1 - 12, XP021017063, ISSN: 1472-6750, DOI: 10.1186/1472-6750-6-36
- DATABASE WPI Week 201267, Derwent World Patents Index; AN 2012-L80326, XP002738783
- LI ET AL.: "A one-step ligation system for rapid generation of armed, conditionally-replicating adenoviruses.", BIOTECHNOLOGY LETTERS, vol. 35, August 2013 (2013-08-01), pages 1215 - 1221, XP002738784
- D. MCVEY ET AL: "Rapid Construction of Adenoviral Vectors by Lambda Phage Genetics", JOURNAL OF VIROLOGY, vol. 76, no. 8, 15 April 2002 (2002-04-15), pages 3670 - 3677, XP055184177, ISSN: 0022-538X, DOI: 10.1128/JVI.76.8.3670-3677.2002

## Description

The present disclosure relates to a method of making an adenovirus plasmid comprising a part or all of an adenovirus genome and one or more original restriction sites allowing rapid and flexible manipulation of the adenovirus genome, and methods of preparing adenovirus constructs, for example comprising a transgene. The disclosure also extends to plasmids prepared bythe method.

### BACKGROUND

It is desirable to insert transgenes into adenoviruses for many reasons, for example, to arm therapeutic viruses to increase therapeutic impact or to deliver genes to target cells using a replication competent virus or a replication deficient viral vector.

Typically, to insert a transgene into a virus genome, a plasmid is generated comprising the adenoviral genome, the transgene is then inserted into the plasmid, for example employing homologous recombination and then the viral genome is excised from the plasmid. However, for reasons described herein, flexible plasmids that can be used for both replication competent viruses and replication deficient viral vectors and, for exampe which can accept large transgenes in a known and predictable location are not always readily available, especially if the transgene is to be inserted in an unusual location, such as outside the E1 or E3 region.

The problems associated with inserting transgenes into adenoviruses for therapeutic and diagnostic purposes fall into 3 main categories. Firstly, not all adenoviruses are ideal candidates for therapeutic and diagnostic applications, for example, Ad5 (a subgroup C adenovirus) immunity is prevalent in the human population and consequently the virus is rapidly cleared by the immune system after it is administered. To overcome this problem, adenoviruses to which there is less prevalent immunity have been utilised. However, much of the genomic work to date has been on Ad5. Therefore, the materials and resources for alternative adenoviruses are often not available.

Secondly, not all adenoviruses can accept large transgenes and maintain their stability as a viable viruses. Furthermore, the adenovirus genome is large and there is little room to insert additional genetic material without affecting a function of the virus, for example the function of packaging the virus into the viral capsid may be adversely affected, which in turn is likely to impact the infectivity of the virus.

To overcome this problem deletions have been made to the genome. This strategy is particularly suitable for replication deficient viral vectors because one or more genes are removed which are essential to replication. This both limits the vector's ability to replicate *in vivo* and creates space in the genome thereby allowing insertion of large transgenes. These transgenes can be expressed *in vivo,* regardless of the viral vector's inability to replicate. Most frequently the E1 gene has been deleted. Prior art systems, such as the ADEASY system (Agilent Technologies), allow insertion of transgenes to the E1 region. In some instances part, or all, of the E3 region is deleted, see for example WO2011/0123564 and the gene may be inserted in the region deleted.

Thus, typically the transgene is inserted in the same position that the deletion occurred. The site of transgene insertion has largely been limited to the location of early genes which can be problematic because it is more likely to affect virus gene expression, virus life-cycle and/or speed of replication. In particular, deleting the E1 region is not appropriate for the replication competent adenoviruses and as discussed it may be useful to insert a transgene such that it is not in a location of an early gene to ensure that the impact on the virus life-cycle is minimised.

Thirdly, the adenovirus genome is not easy to manipulate because the genome is densely packed and has very little intergenic material where a transgene might be safely inserted without affecting the virus life-cycle and/or a function, such as transcription. Furthermore, there are few, if any, restriction sites in the intergenic regions and even fewer that only occur once in the genome. The latter is relevant because when a restriction site occurs more than once in the virus genome then the ability to selectively insert a transgene in one location employing that restriction site is severely impeded. Therefore, it is desirable to provide a plasmid that can be used to manipulate a replication competent virus and wherein transgenes may be inserted in a location removed from the early genes.

One strategy that can be utilised with replication competent viruses is to employ a non-biasedly inserting transposon to insert the transgene into the genome (as described in Jin *et al* 2004). The transposon may be inserted in the late genes and thus this technology does not suffer from the disadvantages of the systems described above. Jin *et al* hypothesised that the site of location of insertion of the gene is influenced by the type the gene being inserted and, whilst it was possible to replace some of the genes after insertion, in some instances this was difficult to replace the inserted gene or the replacement gene was inserted in a different orientation. The random nature of transposon insertion provides many possible insertion sites. Therefore, predictability and reproducibility of insertion may be compromised as a result. Furthermore, the transposon inserts itself into the genome along with the transgene and in theory could "move" the location of the gene in the virus genome at a later date. However, whilst the randomly inserting transposon is a wonderful tool for investigating the virus genome the biggest disadvantage of this approach is that it does not allow rational design of the virus construct.

Therefore, it is desirable to provide a plasmid that can be used to manipulate a replication competent virus and in which transgenes can reproducibly be inserted in a location removed from the early genes. The present inventors set out to overcome one or more of the problems described above by generating a plasmid with a combination of restriction sites that can be used to selectively insert a transgene specifically into a location that this not the site of an early gene.

The present inventors have developed adenovirus plasmids comprising original restriction sites in the vicinity of the L5 gene. The plasmids of the present disclosure allow generation of viruses with restriction sites/transgenes in locations other than the early gene sites, for example for replication competent adenoviruses with the E1 region intact or replication deficient adenoviruses, such as with E1 and/or E3 deleted or interrupted.

Li et al, Biotechnol Lett (2013), 35: 1215-1221 describes a one-step ligation system for rapid generation of armed, conditionally-replicating adenoviruses.

### SUMMARY OF INVENTION

The invention is as defined in claim 1.

In one embodiment the method comprises a further step c): performing homologous recombination between the shuttle vector of step a) or step b) and the adenovirus genome to form a plasmid.

In one embodiment there is provided a method of preparing an adenovirus plasmid as defined in claim 6.

In one embodiment step b) is preformed prior to step a).

In one embodiment step b) is performed after step a).

In one embodiment the 5' arm comprises about 2.4 to 4.7 kb of the 5' end of an adenovirus genome.

In one embodiment the 3' arm comprises about 3.3 to 4.8 kb of the 3' end of an adenovirus genome.

In one embodiment the period over which the one-step three-way ligation performed is at least 50 minutes, for example 1 hour or more, such as 2, 3, 4, 5, 6, 7, 8, 9, 10, 11, 12, 13, 14, 15, 16, 17, 18, 19, 20, 21, 22, 23 or 24 hours.

In one embodiment the one-step three-way ligation is performed in a temperature range of about 10 to 40°C, for example 20 to 25°C, such as at approximately room/ambient temperature.

In one embodiment the adenovirus is a human adenovirus, for example a type B subgroup, such as a virus selected from EnAd, OvAd1, OvAd2, Ad3, Ad5 (which is a group C virus) and Ad11. In one embodiment the adenovirus is not Ad5. In one embodiment the adenovirus is not a group A virus. In one embodiment the adenovirus is not a group C virus.

In one embodiment the adenovirus is replication capable or competent, such as replication competent. In one embodiment the adenovirus is not a conditionally replicating virus. In one embodiment the adenovirus is replication deficient.

In one embodiment the unique restriction site is independently selected from Fsel, Notl, Sbfl and Sgfl, such as Notl or Sbfl and Sgfl or Fsel and Notl and Sbfl and Sgfl.

In one embodiment the first restriction site in the **vector fragment** and the first restriction site in the **3'-arm** are the same. In one embodiment the second restriction site in the **vector fragment** and the second restriction site in the **5'-arm** are the same. In one embodiment the third restriction site in the **5'-arm** and the third restriction site in the **3'-arm** are the same.

In one embodiment the first and second restriction sites are the same.

In one embodiment the **vector fragment** is dephosphorylated prior to ligation.

In one embodiment the origin of replication is p15A.

In one embodiment the selection marker gene is KanR or AmpR, such as KanR.

In one embodiment step c) is performed at a ratio of 3.5 parts:1.5 parts, shuttle vector of step a) or b) to adenovirus genome respectively, for example in electrocompetent BJ5183 cells.

In one embodiment the method comprises the further step of inserting at least one transgene, for example in a location other than the location of an early gene, such as associated with the fibre L5. In one embodiment the transgene is in the form of cassette, for example that comprises a splice acceptor sequence.

In one embodiment the transgene is under the control of an endogenous adenovirus promoter, for example the major late promoter. In one embodiment a gene or genes placed after L5 are under the control of the major late promoter or under the E4 promoter. In one embodiment a gene or genes placed before L5 are under the control of the major late promoter or the E3 promoter. Genes placed directly before L5 start codon can be under the control of the major late promoter and will generally need to contain a regulatory element that allows the expression of L5.

In one embodiment a gene or genes placed after L5 are under the control of an exogenous promoter. In one embodiment the method further comprises the step of excising the adenovirus genome from the plasmid and forming a virus or viral vector.

Thus, the method of the present disclosure provides plasmids of the present disclosure and intermediates such as the shuttle vector.

The plasmid prepared according to the present method may further comprise a transgene, for example in the form of a transgene cassette.

The transgene may be selected from the group comprising a therapeutic gene of interest which encodes a therapeutic protein, peptide or RNA such as an antibody or antibody domain, pro-drug converting enzyme, immunomodulator, enzyme, siRNA, transcription factor, intracellular signalling or surface membrane protein, antigen; and a reporter gene or imaging agent, such as luciferase or eGFP. Advantageously the method provides a flexible means of generating novel adenovirus plasmids and the intermediate shuttle vectors wherein the introduction of original restriction sites, for example in step b) permits manipulation of the adenovirus genome outside of the regions regulating early gene expression.

### BRIEF DESCIPTION OF THE FIGURES

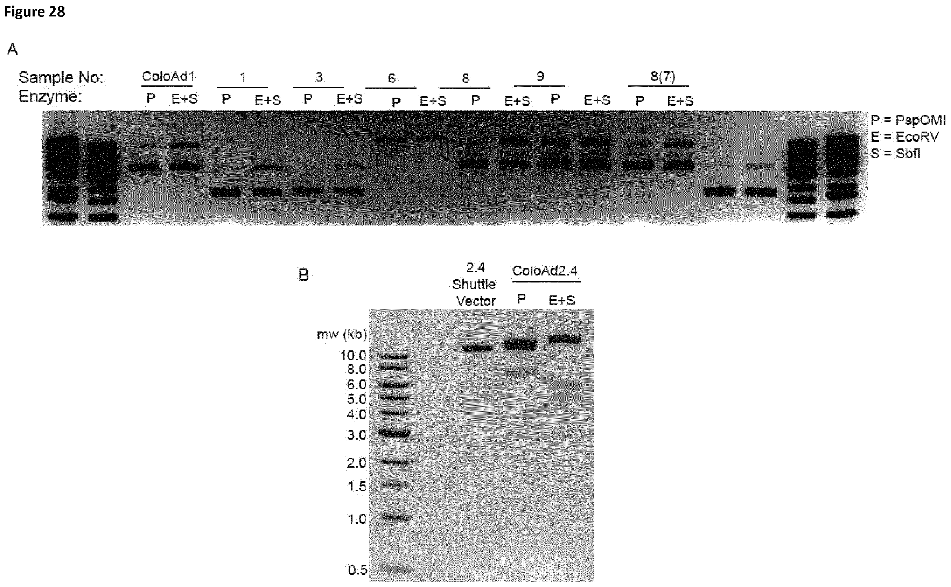

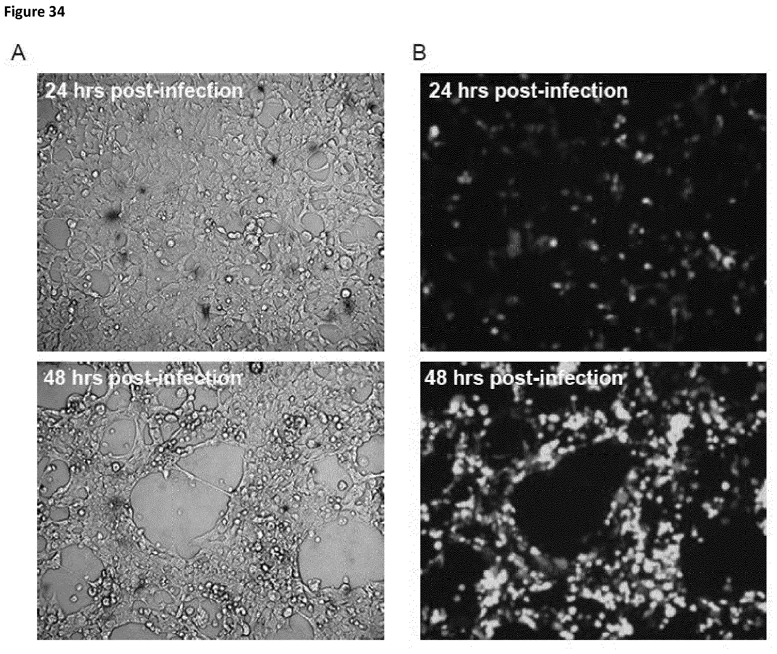

- **Figure 1**: shows a schematic of the ColoAd1 genome. Early genes (E1, E2, E3 and E4) are represented in dark grey and late genes (L1, L2, L3, L4 and L5) in light grey
- **Figure 2**: shows schematics of the ColoAd plasmids - ColoAd2.0, ColoAd2.1 and ColoAd2.4.
- **Figure 3**: shows specific alterations in ColoAd1 genome sequence in ColoAd2.0, ColoAd2.1 and Colo2.4 plasmids. All base pairs shown in black are additional to the ColoAd1 genome sequence. The sequences and names of the specific restriction enzymes are indicated.
- **Figure 4**: shows a schematic showing PspOMI restriction site locations in the ColoAd1 genome.
- **Figure 5**: shows an overview of ColoAd plasmid construction.
- **Figure 6**: shows restriction site map of the ColoAd1 shuttle vector
- **Figure 7**: shows a generic transgene cassette design for insertion into the ColoAd2.4 plasmid or ColoAd2.4 shuttle vector.
- **Figure 8**: shows the 12kb ColoAd1 shuttle vector with a p15A origin of replication, a Kanamycin resistance gene, the ITRs, E1A, E1B genes, partial E2B gene, partial E3 gene, Fibre gene and E4 genes of ColoAd1
- **Figure 9**: shows a map of ColoAd1 detailing PspOMI and Acll restriction sites.
- **Figure 10**: shows:
**A** - The 5' arm PCR amplification product. A ~4.6kb fragment containing ColoAd15' ITR, E1A, E1B and partial E2B genes flanked by 5' Ascl and 3' PspOMI restriction sites.
**B** - The 3' arm PCR amplification product. A ~4.5 kb fragment containing E3, fibre, E4 and 3' ITR of ColoAd1 flanked by 5' PspOMI and 3' Ascl restriction sites.
**C** - The p15A-KanR vector fragment PCR product. A ~2.9kb fragment containing 5' and 3' Ascl restriction sites.

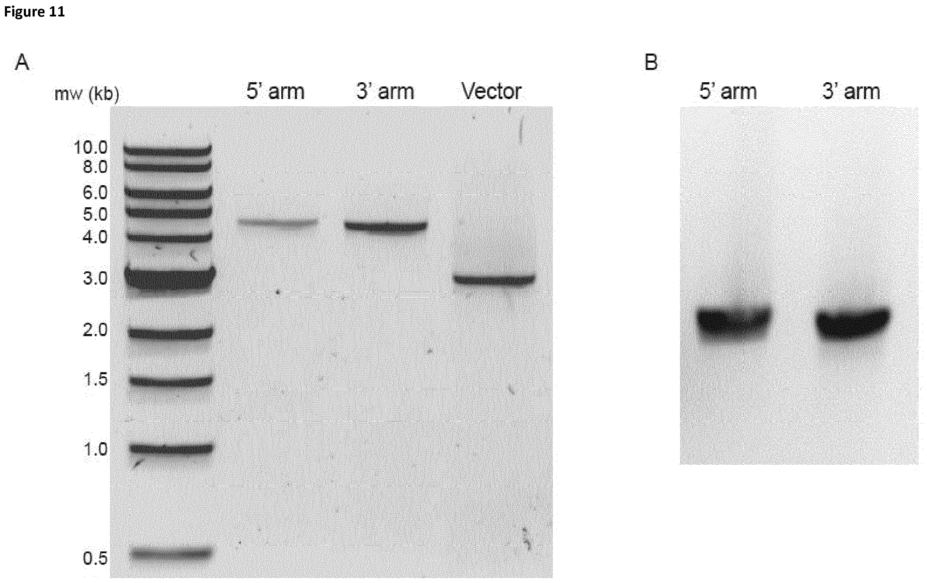

- **Figure 11**: shows:
**A** - PCR products of the 5' arm, 3' arm and p15a KAN vector fragment. Products were 4.6kb, 4.5kb and 3kb respectively
**B** - PCR products of the 5' arm and 3' arm.

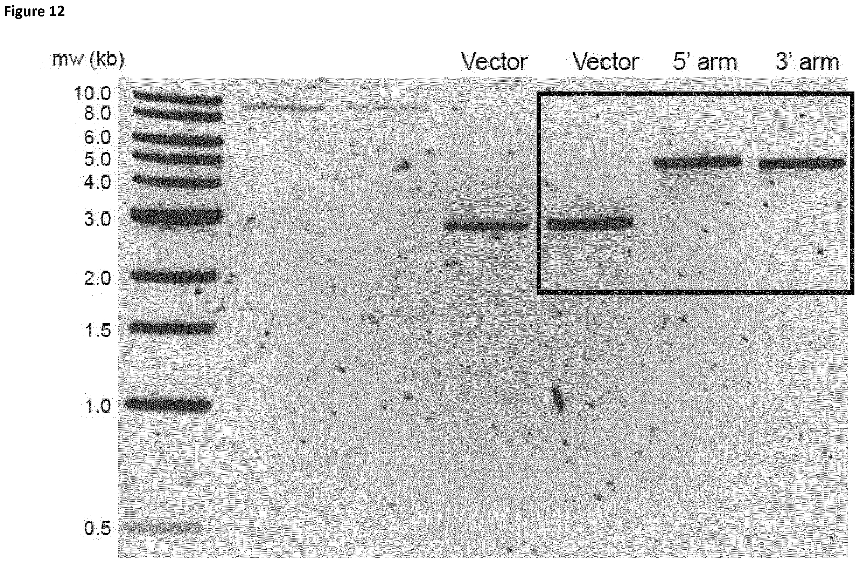

- **Figure 12**: shows Ascl and Ascl/PsPOMI digested p15A-Kan vector fragment and 5' and 3' arm. Digest products are highlighted in the black box.

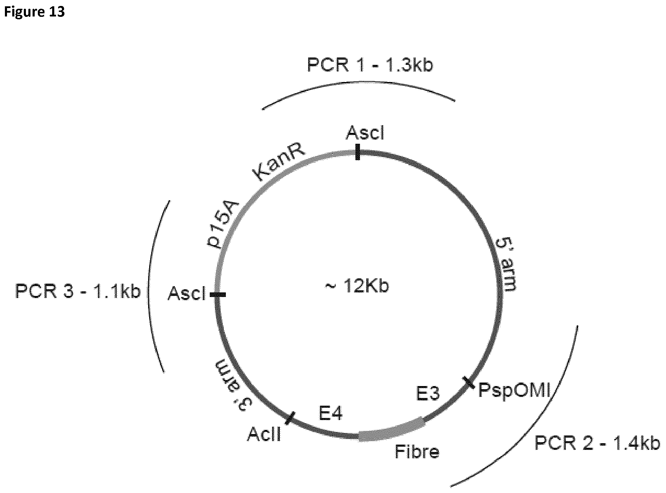

- **Figure 13**: shows a schematic showing the primer binding regions and products expected for each PCR amplification.

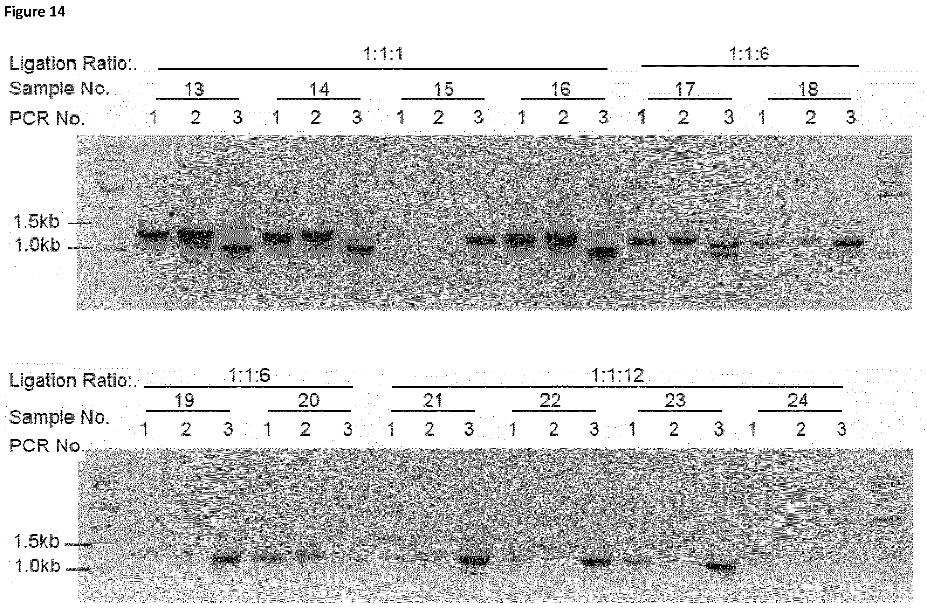

- **Figure 14**: shows gels showing PCR products from representative screened clones. Constructs 13, 14 and 16 showed correctly sized PCR products. These constructs were produced following three-way ligation reaction at a 1:1:1 (5'arm: 3'arm: p15a vector fragment). None of the constructs using the 1:1:6 or 1:1:12 ligation ratios showed the correctly sized PCR products.

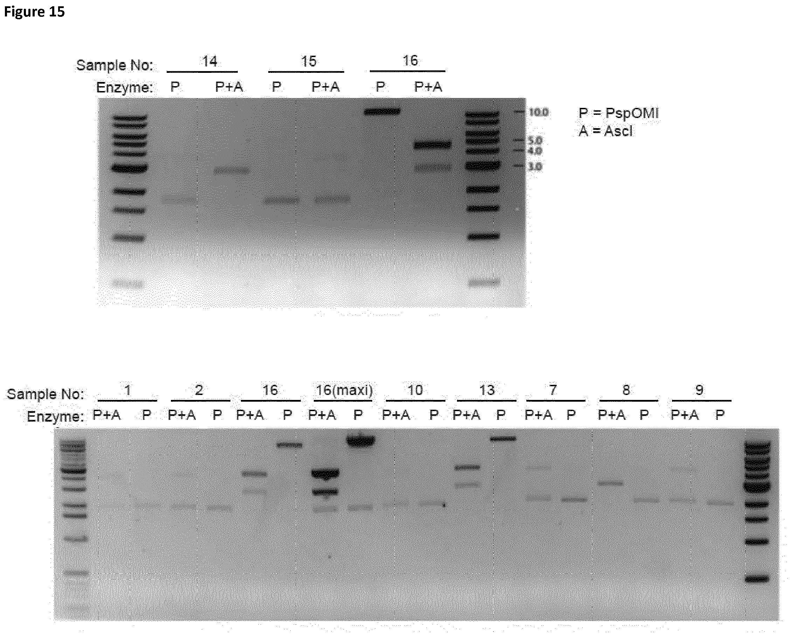

- **Figure 15**: shows restriction analysis with PspOMI or Ascl/PspOMI of selected constructs. Constructs 13 and 16 produced following three-way ligation at a ratio of 1:1:1 showed correctly sized digest products. Sample no. 16 (maxi) corresponds to a digested maxi prep which had been produced from construct 16.

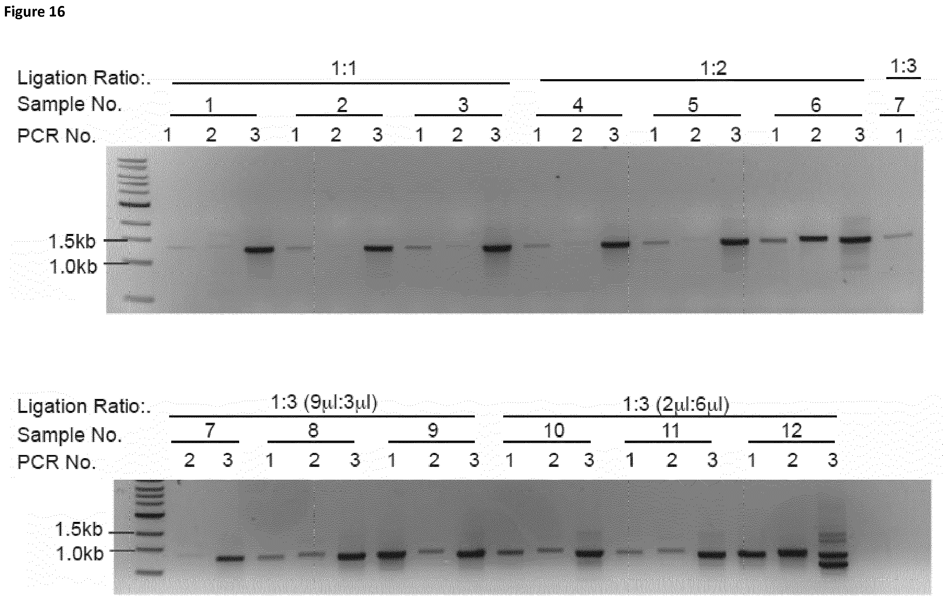

- **Figure 16**: shows gels showing PCR products from constructs generated using the two-step ligation method to make the ColoAd1 Shuttle vector. None of the constructs screened showed the correct PCR products.

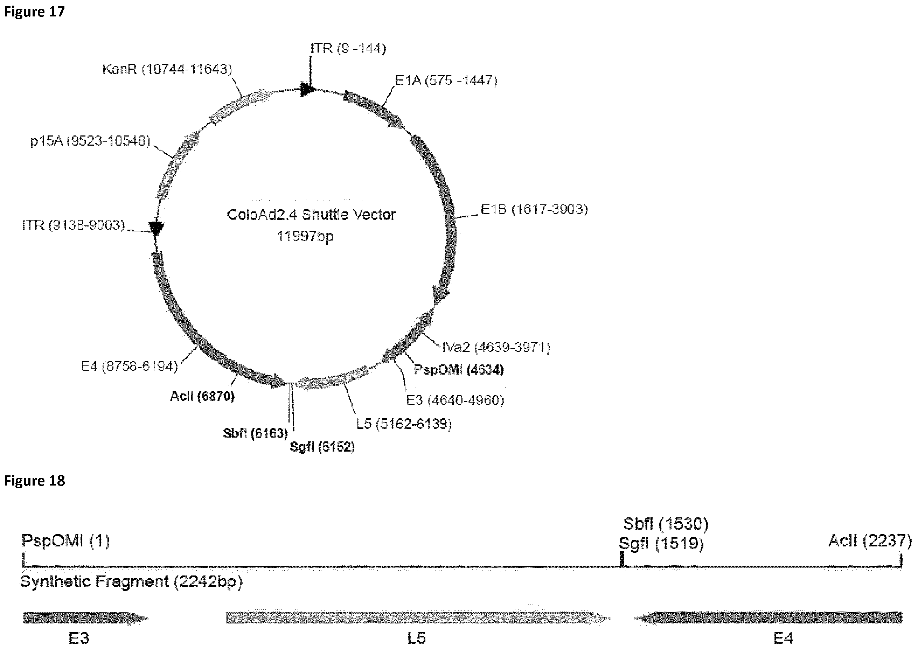

- **Figure 17**: shows the ColoAd2.4 shuttle vector. The shuttle vector contains Sgfl and Sbfl original restriction sites downstream of the Fibre gene.
- **Figure 18**: shows schematic of the ColoAd2.4 synthetic fragment with flanking PsPOMI and Acll restriction sites.

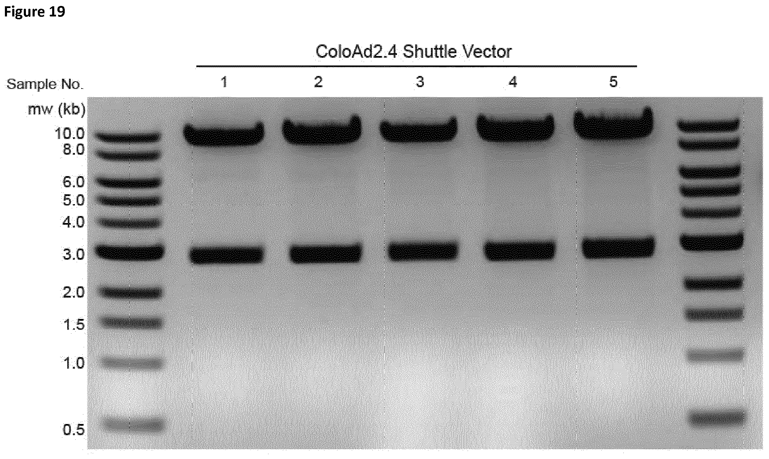

- **Figure 19**: shows restriction analysis of putative ColoAd2.4 shuttle vector constructs. All 5 constructs showed correctly sized bands corresponding to the ColoAd2.4 shuttle vector; a 3 kb and 9 kb band following EcoRV and Sbfl digest

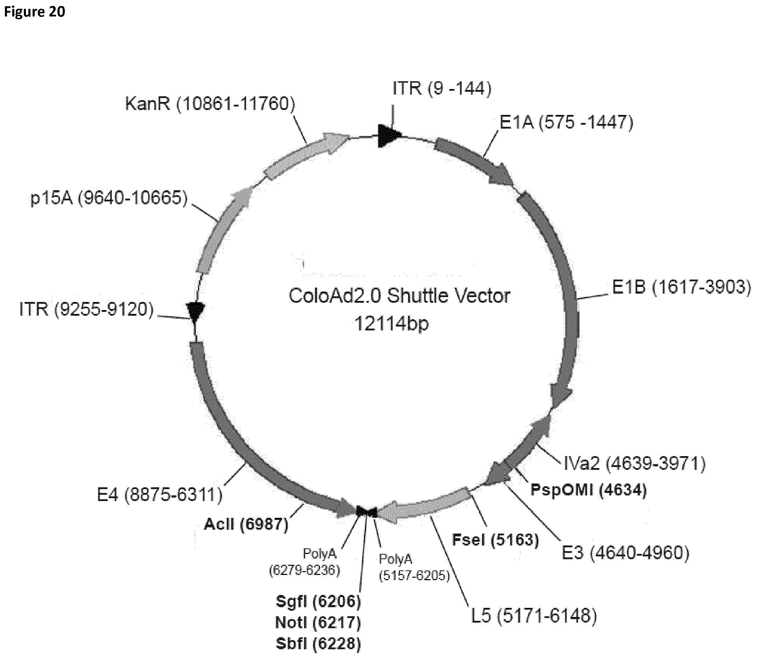

- **Figure 20**: shows the ColoAd2.0 shuttle vector. The shuttle vector contains an original Fsel site upstream of the Fibre (L5) gene and 2 polyA sequences and original Sgfl, Notl, Sbfl sites downstream of the Fibre (L5) gene.

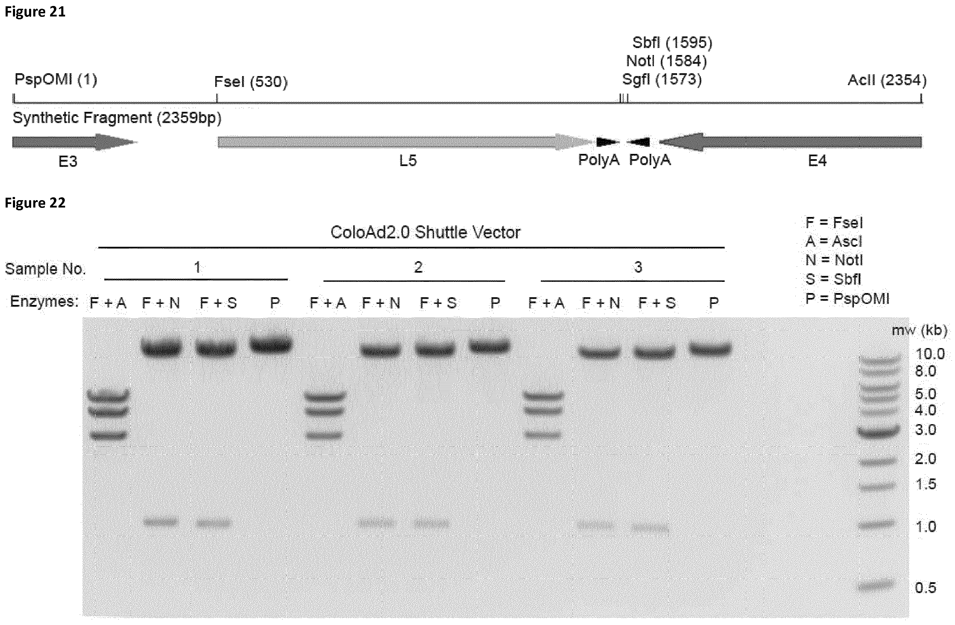

- **Figure 21**: shows a schematic of the ColoAd2.0 synthetic fragment with flanking PspOMI and Acll restriction sites
- **Figure 22**: shows restriction analysis of putative ColoAd2.0 shuttle vector constructs with the enzymes Fsel, Ascl, Sbfl or PspOMI. All five constructs show the correctly sized bands corresponding to the ColoAd2.0 shuttle vector

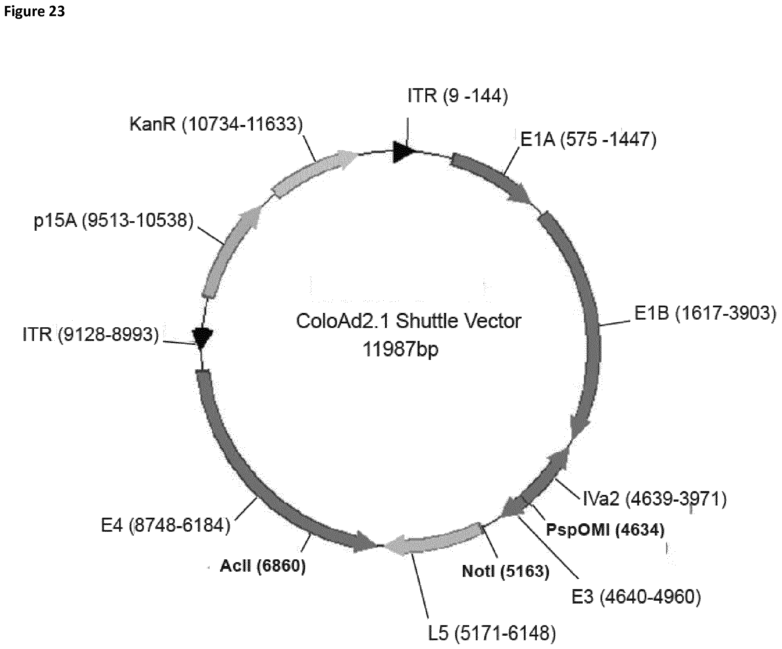

- **Figure 23**: shows the ColoAd2.1 shuttle vector. The shuttle vector contains an original Notl site upstream of the Fibre (L5) gene.

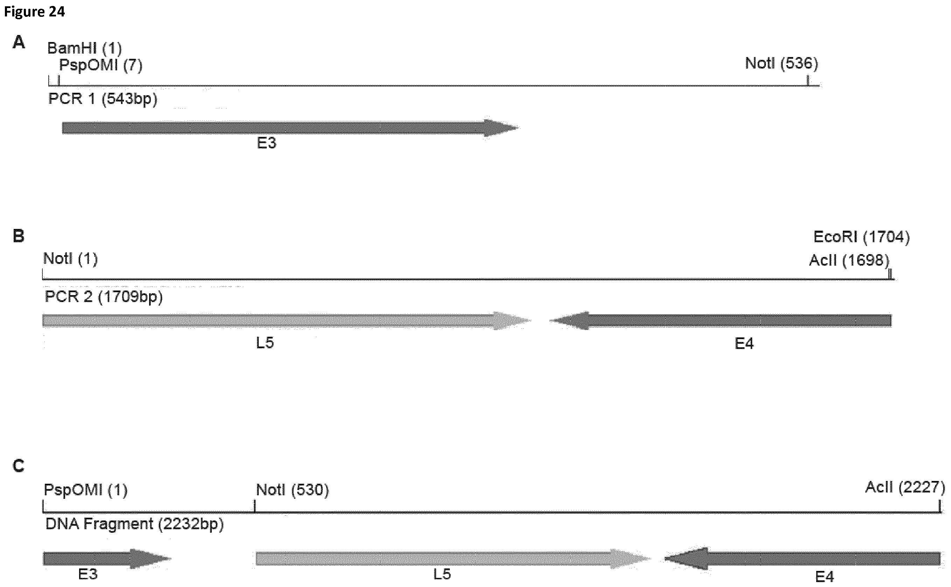

- **Figure 24**: schematics of the PCR 1 (A) and PCR 2 (B) products used to construct the DNA fragment (C) for insertion into the ColoAd1 shuttle vector to generate the ColoAd2.1 shuttle vector

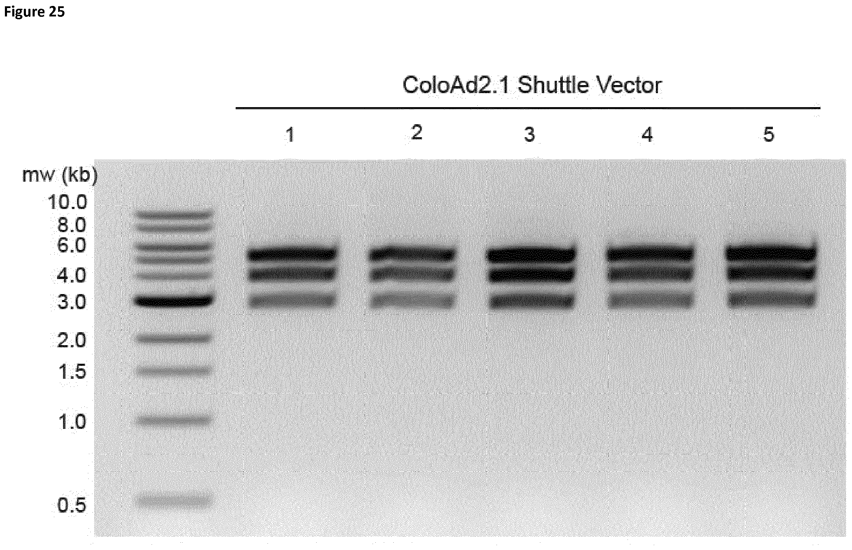

- **Figure 25**: shows restriction analysis of putative ColoAd2.1 shuttle vector constructs. All 5 constructs showed correctly sized bands corresponding to the ColoAd2.1 shuttle vector; 3kb, 4kb and 5kb bands following Ascl and Notl digest.

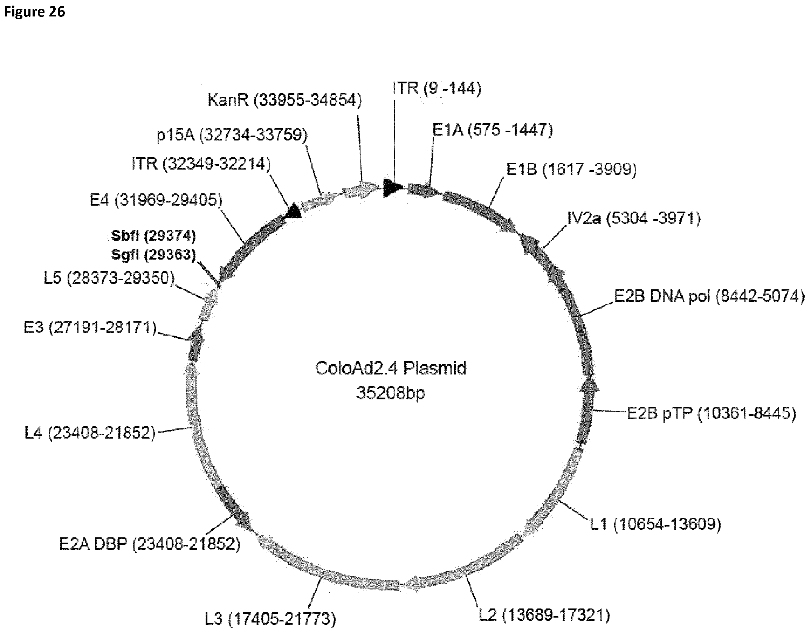

- **Figure 26**: shows the recombinant ColoAd2.4 plasmid. The recombinant contains a p15A origin of replication, kanamycin resistance and the ColoAd1 genome with original Sgfl and Sbfl restriction sites downstream of fibre.

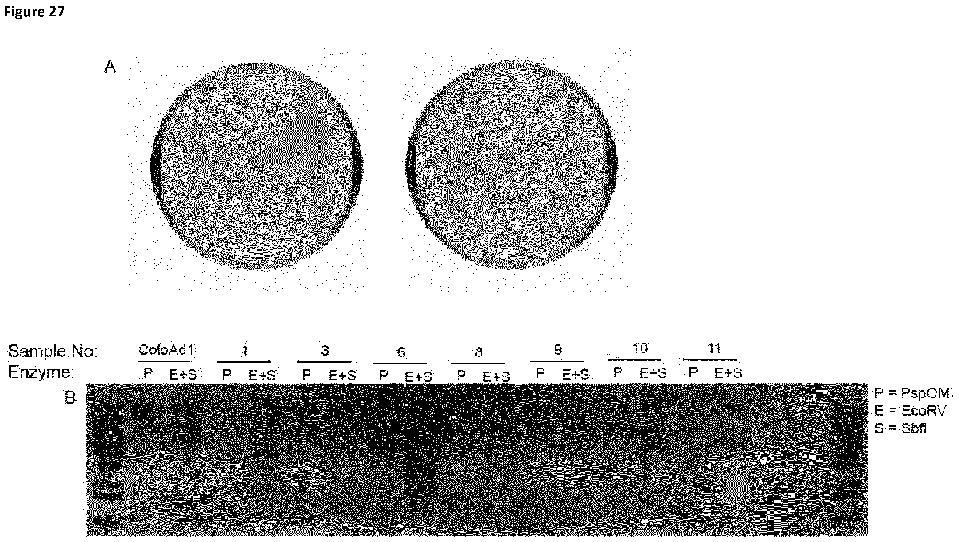

- **Figure 27**: shows:
**A** - LB + Kanamycin plates spread with electroporated BJ5183 cells. The left plate is the negative control and right plate is the ColoAd1 + linearised ColoAd2.4 shuttle vector recombination.
**B** - Restriction digested ColoAd2.4 recombinants. Candidate recombinants were digested with EcoRV and Sbfl (E+S). Recombinants 3, 8 and 10 showed bands of 22kb, 5.5kb, 4.7kb and 2.8kb indicating a correctly formed recombinant. Recombinants were also digested with PspOMI (P) giving bands of 16kb, 12kb and 7kb
- **Figure 28**: shows restriction Analysis of putative ColoAd2.4 recombinants. Recombinant 4 showed correct sized bands of 16kb, 12 kb and 7 kb on PspOMI digestion (P) and bands of 22kb, 4.7kb, 5.5kb and 2.8kb on Sbfl and EcoRV digestion (E+S). The digests confirmed the presence of a recombinant ColoAd2.4 in #4 only.

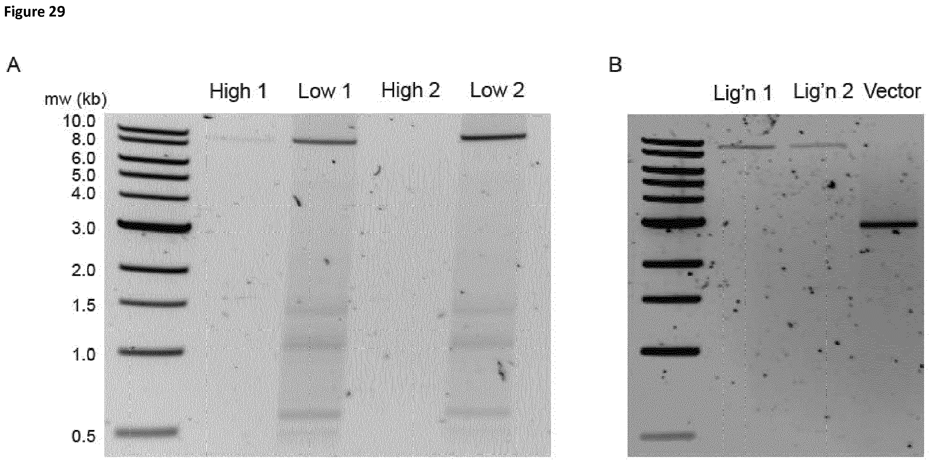

- **Figure 29**: shows:
**A** - PCR amplification products following 5'arm - 3' arm ligation. PCRs following low volume ligations produced ~ 9.1 kb fragments using either 0198 (1) or 0199 (2) primers. PCRs following high volume ligations were not efficient.
**B** - Ascl digested low volume 5' arm - 3'arm ligation products (~9.1 kb, lanes 1 & 2) and Ascl digested p15A-Kan vector fragment (~3 kb, lane 3).

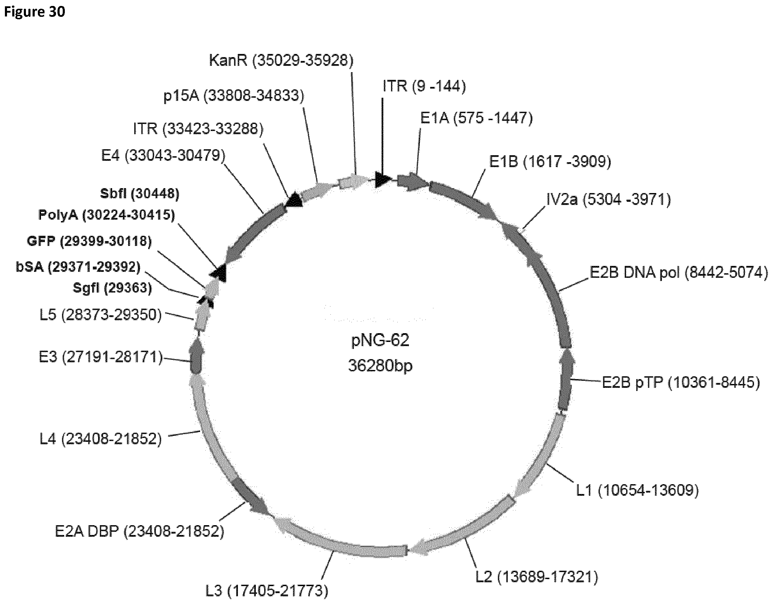

- **Figure 30**: shows a map of the plasmid pNG-62. The was generated from plasmid ColoAd2.4 and contains a p15A origin of replication, kanamycin resistance cassette and the ColoAd1 genome with a GFP reporter gene transgene cassette inserted between original Sgfl and Sbfl restrictions sites

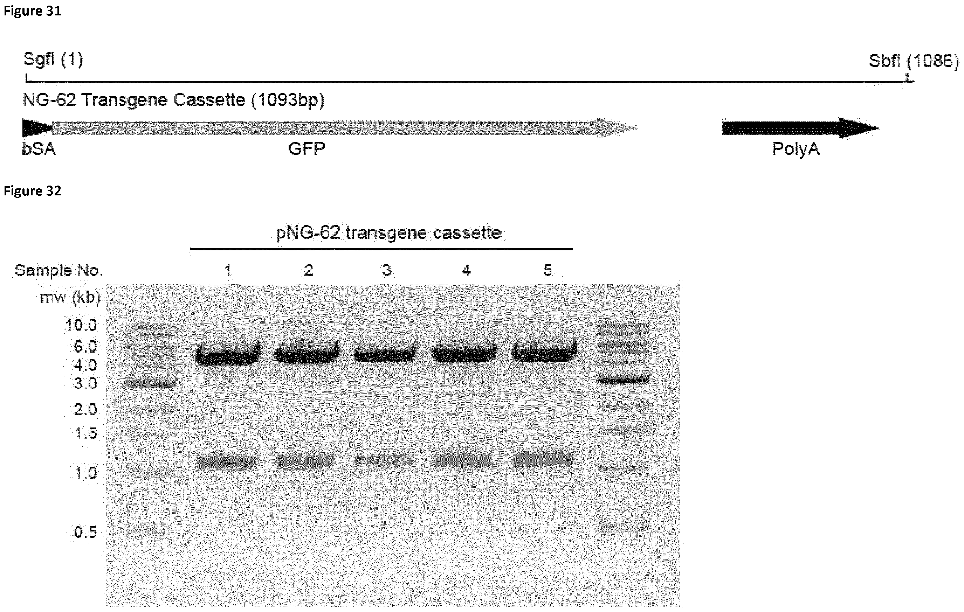

- **Figure 31**: schematic of the transgene cassette present in the pNG-62 plasmid. The cassette contains a branched splice acceptor sequence (bSA), KOZAK sequence, green fluorescent protein (GFP) cDNA and a SV40 late polyA sequence. The cassette is flanked by Sgfl and Sbfl restriction sites for insertion in the ColoAd2.4 vector
- **Figure 32**: restriction digested constructs containing the pNG-62 transgene cassette. All five constructs contain the correctly sized cassette

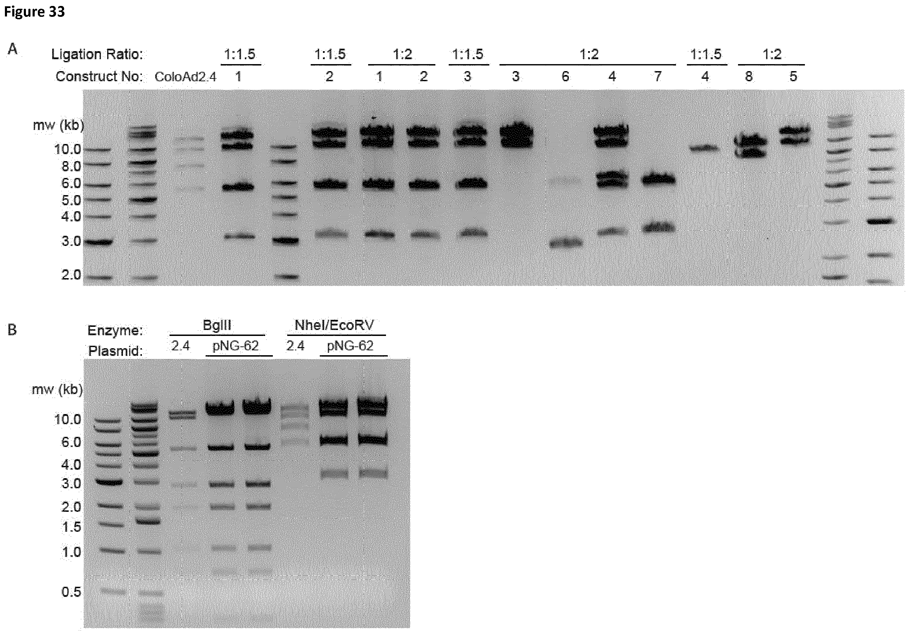

- **Figure 33**: **A** - shows preliminary restriction analysis of pNG-62 plasmids digested with the enzymes Nhel and EcoRV. Constructs numbered 1, 2 & 3 from a 1.5:1 ligation ratio and 1, 2 & 4 from a 2:1 ligation ratio showed correct banding patterns corresponding to the pNG-62 plasmid
**B** - shows diagnostic restriction digest with the enzyme Bglll or enzymes Nhel and EcoRV of two pNG-62 constructs and the plasmid from which pNG-62 was constructed, ColoAd2.4. The banding pattern of DNA fragments confirmed construction of the pNG-62 plasmid
- **Figure 34**: **A -** Brightfield microscopy images of AD293 cells infected for 24hrs (upper panel) or 48 hrs (lower panel) with NG-62 virus particles harvested from Hek293 cells transfected with NG-62 genomic DNA
**B** - Fluorescent microscopy images corresponding to the brightfield images in A, showing GFP expression in AD293 cells infected for 24hrs (upper panel) or 48hrs (lower panel) with NG-62 virus particles

### DETAILED DESCRIPTION

Vector as employed herein refers to a DNA molecule used as a vehicle to artificially carry genetic material into another construct or cell, for example where it can be replicated and/or expressed. The method of preparing the shuttle vector and the shuttle vectors themselves have allowed the present inventors to build plasmids that contain all the necessary functionality to allow manipulation of the adenovirus genome.

Shuttle vector as employed herein refers to a vector that, for example can propagate in two types of host cells, typically bacterial and mammalian cells.

Prior art shuttle vectors generally contain only a minimal amount of adenovirus genomic DNA in the 3' arm, such as an inverted terminal repeat (ITR) sequence of about 100 base pairs. This 3' fragment is sufficient to allow recombination, but does not allow any manipulation of the genome at the 3' end, where the E4, E3 and the L5 genes are located. Assembling small shuttle vectors with short 3' arms is usually carried out using PCR methods and a simple ligation of two DNA fragments. However, ligations to generate larger shuttle vectors, in which a number of adenovirus genes can be manipulated, become more difficult and unpredictable because three DNA fragments of significant size are used. Generally the 3'-arm (fragment iii) employed in the methods according to the present disclosure includes a E3 site and/or E4 site, as appropriate. In one embodiment the 3'-arm comprises the 3' ITRs, L5 and an E3 site, for example as a fragment of the virus genome, i.e. wherein the genetic elements have the corresponding positions as found naturally in the virus (and for example the fragment does not comprise an E4 site). In one embodiment the 3'-arm comprises the 3' ITRs, L5 and an E4 site, for example as a fragment of the virus genome, i.e. wherein the genetic elements have the corresponding positions as found naturally in the virus (and for example the fragment does not comprise an E3 site). In one embodiment the 3'-arm comprises the 3' ITRs, L5, an E3 site and an E4 site, for example as a fragment of the virus genome, i.e. wherein the genetic elements have the corresponding positions as found naturally in the virus.

In general in the prior art, when ligating three pieces of DNA to form a shuttle vector, a two-step ligation is employed wherein two pieces are ligated in the first step and then the third piece is ligated in the second step. This is because ligating large pieces of DNA together is an inefficient process. Surprisingly, the usual method did not successfully generate adenoviruses shuttle vectors and plasmids of the required size. Thus, the use of PCR methods and ligation of two DNA fragments via a two-step method of ligation, after several months of work in hands of the present inventors, did not generate any adenovirus shuttle vectors.

The inventors overcame the problem by employing the present one step, three-way ligation method. Surprisingly a robust and efficient one-step, three-way ligation was eventually identified, after performing several experiments under various conditions. The proportions of DNA components in the three-way ligation appear to be of importance in the successful ligation. The one-step method is a little counter intuitive because in theory it is more difficult to assemble three DNA segments simultaneously than two DNA segments simultaneously. However, the presently disclosed methods have been shown by the present inventors to work thereby allowing the preparation of shuttle vector and plasmids of the required size.

Once a shuttle vector comprising the E3 site, L5 gene and E4 site was generated, the inventors were able to introduce original restriction sites into the shuttle vector and then create the plasmid containing the full genome, with novel, original restrictions sites in a location removed from the early genes into which transgenes may be instruced. In this embodiment step b), introduction of the restriction sites, was performed after step a). An alternative approach is to, for example prepare the 3' arm (fragment ii) already containing the restriction sites and or transgenes. One way to achieve this is by synthesising the 3' arm fragment to have all the desired structure and function prior to performing the ligation step. In the latter embodiment step b) is performed prior to step a). If a cloning platform is required that can employed over and over again then the restriction sites are employed. If only one specific virus construct is required then one may simply insert only the transgenes and machinery necessary for the same to function.

Clearly the 5' arm fragment may also be prepared, for example synthesised, with the required elements, sequence and/or functionality.

When synthetic adenovirus fragments are employed they may be assembled to provide a fully functioning virus or viral vector.

DNA construct as employed herein refers to a shuttle vector or plasmid.

Virus construct as employed herein refers to replication capable virus or replication deficient virus according to the present disclosure.

### Adenoviruses

The present disclosure is broadly applicable to all types of adenoviruses and is particularly suitable for human adenoviruses for example as shown in Table 1, such as subgroup B adenoviruses and specifically to the chimeric adenoviruses EnAd (Enadenotucirev), OvAd1 and OvAd2.

Unless the context indicates otherwise adenovirus as employed herein is a generic term referring and adenovirus of any origin, serotype and including viral vectors. Unless the context indicates otherwise adenovirus genome as employed herein means the entirety of an adenovirus' genetic DNA.

Genomic DNA is part or all of the DNA from an adenovirus genome.

Adenoviruses are non-enveloped icosahedral double-stranded DNA viruses with a linear genome of approximately 34 to 48 kilobase pairs (Kb). Due to the size of the genome, the virus can incorporate about an additional 10% of the genome of foreign DNA without significant impact on its stability or its infectivity. The introduction of longer sequences therefore generally requires the removal of some of the virus' genes.

In one embodiment the adenovirus is a human adenovirus. As employed herein human adenovirus refers to any adenovirus that can be assigned to any of the over 50 currently known adenoviral serotypes, which are classified into subgroups A-F based on various attributes including their haemagglutination properties (see Shenk 2001), and further extends to any, as yet, unidentified or unclassified adenoviral serotypes. See, for example, Strauss, "Adenovirus infections in humans," in The Adenoviruses, Ginsberg, Plenum Press, New York, NY, pp. 451-596 (1984) and Shenk, "Adenoviridae: The Viruses and Their Replication," in Fields Virology, Vol.2, Fourth Edition, Knipe, 35ea., Lippincott Williams & Wilkins, pp. 2265-2267 (2001), as shown in **Table 1:**

| **SubGroup** | **Adenoviral Serotype** |
|---|---|
| A | 12,18,31 |
| B | 3,7,11,14,16,21,34,35,51 |
| C | 1,2,5,6 |
| D | 8-10,13,15,17,19,20,22-30,32,33,36-39,42-49,50 |
| E | 4 |
| F | 40,41 |

All human adenovirus genomes examined to date have the same general organisation i.e., the genes encoding specific functions are located at the same position in the viral genome. Each end of the viral genome has a short sequence known as the inverted terminal repeat (or ITR), which is required for viral replication. The viral genome contains five early transcription units (E1A, E1B, E2, E3, and E4), three delayed early units (IX, IVa2 and E2 late) and one late unit (major late) that is processed to generate five families of late mRNAs (L1-L5). Proteins encoded by the early genes are primarily involved in replication and modulation of the host cell response to infection, whereas the late genes encode viral structural proteins. Early genes are prefixed by the letter E and the late genes are prefixed by the letter L.

A summary of the location of genes in the Ad11 genome is provided in the Examples.

ITRs are common to all known adenoviruses. The Inverted Terminal Repeat (ITR) sequences were so named because of their symmetry, and are the viral chromosome origins of replication. Another property of these sequences is their ability to form a hairpin. The 5' ITR as employed herein refers to the ITR at the 5' end of the genome. The 3' ITR as employed herein refers to the ITR at the 3' end of the genome.

L5 gene as employed herein means the fibre gene. The fibre gene encodes the fibre protein which is a major capsid component of adenoviruses. The fibre functions in receptor recognition and contributes to adenovirus' ability to selectively bind and infect cells. The fibre gene, may for example comprise in region of 986 base paries. In one embodiment the fibre is defined by positions 30811-31788 of the genome, for example the Ad11 genome, in particular as defined in SEQ ID NO: 1 of US7,459,153 see also virus deposit Genbank accession: AY598970.

Non-human adenoviruses include ovine, porcine, canine and chimp viruses.

In one embodiment the adenovirus genome is a subgroup B adenovirus genome. Subgroup B as employed herein means a serotype B adenovirus. Subgroup B adenovirus include Ad3, Ad7, Ad11, Ad14, Ad16, Ad21, Ad34, Ad35, Ad51. The most widely studied adenovirus, Ad5, is a subgroup C adenovirus. Ad5 immunity is common in the human population making it a poor candidate for therapy because the virus is likely to be neutralised by a rapid immune response.

In one embodiment the adenovirus is selected from EnAd (Enadenotucirev SEQ ID NO: 1 also known as ColoAd1 a replication competent oncolytic chimeric adenovirus - see WO2005/118825), OvAd1 (see WO2008080003 - SEQ ID NO: 1 therein), OvAd2 (see WO2008080003 - SEQ ID NO: 2 therein), Ad3 (Genbank accession: DQ086466), Ad11 such as Ad11p (Genbank accession: AY598970) and Ad5, for example EnAd (SEQ ID NO.1), OvAd1 or OvAd2, in particular EnAd.

In one embodiment the adenovirus genome employed in the method of the present disclosure has at least 95% sequence identity to EnAd (SEQ ID NO: 1), such as 96, 97, 98, 99 or 100% sequence identity. In one embodiment the adenovirus genome has at least 95% sequence identity to OvAd1 (SEQ ID NO: 1 of WO2008080003), such as 96, 97, 98, 99 or 100% sequence identity. In one embodiment the adenovirus genome has at least 95% sequence identity to OvAd2 (SEQ ID NO.2 of WO2008080003), such as 96, 97, 98, 99 or 100% sequence identity. In one embodiment the adenovirus genome has at least 95% sequence identity to Ad3 (Genbank accession: DQ086466), such as 96, 97, 98, 99 or 100% sequence identity. In one embodiment the adenovirus genome has at least 95% sequence identity to Ad11p (Genbank accession: AY598970), such as 96, 97, 98, 99 or 100% sequence identity. Advantageously, EnAd has a wild type Ad11p capsid, a chimeric E2B region derived from both Ad3 and Ad11 and deletions in the E3 and E4 region (in particular the whole of E3 is deleted and E4orf4 is deleted). These structural changes provide additional 'space' in the ColoAd1 genome for the insertion of transgene cassettes expressing, for example therapeutic or immunomodulatory agents. Furthermore, because it is a subgroup B adenovirus, pre-existing immunity in humans is less common in comparison to Ad5.

The structural changes in EnAd result in a genome that is approximately 3.5kb smaller than Ad11p thereby providing additional "space" for the insertion of transgene cassettes. EnAd is a suitable vehicle for delivering a wide range of therapeutic proteins, for example that augment or synergise with EnAd's potent anti-cancer activity. OvAd1 and OvAd2 are also chimeric adenoviruses similar to ColoAd1 which also have additional "space" in the genome (see WO2008/080003).

Sequence identity as employed herein refers to two polynucleotide or amino acid sequences being identical (i.e., on a nucleotide-by-nucleotide or residue-by-residue basis) over the comparison window, for example over their full length. The term "percentage of sequence identity" is calculated by comparing two optimally aligned sequences over the window of comparison, determining the number of positions at which the identical nucleic acid base (e.g., A, T, C, G, U, or I) or residue occurs in both sequences to yield the number of matched positions, dividing the number of matched positions by the total number of positions in the comparison window (i.e., the window size), and multiplying the result by 100 to yield the percentage of sequence identity.

The term "substantial identity" as used herein denotes a characteristic of a polynucleotide or amino acid sequence, wherein the polynucleotide or amino acid comprises a sequence that has at least 85 percent sequence identity, for example at least 90 to 95 percent sequence identity, in particular at least 99 percent sequence identity as compared to a reference sequence over a comparison window of at least 18 nucleotide (6 amino acid) positions, frequently over a window of at least 24-48 nucleotide (8-16 amino acid) positions, wherein the percentage of sequence identity is calculated by comparing the reference sequence to the sequence which may include deletions or additions which total 20 percent or less of the reference sequence over the comparison window. The reference sequence may be a subset of a larger sequence.

The term "similarity", when used to describe a polypeptide, is determined by comparing the amino acid sequence and the conserved amino acid substitutes of one polypeptide to the sequence of a second polypeptide.

The term "homologous", when used to describe a polynucleotide, indicates that two polynucleotides, or designated sequences thereof, when optimally aligned and compared, are identical, with appropriate nucleotide insertions or deletions, in at least 70% of the nucleotides, usually from about 75% to 99%, and in particular at least about 98 to 99% of the nucleotides. In one embodiment the comparison is performed across the full length of a given sequence.

In one embodiment the adenovirus genome has a chimeric E2B region, for example a recombinant chimeric adenovirus, or a variant or derivative thereof, having a genome comprising an E2B region wherein said E2B region comprises a nucleic acid sequence derived from a first adenoviral serotype and a nucleic acid sequence derived from a second adenoviral serotype; wherein said first and second serotypes are each independently selected from the adenoviral subgroups B, C, D, E, or F and are distinct from each other; and wherein said chimeric adenovirus is oncolytic and demonstrates an enhanced therapeutic index for a tumor cell, in particular as disclosed in WO2005/118825.

In one embodiment the adenovirus genome has part or all of the E3 region deleted or mutated, for example the E3 region is mutated. In one embodiment the adenovirus genome has part or all of the E4 region deleted or mutated, for example E4orf4 is deleted.

### Replication Deficient and Replication Capable

In one embodiment the adenovirus genome employed is from an adenovirus capable of replication, in particular replication competent. In one embodiment a virus or viral construct of the present disclosure is replication capable, in particular replication competent. In one embodiment EnAd is capable of replication.

Capable of replication as employed herein generally refers viruses capable of replicating, for example without the need for a packaging cell line. They are also referred to herein as viruses, replication competent viruses (which is a subgroup of replication capable viruses), and conditionally replicating viruses (also a subgroup of replication capable viruses) or live viruses. Thus, replication capable viruses as employed herein refers to conditionally replicating viruses and replication competent viruses. Conditionally replicating viruses as employed herein refers to viruses that can replicate in cells, such as cancers cells, which express or over-express or under-expresses a certain gene, for example cells which under express p53 gene.

Replication competent viruses are viruses that have all the necessary machinery to replicate, for example *in vivo* and/or *ex vivo,* without the assistance of a help virus or complementary/packaging cell line.

Unless the context indicates otherwise "virus" (as opposed to adenovirus which is defined above) as employed herein refers to a replication capable, for example a replication competent virus. Thus, in one embodiment virus as employed herein generally refers to a replication competent virus, such as a therapeutic virus, such as an oncolytic virus.

In one embodiment the adenovirus genome employed in a method according to the present disclosure is from an adenoviral vector which is not capable of replication. In one embodiment the virus or viral conconstruct of the present present disclosure is non-replication (also referred as replication deficient). Non-replicating adenoviruses, for example viral vectors (employed as a vehicle for delivering transgenes, such as vaccine antigens, intracellular delivery of antibodies and the like) generally have one or more genes removed which are essential to replication. Unless indicated otherwise viral vector as employed herein refers to a replication deficient

Replication deficient adenoviruses are those which require a helper virus or complementary/packaging cell line to replicate. Generally, adenoviruses (for example those comprising a transgene) that require a packaging cell line to replicate are referred to as viral vectors. In one embodiment EnAd is rendered replication deficient, for example by deletion of part or all of the E1 region.

The reasons for the removal of the replication-essential gene or genes are two-fold. Firstly, it is considered that the vectors have a simplified safety profile due to their inability to replicate *in vivo.* Secondly, the deletion of the genes allows insertion of large transgenes by creating space in the genome. These transgenes can be expressed *in vivo,* regardless of the viral vector's inability to replicate. In one embodiment the E1 gene is deleted from the virus in the creation of a viral vector and alternatively or in addition in some instances part or all of the E3 region is deleted.

A packaging cell line is a recombinant cell line which has been prepared to supplement the virus in question with the genes in which it is deficient. Packaging cell as employed herein means a cell that is capable of providing an essential element for the replication of a virus where the virus is deficient in that element. Examples of packaging cell lines include the PerC6 cell line. In one embodiment the packaging cell line is a HEK cell.

### Virus genes

E1 plays a role in viral replication. By deleting or mutating the E1 region, viruses are unable to replicate without the aid of a specific "packaging" cell line.

In one embodiment the E1 region in the genome of a viral vector of the present disclosure is wholly or partially deleted. In one embodiment the E1 site is a deletion of the E1 region or a fragment thereof is deleted in the 5' arm.

E1 site as employed herein refers to the location of the E1 region, regardless of whether the whole, part or none of the regions is deleted or replaced. The term "E1 site" includes where E1 is mutated, partially deleted, wholly deleted or wholly intact and non-mutated. The E1 site can be absent when the fragment or sequence starts after or ends before the E1 region, for example including starting after any non-coding region associated with the E1, such as the sequence starting in the E2 region. In one embodiment the E1 site consists of the E1 region or a functional fragment or mutation thereof. In one embodiment E1 is present and complete and/or functional.

The E3 encodes proteins important for modulating the host cell's response to viral infection.

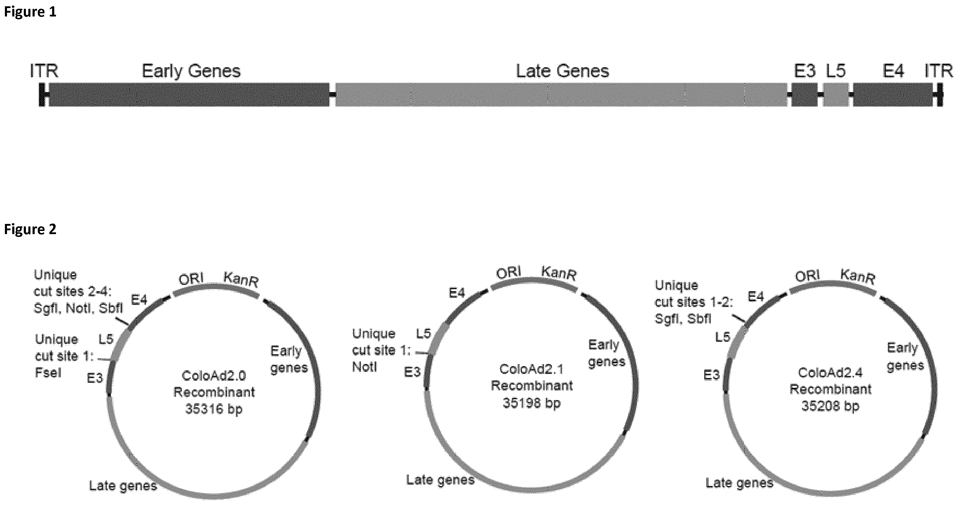

E3 site as employed herein refers to the position in the adenovirus genome where the E3 gene or region is found or would be expected to be found. The E3 gene may be wholly present, present as a fragment or wholly absent, partially or fully mutated. Thus the E3 site as employed herein refers to the location of the E3 region, regardless of whether the whole, part or none of the regions is deleted or replaced. Given that the genome architecture of adenoviruses is uniform between all known human adenoviruses (see Figure 1), the skilled person is able to identify the E3 site whether the gene is present or deleted. The E3 site may in fact be absent when the fragment or sequence concerned starts beyond the E3 region or terminates before the E3 region, for example including any non-coding region associated with the E3, such as the sequence starting in the L5 region.

E4 site as employed herein refers to the position in the adenovirus genome where the E4 gene or region is found or would be expected to be found. The E4 gene may be wholly present, present as a fragment or wholly absent, partially or fully mutated. Thus the E4 site as employed herein refers to the location of the E4 region, regardless of whether the whole, part or none of the regions is deleted or replaced. Given that the genome architecture of adenoviruses is uniform between all known human adenoviruses (see Figure 1), the skilled person is able to identify the E4 site whether the gene is present or deleted. The E4 site may in fact be absent when the fragment or sequence concerned starts beyond the E4 region or terminates before the E4 region, for example including any non-coding region associated with the E4, such as the sequence starts in the L5 region.

The L5 gene encodes the late expressed capsid protein fibre.

In one embodiment the L5 region is the full length sequence or a function fragment thereof, for example a fragment that retains 50% or more, such as 60, 70, 80, 90 or 100% of the activity of the wild-type gene, in particular in an *in vitro* assay. In one embodiment the L5 comprises 80% or more of the genomic DNA an L5 gene, for example 85, 90, 91, 92, 93, 94, 95, 96, 97, 98 or 99%.

In one embodiment part or all of the E3, E4 or L5 genes are independently mutated.

Mutated as employed herein refers a modification in the relevant gene, for example a point mutation, a deletion mutation, an addition mutation, a forward mutation, a substitution mutation and/or a frameshift mutation. In one or more embodiments the amount of genetic material that is mutated is 10% or less of the relevant sequence. In essential genes, for example L5 (the fibre gene) the function of the gene is maintained in the mutated gene, such as 50, 60, 70, 80, 90 or 100 of the function is retained, or the function is increased i.e. is greater than 100% of the unmutated gene.

### Restriction sites

Restrict site as employed herein is a short DNA sequence specifically recognised and cut by a restriction enzyme.

Suitable restriction site as employed herein means a site that may be employed (in conjunction with an appropriate restriction enzyme) to specifically cleave the genomic DNA of the virus in a given location, for example in a region of the DNA that is not an early gene. In one embodiment the DNA is cleaved to facilitate the insertion of a transgene. In one embodiment a suitable site typically is present near the 5' end of the genome and/or the 3' end of the virus genome or the corresponding location in the 3' arm fragment and/or the 5' arm fragment, this allows the DNA to be linearised and manipulated, for example as shown in the Figures.

In one embodiment a suitable restriction site is located approximately 4-5 Kb from the ends of the 3' and/or 5' ITRS such that the shuttle vector will have approximately 4-5 Kb 3' and/or 5' arms. Advantageously this permits efficient homologous recombination between the shuttle vector and the adenovirus genome. This design also allows the shuttle vector to contain the E1 gene (for example in the 5' arm) and the Fibre and E4 gene (for example in the 3' arm) so that any regions surrounding these genes may be manipulated for transgene cassette insertion.

In one embodiment the shuttle vector comprises 1, 2, or 3 further suitable/original restriction sites. In one embodiment a suitable restriction site is not inserted at any other location in the shuttle vector. In one embodiment restriction sites are incorporated around at least one early gene or a part thereof, for example independently selected from E1, E2, E3 and E4, to provide further options for the skilled person to manipulate the genome.

A suitable restriction site will general be an original restriction site. Original, novel, unique in the context of restrictions sites are employed interchangeably herein, and is/are intended to refer a restriction site that can be cut specifically. Restriction site or in some instances a pair of restriction sites may be cut specifically, when they only occur in the location that it is desirable to cut. Thus if the restriction site only occurs once or a pair of restrictions sites occur only once in the virus genome or genomic DNA then this restriction site or sites (in the case of a pair) can be cut specifically by an appropriate enzyme. Thus, in one embodiment original, novel or unique refers to the introduction of a restriction site or pair of restriction sites that was not previously present anywhere in the virus genome or the relevant genomic DNA. Thus, in one embodiment a suitable restriction site is non-natural (exogenous) to the adenovirus genome. In one embodiment the original restriction site or pair of restriction sites occurs only once in the virus genome or genomic DNA. In one embodiment a suitable restriction site will produce sticky ends. In one embodiment a suitable restriction site will be cleaved by an appropriate commercially available restriction enzymes.

Thus, a restriction site is a location in a DNA sequence that can be cut by a restriction enzyme, usually an enzyme specific to the sequence. In one embodiment the restriction site comprises 3 to 22 base pairs, for example 4 to 22, such as 5, 6, 7, 8, 9, 10, 11, 12, 13, 14, 15, 16, 17, 18, 19, 20, 21 or 22 base pairs.

Introduced or introducing as employed herein generally refers to inserting a restriction site that is not found in the native, wild-type or starting adenovirus genome.

In one embodiment original restriction site as employed herein means a restriction site that occurs only once in the plasmid or in virus contruct. This permits certainty that a given restriction enzyme will cut the plasmid in only one known location. In the context of the present disclosure an original restriction site is typically one which has been introduced into the genome and was not naturally occurring.

In one embodiment original restriction site as employed herein refers to a restriction site introduced into the adenovirus genome by recombinant techniques, in particular where the restriction site introduced in not naturally occurring in the virus and thereby give specificity in the location of the virus is genome is cut when the given restriction enzyme is employed.

Thus, in another embodiment original restriction site as employed herein means a restriction site that occurs only once in the plasmid, virus or shuttle vector. This permits certainty that a given restriction enzyme will cut the plasmid in only one known location.

In particular the original restriction sites permit transgene cassettes to be inserted in to the genome at, for example a location removed from the early genes.

The method is broadly applicable to adenoviruses and suitable restriction sites that may provide appropriate 5' and 3' arms from which to build a shuttle vector have been identified in ColoAd1, OvAd1, OvAd2, Ad3, Ad11 and Ad5.

Thus, the method of the present disclosure may be used as a starting point to generate therapeutic viruses with enhanced activity conferred by, for example the introduction of transgenes.

In one embodiment the original restriction site is independently selected from Fsel, Notl, Sbfl and Sgfl, for example Notl or Sbfl and Sgfl or Fsel and Notl and Sbfl and Sgfl. Other restriction enzymes that cut at the same sequence are interchangeable.

Fsel as employed herein refers to a restriction site cut by a restriction enzyme at the following sequence -GGCCGG/CC-. Other restriction enzymes that cut at the same sequence are interchangeable.

Notl as employed herein refers to a restriction site cut by a restriction enzyme at the following sequence -GC/GGCCGC-. Other restriction enzymes that cut at the same sequence are interchangeable.

Sbfl as employed herein refers to a restriction site cut by a restriction enzyme at the following sequence -CCTGCA/GG-. Other restriction enzymes that cut at the same sequence are interchangeable.

Sgfl as employed herein refers to a restriction site cut by a restriction enzyme at the following sequence -GCGAT/CGC-. Other restriction enzymes that cut at the same sequence are interchangeable.

In one embodiment two or more original restriction sites are inserted at a given location in the genome.

In one embodiment the original restriction site is a single original restriction site in a location between the L5 gene and the E3 site. Such as a single original restriction site introduced in a location between the L5 gene and the E3 site, such as Notl. In one embodiment this single original restriction site is the only original restriction site introduced into the plasmid or virus construct.

In one embodiment the original restriction site is one or two (such as two) original restriction sites in a location between the L5 gene and the E4 site, for example two original restriction sites introduced in a location between the L5 gene and the E4 site, such as one Sbfl site and one Sgfl site. In one embodiment these are the only two original restriction sites introduced into the shuttle vector, plasmid or virus construct.

In one embodiment the original restriction site is a single original restriction site in a location between the L5 gene and the E3 site and three original restriction sites in a location between the L5 gene and the E4 site, for example a single original restriction site introduced in a location between the L5 gene and the E3 site, such as one Fsel site, and three original restriction sites introduced in a location between the L5 gene and the E4 site, such as one Notl site, one Slbfl site and one Sgfl site. In one embodiment these are the only four original restriction sites introduced into the shuttle vector, plasmid or viral construct.

In one embodiment Fsel is an original restriction site suitable for introduction to an adenovirus genome selected from the group ColoAd1, Ad11, Ad3, OvAd1 and OvAd2. Rigl is a known restriction enzyme that cuts at the same sequence as Fsel, therefore Fsel restriction sites are also known as Rigl restriction sites.

In one embodiment Notl is an original restriction site suitable for introduction to an adenovirus genome selected from the group ColoAd1 and Ad11. CciNI is a known restriction enzyme that cuts at the same sequence as Notl, therefore Notl restriction sites are also known as CciNI restriction sites.

In one embodiment Sbfl is an original restriction site suitable for introduction to an adenovirus genome selected from the group ColoAd1, Ad11, Ad3, OvAd1 and OvAd2. Sdal and Sse83871 are known restriction sites that cut at the same sequence as Sbfl, therefore Sbfl restriction sites are also known as Sdal restriction sites or Sse83871 restriction sites.

In one embodiment Sgfl is an original restriction site suitable for introduction to an adenovirus genome selected from the group ColoAd1, Ad11, Ad3, OvAd1 and OvAd2. AsiSI, Rgal and SfaAl are known restriction sites that cut at the same sequence as Sgfl, therefore Sgfl restriction sites are also known as AsiSI restriction sites, Rgal restriction sites or SfaAl restriction sites.

In one embodiment Clal is an original restriction site suitable for introduction to the Ad5 adenovirus genome. Clal as employed herein means a restriction site cut by a restriction enzyme at the following sequence - AT/CGAT -. BspDl, Zhol, BspDl, Banlll, Bsa29I, BseCl, BshVI, BsiXI, Bsp106I, BspXl, Bsu15I and BsuTUI are known restriction sites that cut at the same sequence as Clal, therefore Clal restriction sites are also known as BspDl restriction sites, Zhol restriction sites, BspDl restriction sites, Banlll restriction sites, Bsa29I restriction sites, BseCl restriction sites, BshVI restriction sites, BsiXI restriction sites, Bsp106I restriction sites, BspXl restriction sites, Bsu15I restriction sites or BsuTUI restriction sites. In one embodiment PacI is an original restriction site suitable for introduction to the Ad5 adenovirus genome. PacI as employed herein means a restriction site cut by a restriction enzyme at the following sequence -TTAAT/TAA -.

In one embodiment Mlul is an original restriction site suitable for introduction to an adenovirus genome selected from the group Ad3 and OvAd2. Mlul as employed herein means a restriction site cut by a restriction enzyme at the following sequence - A/CGCGT -.

In one embodiment BstBI is an original restriction site suitable for introduction to the Ad5 adenovirus genome. BstBI as employed herein means a restriction site cut by a restriction enzyme at the following sequence - TT/CGAA -. Sful, Asull, Bpu14I, BsiCl, Bsp119I, BspT104I, Csp45I and NspV are known restriction sites that cut at the same sequence as BstBI, therefore BstBI restriction sites are also known as Sful restriction sites, Asull restriction sites, Bpu14I restriction sites, BsiCI restriction sites, Bsp119I restriction sites, BspT104I restriction sites, Csp45I restriction sites or NspV restriction sites.

In one embodiment Bcll is an original restriction site suitable for introduction to an adenovirus genome selected from the group ColoAd1, Ad11, Ad3, Ad5, OvAd1 and OvAd2. Bcll as employed herein means a restriction site cut by a restriction enzyme at the following sequence - T/GATCA -. BsiQl, Fbal and Ksp221 are known restriction sites that cut at the same sequence as Bcll, therefore Bcll restriction sites are also known as BsiQI restriction sites, Fbal restriction sites or Ksp221 restriction sites. These restriction sites are generally not preferred.

In one embodiment blunt cutting restriction enzyme sites are employed as original restriction sites, for example Pmel, Srfl and Swal.

In one embodiment Pmel is an original restriction site suitable for introduction to an adenovirus genome selected from the group ColoAd1, Ad11, OvAd1 and OvAd2. Pmel as employed herein means a restriction site cut by a restriction enzyme at the following sequence -GTTT/AAAC-.

In one embodiment Srfll is an original restriction site suitable for introduction to an adenovirus genome selected from the group ColoAd1, Ad11, Ad3, Ad5, OvAd1 and OvAd2. Srfll as employed herein means a restriction site cut by a restriction enzyme at the following sequence -GCCC/GGGC-.

In one embodiment Swal is an original restriction site suitable for introduction to an adenovirus genome selected from the group ColoAd1, Ad11, Ad3, Ad5, OvAd1 and OvAd2. Swal as employed herein means a restriction site cut by a restriction enzyme at the following sequence -ATTT/AAAT-.

In one embodiment the original restriction site other than Notl is in a location between the L5 gene and the E3 site. In one embodiment there are two original restriction sites in a location between the L5 gene and the E4 site, such as one Sbfl and one Sgfl.

In one embodiment there are 4 original restriction sites wherein one original restriction site is in a location between the L5 gene and the E3 site and wherein three original restriction sites are in a location between the L5 gene and the E4 site. Such as wherein the one original restriction site is Fsel and the three original restriction sites are one each of Sbfl, Sgfl and Notl.

In one embodiment the adenovirus genome comprising one or more original restriction sites has the sequence SEQ ID NO. 32. In one embodiment the adenovirus genome comprising one or more original restriction sites has the sequence SEQ ID NO. 33. In one embodiment the adenovirus genome comprising one or more original restriction sites has the sequence SEQ ID NO. 34.

In one embodiment the adenovirus comprising one or more original restriction sites has at least 95% sequence identity to SEQ ID NO. 32, such as 96, 97, 98, 99 or 100% identity. In one embodiment the adenovirus comprising one or more original restriction sites has at least 95% sequence identity to SEQ ID NO. 33, such as 96, 97, 98, 99 or 100% identity. In one embodiment the adenovirus comprising one or more original restriction sites has at least 95% sequence identity to SEQ ID NO. 34, such as 96, 97, 98, 99 or 100% identity.

The plasmids and shuttle vectors prepared have a novel combination of original restriction sites located around the genome of the adenovirus. These can then be selected and employed to give control over which part the adenovirus genome is to be manipulated.

The original restriction sites are introduced strategically to allow for example a transgene to be inserted at the location of one restriction site or alternatively for sections of genome to be deleted between two chosen restriction sites, as desired.

In one embodiment the plasmid further comprises at least one E1 original restriction site. E1 original restriction site as employed herein refers to a restriction site introduced into the E1 region of the adenovirus genome. In some embodiments the E1 gene is present but interrupted by the introduced E1 original restriction site, in other embodiments the E1 gene is deleted and replaced by the introduced E1 original restriction site, in yet other embodiments the E1 gene is present but its function is not altered by the introduced E1 original restriction site.

In one embodiment the restriction site or sites are independently selected from:
- sequence GCGGCCGC cut by Notl and CciNI leaving 5' -GGCC overhangs,
- sequence GGCCGGCC cut by Fsel and Rigl leaving 3' -CCGG overhangs,
- sequence GCGATCGC cut by AsiSI, Rgal, Sgfl and SfaAl leaving 3' -AT overhangs
- sequence CCTGCAGG cut by Sbfl, Sdal and Sse83871 leaving 3' - TGCA overhangs
- sequence TGATCA cut by Bcll, Fbal, Ksp221 and BsiQ1 leaving 5' - GATC overhangs
- sequence CAAAACGTCGTGAGACAGTTTG [SEQ ID NO: 41] cut by I-Cre1 leaving 3' - GTGA overhangs
- sequence TAACTATAACGGTCCTAAGGTAGCGAA [SEQ ID NO: 42] cut by I-Ceul leaving 3' CTAA overhangs
- sequence TAGGGATAACAGGGTAAT [SEQ ID NO: 43] cut by I-Sce1 leaving 3' ATAA overhangs
- sequence GCCCGGGC cut by Srfl leaving blunt ends
- sequence GTTTAAAC cut by Mssl, Pmel leaving blunt ends
- sequence ATTTAAAT cut by Swal, Smil leaving blunt ends
- sequence GGCGCGCC cut by Ascl, PalA1 and Sgsl leaving 5' CGCG overhangs
Other restriction enzymes that cut the same recognition sites may also be suitable.

In one embodiment the first restriction site found in the 3'arm and the vector fragment are the same restriction site (i.e. a site cut by the same restriction enzyme) This facilities joining the 5' end of the vector fragment to the 3' end of the 3' arm.

In one embodiment the second restriction site found in the 5' arm and the vector fragment are the same restriction site (i.e. a site cute by the same restriction enzyme). This facilitates joining the 3' end of the vector fragment to the 5' end of the 5' arm.

In one embodiment the first and second restriction enzyme sites are independently selected from the group Fsel, Rigl, Notl, CciNI, Sbfl, Sdal, Sse83871, Sgfl, AsiSI, Rgal, SfaAl, Clal, BspDl, Zhol, BspDl, Banlll, Bsa29I, BseCl, BshVI, BsiXI, Bsp106I, BspXl, Bsu15I, BsuTUI, Pacl, Mlul, BstBI, Sful, Asull, Bpu14I, BsiCl, Bsp119I, BspT104I, Csp45I, NspV, Bcll, BsiQl, fbal, Ksp221, Pmel, Srfl and Swal.

In one embodiment the first restriction sites is Ascl. In one embodiment the second restriction enzyme sites is Ascl. Ascl as employed herein means a restriction site cut by a restriction enzyme at the following sequence -GG/CGCGCC-. Other restriction enzymes that cut at the same sequence are interchangeable. In one embodiment the first and second restriction sites are the same. In one embodiment the first and second restriction enzyme sites are each Ascl.

In one embodiment where a restriction enzyme site is used as the first and/or second or third restriction enzyme site the genomic DNA will be cut in more than one location.

In one embodiment a restriction site in an adenovirus genome or genomic DNA (such as the 5' arm), comprises a restriction site PspOMI, for example which is located at positions 4628, 20891 and 27840 of the genome or a position corresponding thereto. In one embodiment the adenovirus is EnAd or genomic DNA is derived therefrom.

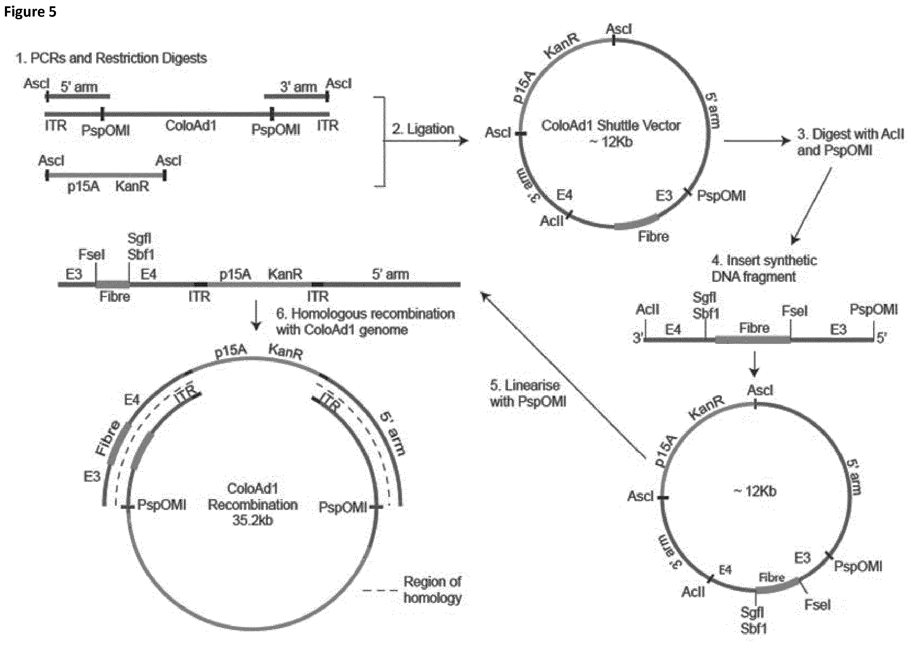

In one embodiment the third restriction site refers to one type of restriction site, for example PspOMI. Thus, in one embodiment the third restriction site in the 3' arm and the 5' arm are the same. When sequence of the third restriction site in the 3' arm and the 5' arm are the same this facilities the joining the 3' end of the 5' arm to the 5' end of the 3' arm when preparing the shuttle plasmid. Furthermore the shuttle vector can later be cleaved at this site to linearise the sequence and facilitate homologous recombination with the plasmid comprising the adenovirus genome, see for example Figure 5.

In one embodiment the first and last sites are utilised in generating the shuttle vector, for example as shown in the Figures.

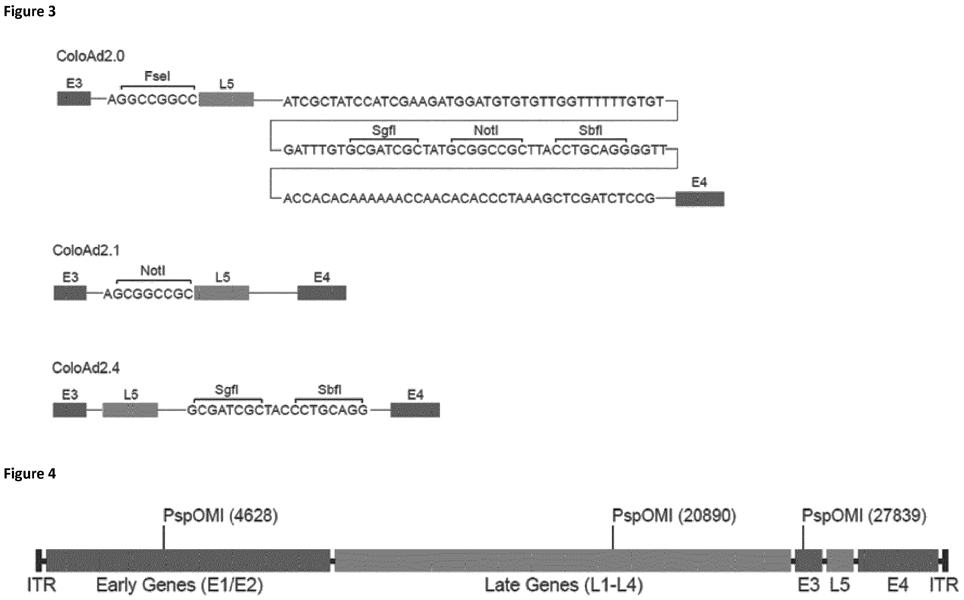

In one embodiment a restriction site is designed to be near the 5' end of the virus genome (for example EnAd genome) and also the 3' end of the virus genome, in particular about 4-5kb from the ends of the 3' and 5' ITRS such that the shuttle vector had about 4-5kb 3' and 5' arms, as shown in Figure 4. Thus in one embodiment PspOMI is the "third restriction site" in the 5' arm and/or the 3' arm. This pair of restrictions site allows the genomic DNA o be linearised and recombined with the virus genome (for example EnAd genome) to form a plasmid, without introducing any undesired alterations to the genome sequence.

This amount of DNA permits efficient homologous recombination between the shuttle vector and the virus genome, for example the EnAd genome. These design criteria also ensured the shuttle vector contains the E1 genes and the Fibre and E4 genes so that these genes and regions surrounding these genes could be manipulated for transgene cassette insertion, as desired.

In one embodiment the first and second restriction sites are the substrate for the same restriction enzyme and third restriction site is the substrate for a different restriction enzyme.

In one embodiment the first restriction site is the substrate for a different restriction enzyme to the second restriction site and the third restriction site is the substrate for a different restriction enzyme to each of the first and second restriction sites.

The present inventors have further identified the following suitable restriction sites in other adenovirus genomes.

In Ad5, the enzyme Sphl (-GCATG/C-) may be employed. The restriction sites are located at positions 3661 and 31220 in the Ad5 genome. These sites are at the end of the E1B gene and at the start of fibre gene and produce PCR fragments for shuttle vector construction of 3.6 kb and 4.7 kb. These sites therefore allow construction of a shuttle vector in which transgenes may be inserted into the E1 region or in the vicinity of the fibre gene.

In Ad3, OvAd1 and OvAd2 the site Asel (-AT/TAAT-) may be employed. The restriction sites are located at positions 2469 and 31940 in the Ad3 genome, at positions 2488 and 31724 in the OvAd1 genome and at positions 2483 and 31949 in the OvAd2 genome. For all genomes these sites are located in E1B and inside the fibre gene and produce PCR fragments for shuttle vector construction of 2.5kb and 3.4kb. These sites therefore permit construction of a shuttle vector in which transgenes may be inserted into the E1 region or in the vicinity of the fibre gene.

In Ad11 Psil (-TTA/TAA-) may be employed. Unlike PspOMl, Sphl and Asel, this enzyme is a blunt cutter. The Psil restriction sites are located at positions 2648 and 30890 in the Ad11 genome. These sites are in the E1B gene and at the start of the fibre gene and produce PCR fragments for shuttle vector construction of 2.6kb and 4kb. As above, these sites therefore allow construction of a shuttle vector into which original restriction sites may be inserted in the E1 region or in the vicinity of the fibre gene.

In the process of amplifying the 5' and 3' ends of the genome to generate the 3' arm and the 5' arm typically a linking restriction site will be added using suitable primers during PCR. The same linking restriction site is inserted into the vector fragment containing the bacterial replication of origin and the selection marker gene during amplification. This linking restriction site is designed to permit joining of the adenovirus genome to the vector fragment containing the bacterial origin of replication and selection marker gene. The first restriction enzyme site, second restriction enzyme site and third restriction enzyme site are all examples of linking restriction sites.

In one embodiment the third restriction site in the 3' arm and the 5' arm are in corresponding locations in the plasmid containing the adenovirus DNA. In one embodiment the 5' arm has a linking restriction site introduce upstream of the 5' ITR and the 3' arm has a linking restriction site introduced downstream of the 3' ITR. The restriction sites permit excision of the vector fragment comprising bacterial replication of origin and the selection marker gene. These restriction sites, alternatively known as the first and second restriction enzyme sites, permit the excision of the adenovirus genome from the plasmid.

In one embodiment a linking restriction site is AscI. Other restriction enzymes that cut at the same sequence are interchangeable.

### Fragments

**Vector fragment** in the context of the method to make the shuttle vector refers to a fragment of DNA comprising a replication of origin and selection marker. In one embodiment the vector fragment is in the region of 2 to 4 Kb long. The vector fragment starts at the 5' end of the vector fragment with a first restriction enzyme site arranged to permit ligation with the 3' end of the 3' arm. It further comprises a low copy bacterial replication of origin and a selection marker gene and terminates at the 3' end of the vector fragment with a second restriction enzyme site arranged to permit ligation with the 5' end of the 5' arm. In one embodiment the vector fragment is no more than 3Kb. This is sufficient to include a replication of origin and selection marker gene.

A **5' arm** as employed herein refers to a DNA fragment of, for example a few thousand base pair from the 5' end of the adenovirus genome including the 5' ITR (inverted terminal repeat). The precise length of the arm is determined by the location of suitable restriction sites. In one embodiment the 5' arm is in the region of about 2 to 5 Kb in length, such as about 2.1, 2.2, 2.3, 2.4, 2.5 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8 or 4.9 kb long, and contains an E1 site, for example the entire E1 gene or the site from which the E1 gene has been deleted or partially deleted. In one embodiment the 5' arm is 4627 base pair long, for example from the 5' end of the EnAd genome.

The 5' arm starts at the 5' end with a second restriction site (arranged as discussed above to permit ligation with the 3' end of the vector fragment),comprises the 5' ITR and terminates at the 3' end of the 5' arm with a third restriction site (arranged to allow ligation with the 5' end of the 3' arm).

In one embodiment the 5' arm includes a non-mutated E1 region and so can be employed in the preparation of replication competent viruses.

In one embodiment the amount of adenoviral genomic DNA in the 5' arm is minimal, such as ITR. In one embodiment the amount of adenoviral genomic DNA in the 5' arm is in the range of 100bp to 5kb. A **3' arm** as employed herein refers to a DNA fragment, for example a few thousand base pairs from the 3' end of the adenovirus genome including the 3' ITR (inverted terminal repeat). The precise length of the arm is determined by the location of suitable restriction site. In one embodiment the 3' arm is in the region of about 3 to 5 kb long such as about 3.1, 3.2, 3.3, 3.4, 3.5, 3.6, 3.7, 3.8, 3.9, 4.0, 4.1, 4.2, 4.3, 4.4, 4.5, 4.6, 4.7, 4.8 or 4.9 kb long, and contains the entire Fibre (L5) gene. In one embodiment in the 3' arm is 4482 base pairs long, for example from the 3' end of the EnAd.

The 3' arm starts at the 5' end of the 3' arm with a third restriction site (for example arranged to permit ligation with the 3' end of the 5' arm) comprises the L5 gene and 3' ITR and terminates at the 3' end with a first restriction enzyme site (for example arranged to permit ligation with the 5' end of the vector fragment).

In one embodiment the 3' arm contains the fibre gene, also known as L5. Advantageously including the fibre gene in the 3' arm allows the manipulation of the late genes and more flexibility in the engineering of the virus.

"In a location between" as employed herein refers to being bordered or sandwiched by the regions specified, for example any location between the last nucleic acid of the one specified region, such as the E3 site and the first nucleic acid of the second specified regions, such as the L5 gene. First nucleic acid in this context includes the first nucleic acid of a fragment of the relevant gene/region (i.e. is not limited the literal first nucleic acid of the full length gene but rather refers to the first occurring nucleic assigned to the gene in a given construct). In one embodiment the location is between the last nucleic acid of the L5 gene and the first nucleic acid of the E4 site when travelling in a 5' to 3' direction.

In the vicinity of the L5 gene as employed herein refers to a location between the E3 site and the L5 gene and/or a location a location between the L5 gene and the E4 site. In one embodiment one or more restriction sites are independently located or inserted in a non-coding region. In one embodiment the genetic material inserted does not change or interrupt the function of the L5 gene.

It is particularly advantageous to insert one or more transgenes into the adenovirus genome in the vicinity of the L5 gene because transgenes in this location are less likely to interfere with viral stability and replication. In one embodiment genes placed after L5 may be under the control of the major late promoter or under the control of the E4 promoter. In one embodiment genes placed after L5 may be under the control of an exogenous promoter. In one embodiment genes placed before L5 may be under the control of the major late promoter or the E3 promoter. In one embodiment genes placed directly before L5 start codon can be under the control of the major late promoter and will generally need to contain a regulatory element that allows the expression of L5.

In some circumstances, inserting the transgene abutted to the fibre gene, for example where the transgene is directly next to or within a few bases of the L5 gene, may be advantageous. In one embodiment is abutted to the L5 gene on the E3 side of the genome and, for example permits a different level of transgene expression compare to the post-fibre site.

In one embodiment locating the transgene gene adjacent to L5 gene also allows regulation of the expression of the fibre protein which in turn allows regulations of virus activity.

Thus in one embodiment the transgene is inserted in a location abutted to the fibre gene, for example on the E3 side of the genome, on the E4 side of the genome or both.

In one embodiment a transgene or transgene are independently inserted in the same direction as the genomic DNA. In one embodiment the transgene or transgenes are independently inserted in a direction opposing the genomic DNA.

In one embodiment the 3' arm comprises in the 5' to 3' direction: a fibre gene; followed by an E4 site, for example an E4 region or a fragment thereof; and followed in turn by the ITR (inverted repeat region).

In one embodiment the 3' arm comprises in the 5' to 3; direction: an E3 site, for example an E3 region, a fragment thereof or an E3 site where the E3 region is deleted; followed by a fibre gene; followed in turn by an E4 region or a fragment thereof also known as the E4 site, in turn followed by the ITR. In one embodiment the 3' arm is in the range of 2Kb to 5Kb. In one embodiment adjacent to the fibre gene is a restriction site, for example Fsel, in particular on the E3 side of the fibre. In one embodiment adjacent to the fibre gene is a restriction site, for example Sgfl and/or Sbf1, for example on the E4 side of the fibre gene. In one embodiment the fibre gene is sandwiched by two restriction site, for example Fsel, in particular on the E3 side of the fibre and an Sgfl and/or Sbf1 is located, for example on the E4 side of the fibre gene.

In one embodiment the E4 site further comprises an Acll site, for example at a location disclosed in the Figures, examples and/or sequences herein.

In one embodiment the 5' arm and the 3' arm are each of sufficient lenght to promote efficient homologous recombination, such as approximately 2Kb to 5Kb long.

The 5' arm and/or the 3' arm may be synthetic. Advantageously, using synthetic 5' and/or 3' arms permits the introduction of original restrictions sites into the arms prior to ligation to form a shuttle vector.

In one embodiment in a shuttle vector according to the present disclosure (for example an EnAd shuttle vector) PspOMI is the third restriction enzyme site. PspOMI as employed herein refers to a restriction site cut by a restriction enzyme at the following sequence -G/GGCCC-. Other restriction enzymes such as Apal and Bsp120I, that cut at the same sequence are interchangeable.

In one embodiment there is provided a method of introducing one or more original restriction sites into an adenovirus shuttle vector comprises the steps:
a) identifying two excision restriction sites in the shuttle vector and the DNA sequence between the sites,
b) digesting the shuttle vector at the excision restriction sites identified in step a) to excise a section of DNA,
c) synthesising a DNA fragment wherein the DNA fragment is substantially identical to the excised section of the shuttle vector from step b) further comprising one or more original restriction sites,
d) purifying the digested shuttle vector from step b) and the DNA fragment from step c),
e) ligating the DNA fragment from step d) and the shuttle vector from step d), and
f) identifying a correctly assembled shuttle vectors by transforming cells with the ligated shuttle vector, growing on a selective medium and screening the colonies.

In one embodiment the excision restriction site is AclI. Other restriction enzymes that cut at the same sequence are interchangeable. Acll as employed herein means a restriction site cut by a restriction enzyme at the following sequence -AA/CGTT-.

Acll is used alongside PspOMI to introduce original restriction sites into ColoAd1 shuttle vectors because they flank the fibre gene allowing excision of this region and insertion of a DNA fragment that is substantially identical to the excised region.

### Ligation

One-step three-way ligation as employed herein refers to three sequences of DNA being ligated together in a single step to form circular DNA. In one embodiment screening is performed to establish the DNA shuttle vector is made up of all three sequences in an appropriate orientation.

Appropriate orientation as employed herein is intended to refer to an orientation suitable for use in reassembling/constructing an adenovirus according to the present disclosure.

Equal proportions as employed herein refers to ratio of DNA fragments (sequences) employed are approximately the same, even where the overall volume or concentration is variedEqual proportions refers to a 1:1:1 ratio of the vector fragment, 5' arm and the 3' arm. Ligation ratio as employed herein means the ratio or proportion at which the fragments of DNA to be ligated are provided.

In one embodiment, where the first and second restriction enzymes employed in each of the fragments are the same, and the vector fragment is dephosphorylated prior to the one-step, three-way ligation.

Dephosphorylated as employed herein refers to the phosphate (PO₄³⁻) group is removed from the DNA by hydrolysis. Advantageously, dephosphorylation increases the likelihood of the 5' end and the 3' end of a DNA fragment remaining free to ligate with a different DNA fragment (which is typically not dephosphorylated) as opposed to ligating to each other. In one embodiment the dephosphorylation increases the success of a subsequent ligation step.

Ligation or ligating as employed herein refers to the covalent linking of two ends of DNA molecules, for example using a ligase enzyme.

In one embodiment the one-step three-way ligation uses 2 parts DNA ligase, 4 parts ligase buffer and 2 parts of each of the 5' arm, 3' arm and vector fragment.

Part as employed herein refers to a quantity such as a volume, the absolute amount of which can be varied provided the overall proportion in a mixture remains the same, by way of example only where 1 part is 10mls then 20mls of DNA ligase, 40mls of ligase buffer and 20mls of the 5' arm and 20mls of the 3' arm will be employed.

In one embodiment about 2µl of eluted DNA contains about 40ng of DNA. In one embodiment about 120ng of DNA are ligated. In one embodiment the 120ng of DNA ligated consists of about 40ng each of the 5' arm the 3' arm and the vector fragment.

DNA ligase as employed herein refers to an enzyme that ligates (facilitates the joining) of DNA molecules by catalysing the formation of the phosphodiester bond. In one embodiment the DNA ligase is T4 DNA ligase. T4 DNA ligase is an enzyme derived from bacteriophage T4.

Ligase buffer as employed herein refers to a buffered solution, for example containing magnesium chloride and ATP, such as 50mM Tris-Hcl, 10mM MgCl₂ and 1mM ATP.

Circularised or circular as employed herein refers to the ends of a linear piece of DNA being joined to form a circle or loop.

In one embodiment the one-step three-way ligation is performed for at least 30 minutes, for example at least 50 minutes, such as about 1 hour, more specifically 51, 52, 53, 54, 55, 56, 57, 58, 59, 60, 61, 62, 63, 64, 65, 66, 67, 68, 69 or 70 minutes.

In one embodiment the one-step three-way ligation is performed at approximately room temperature, such as about 15 to 25°C, such as 16, 17, 18, 19, 20, 21, 22, 23, or 24°C. Preferably 16 to 24°C.

In one embodiment the method of making an adenovirus shuttle vector comprising the steps:
a) Identifying a suitable restriction sites in the adenovirus genome,
b) creating a 5' PCR arm and a 3' PCR arm, for example by amplifying the 5' end and the 3' end of the genome and inserting a linking restriction site at each terminal end upstream of the ITR therein,
c) selecting and amplifying a vector fragment with a low copy bacterial origin of replication and a selection marker gene and terminal linking restriction sites and terminating in linking restriction sites that permit ligation to the 5' arm and the 3' arm,
d) double digesting the 5' PCR arm and the 3' PCR arm from step b) for about 2 hours at about 37°C, wherein the double digest takes place in buffer, restriction enzymes and nuclease free water followed by heat inactivation at about 65°C for about 20 minutes to obtain the 5' arm and the 3' arm,
e) single digesting the vector fragment from step b) for about 2 hours at about 37°C, wherein the single digest takes place in buffer, restriction enzyme and nuclease free water followed by treatment with alkaline calf phosphatase at about 37°C for about 1 hour
f) separating the entire volume of the digest products of steps d) and e) on about 0.8% agarose gel, gel purifying and eluting in elution buffer,
g) ligating the digested 3' arm, 5' arm and vector fragment from step f) in a one-step three-way ligation using DNA ligase and ligase buffer for about 1 hour at room temperature at a 1:1:1 ligation ratio of the 5' arm, 3' arm and vector fragment, and
h) identifying a correctly assembled shuttle vectors by transforming cells with the ligated shuttle vector, growing on a selective medium and screening the colonies.

In one embodiment step a) in the method above is optional and the 3' arm and/5'arm is synthesised with the required restriction site and/or transgene.

In one embodiment the method of making an EnAd shuttle vector comprising the steps:
a) identifying suitable restriction sites in the adenovirus genome,
b) creating a 5' PCR arm and a 3' PCR arm by amplifying the 5' end and the 3' end of the genome and inserting a linking restriction site at each terminal end upstream of the ITR,
c) selecting and amplifying a vector fragment with a low copy bacterial origin of replication and a selection marker gene and terminal linking restriction sites and terminating in linking restriction sites that permit ligation to the 5' arm and the 3' arm,
d) double digesting the 5' PCR arm and the 3' PCR arm for 2 hours at 37°C at using 20µl DNA from step b), 4µl of buffer 4, 2µl of Ascl, 2µl of PspOM1 and 8µl of nuclease free water or equivalents thereof followed by heat inactivation at 65°C for 20 minutes to obtain the 5' arm and the 3' arm,
e) single digesting the vector fragment for 2 hours at 37°C at a concentration of 20µl DNA from step b), 4µl of buffer 4, 2µl of Ascl and 8µl of nuclease free water or equivalents thereof followed by treatment with 1µl alkaline calf phosphatase at 37°C for 1 hour,
f) separating the entire volume of the digest products of steps d) and e) on 0.8% agarose gel, gel purifying and eluting in 40µl of elution buffer or the equivalent thereof,
g) ligating the digested 3' arm, 5' arm and vector fragment from step f) in a one-step three-way ligation using 2µl T4 DNA ligase and 4µl ligase buffer for 1 hour at room temperature at a 1:1:1 ligation ratio at 2µl of each of the 5' arm, 3' arm and vector, and
h) identifying a correctly assembled shuttle vectors by transforming cells with the ligated shuttle vector, growing on a selective medium and screening the colonies.

In one embodiment step a) in the method above is optional and the 3' arm and/5'arm is synthesised with the required restriction site and/or transgene.

The skilled person will appreciate that alternate buffers, ligase and volumes may be used and that the proportions are important rather than absolute volume.

Amplifying as employed herein means the process of increasing a single or a few copies of a piece of DNA, for example across several orders of magnitude, generating thousands to millions of copies of a particular DNA sequence, typically using PCR.

Single digest as employed herein means digesting the DNA with a single restriction enzyme known to target a given site in the DNA sequence.

Double digest as employed herein means digesting the DNA with two different restriction enzymes known to target different sites in the DNA sequence.

Correctly assembled as employed herein means the pieces of DNA have been ligated in the correct orientation and position as intended to provide the shuttle vector desired.

Transforming as employed herein means the genetic alteration of a cell resulting from the direct uptake, incorporation and expression of exogenous genetic material (exogenous DNA) from its surroundings.

Growing as employed herein means culturing as commonly understood by the skilled person.

Screening as employed herein means identifying cells and/or DNA constructs with the desired properties. Typical methods employed include, but are not limited to, PCR using primers that span restriction sites, restriction digests, DNA sequencing.

Upstream as employed herein means before in the direction of reading (i.e. 5' to 3'). Downstream as employed herein means after in the direction of reading (i.e. 5' to 3').

Excision as employed herein means removal of.

In one embodiment the plasmid or shuttle vector further comprises one or more polyadenylation sequences that have been introduced to the DNA construct.

Synthetic as employed herein refers to, for example DNA fragment was made by synthetic chemistry techniques, such as in an automated synthesis.

DNA fragment as employed herein refers to a piece of DNA, in particular a portion of adenovirus genomic DNA or a DNA sequence obtained after manipulation by a method disclosed herein. In one embodiment silent base changes are tolerated.

Silent base changes as employed herein refers to a nucleotide change that does not affect the amino acid sequence encoded due to the redundancy of the genetic code.

Substantially identical as employed herein means the DNA fragment is identical to a piece of shuttle vector save for the addition of one or more novel restriction sites.

Purifying as employed herein refers to decontaminating DNA interest, for example by removing DNA which is not of interest.

In one embodiment the method comprises a further step of propagating a shuttle vector of the present disclosure, for example to obtain greater quantities of it.

In one embodiment ligated DNA is transformed into a bacterial cell or cells, for example about 120ng of ligated DNA is transformed. In one embodiment the bacterial cells are ultracompetent bacterial cells, such as XL-10 available from Agilent. Alternatively the plasmid prepared by homologous recombination with the shuttle vector may be replicated in, for example *E. coli,* and thus the need to repeat the assembly of the shuttle vector is avoided.

### Replacing a DNA fragment in the Shuttle Vector

In one embodiment the method comprises the step of ligating a DNA fragment into the shuttle vector, for example after excision of a section of the same, in particular to introduce restriction sites and/or transgenes. In one embodiment the ratio employed is 3:1 DNA fragment to shuttle vector ligation respectively. 3:1 fragment to shuttle vector ligation ratio as employed herein means 3 parts of DNA fragment to 1 part shuttle vector.

In one embodiment the DNA fragment is synthetic or a PCR product. PCR product as employed herein means the DNA fragment was made using PCR.

Further details of modifying the shuttle vector are given in the examples and figures.

Thus, the present disclosure provides methods for making for the first time an adenovirus shuttle vectors and plasmids that is essentially modular and allows the skilled person to manipulate the adenovirus genome at will.

### Preparing the Plasmid

Plasmid as employed herein refers to a small DNA molecule that is physically separate from, and can replicate independently of, chromosomal DNA within a cell. For the purpose of the present disclosure a plasmid is a circular DNA vector generally comprising substantially all of the adenovirus genome joined to a vector fragment comprising a bacterial origin of replication and a selection marker gene (see Figure 2).

Generally, the plasmid according to the present disclosure is prepared by homologous recombination of the linearised shuttle vector with an adenovirus genome. Adenovirus genome in this context refers to all or almost all the adenovirus genome. The skilled person will know that this step is part of reassembling the adenovirus and generally is a precursor to realising the adenovirus construct made by the present disclosure. Thus, the adenovirus genome employed in the recombination step generally comprises all the functional elements, except perhaps a transgene, that will be in the final adenovirus. In one embodiment the method of performing homologous recombination comprises the steps:
a) linearising the shuttle vector by digesting at the third restriction enzyme site,
b) performing homologous recombination between the linearised shuttle vector and the adenovirus genome in electrocompetent cells,
c) identifying a correctly recombined plasmids by growing the cells on a selective medium and screening the colonies.

Linearising as employed herein refers to the process of making circular DNA into linear DNA, typically by digesting the DNA construct with a single restriction enzyme.

In one embodiment the homologous recombination is performed at a 3.5:1.5 shuttle vector to genome ratio.

Electrocompetent as employed herein means cells that are transformed by means of electroporation. Suitable electrocompetent cells include, but are not limited to, BJ5183 cells.

Correctly recombined as employed herein means the plasmids that have undergone homologous recombination have obtained the desired DNA sequence.

In one embodiment the method comprises the further step of introducing a transgene cassette into the plasmid at a restriction site of interest. Restriction site of interest refers to a site identified as occurring in the genome at a location amenable to the insertion of a transgene cassette. Typically restriction sites of interest are the original restriction sites introduced into the shuttle vector and/or plasmid by the method disclosed herein.

In one embodiment the step of introducing a transgene cassette into a plasmid comprises performing a ligation between linearised plasmid and transgene cassette.

In one embodiment the step of introducing a transgene cassette into the plasmid comprises:
- linearising a plasmid and the transgene cassette,
- separating the linearised DNA,
- purifying the separated linearised DNA,
- ligating the plasmid and transgene cassette,
- transforming the ligated DNA, for example into *E. coli,* and
- selecting correctly transformed colonies.

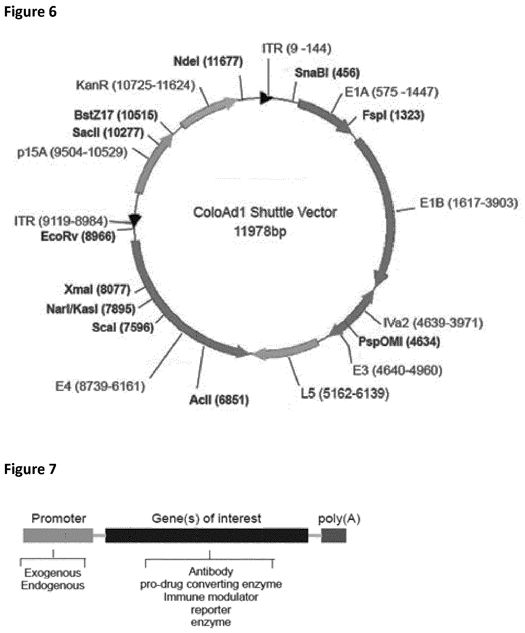

Transgene cassette as employed herein refers to, for example a segment of DNA optionally containing a promoter, which is a regulatory sequence that will determine where and when the transgene is active, or a splice site which is a regulatory sequence determining when a mRNA molecule will be cleaved by the splicesome, a protein coding sequence (i.e. the transgene), usually derived from the cDNA for the protein of interest, optionally containing a polyA sequence and a stop sequence. Figure 7 represents a generic transgene cassette.

In one embodiment the transgene cassette comprises an exogenous promoter, for example a mammalian promoter.

In one embodiment the transgene cassette comprises a regulatory element such as internal ribosome entry sequence.

In one embodiment the transgene cassette comprises a polyA sequence.

In one embodiment, the virus or viral vector can be recovered from the plasmid by cutting the genome with restriction enzymes at the 3' and 5' ends of the sequence to excise the vector fragment DNA comprising the origin of replication and selection marker gene. In one embodiment the same restriction enzyme cuts both sites. The linearised DNA can then be inserted into a suitable host cells, such as a HEK293 cell to generate the final virus or viral vector.

### Transgene

In one embodiment one or more transgenes in the transgene cassette are selected from:
a therapeutic gene of interest which encodes a therapeutic protein, peptide or RNA such as an antibody or antibody domain, pro-drug converting enzyme, immunomodulator, enzyme, siRNA, transcription factor, intracellular signalling or surface membrane protein, or antigen.

Antibody as employed herein means a large Y-shaped protein produced by B-cells that is used by the immune system to identify and neutralize foreign pathogens, such as bacteria and viruses. The antibody recognises a unique part of the foreign antigen. A wide range of different forms of antibody may be employed including monoclonal antibodies, polyclonal antibodies, diabodies, chimeric antibodies, humanised antibodies, bi- and tri-specific antibodies, camalid antibodies, Fab fragments, Fc fragments and Fv molecules, including single-chain Fv (ScFv) antibodies.

Pro-drug as employed herein refers to a molecule that is administered as an inactive (or less than fully active) derivative that is subsequently converted to an active pharmacological agent in the body, often through normal metabolic processes or by use of an appropriate enzyme. Thus a pro-drug serves as a type of precursor to the intended drug. A pro-drug converting enzyme serves as the enzyme that converts a pro-drug to its pharmacologically active form. In one embodiment a viral construct according to thepresent disclosure encode and expresses *in vivo* a pro-drug and an appropriate converting enzyme.

Immunomodulator as employed herein means a modulator of an immune response. Immunomodulators function in adjusting the quality and quantity of the immune response in a desired direction or to a desired level, as in immunopotentiation, immunosuppression, or induction of immunologic tolerance. In one embodiment the immunomodulator is a immunostimulant, for example an adjuvant, such as a DNA sequence rich in CpG.

Enzyme as employed herein refers to a protein suitable for catalyse a specific chemical reaction, for example regulating the rate at which chemical reactions proceed without itself being altered in the process. In one embodiment the enzyme is capable of catalysing a reaction in a living organisms.

Reporter gene as employed herein refers to a gene that produces a product easily detected in eukaryotic cells and may be used as a marker to determine the presence or activity the gene of interest. In one embodiment the reporter gene DNA is closely linked or combined with the DNA sequence of interest. In one embodiment the reporter gene is, for example, luciferase and fluorescent protein such as GFP.

In one embodiment the transgene or genes encode reporter genes for *in vitro* imaging and localisation such as, but not limited to: Sodium iodide symporter (NIS), intracellular metalloproteins (e.g. ferritin, tyrosinase), herpes simplex virus type 1 thymidine kinase (HSV1-tk), GFP and other fluorophores, luciferase, estrogen receptor and other inducible reporter genes.

In one embodiment the transgene is not a reporter gene or imaging agent, such as luciferase or eGFP. Exogenous mammalian promoter as employed herein refers to a DNA element, usually located upstream of the gene of interest, that regulates the transcription of a gene.

Regulatory element as employed herein refers to a DNA sequence that either permits use of endogenous promoters, such as the major late promoter, or other DNA sequence that regulates expression, for example expression of multiple genes, such as polycystronic sequences.

Internal ribosome entry sequence (IRES) as employed herein refers to a nucleotide sequence that allows for translation initiation in the middle of a messenger RNA (mRNA) sequence.

PolyA sequence as employed herein refers to a DNA sequence, usually containing an AATAAA site, that once transcribed can be recognised by a multiprotein complex that cleaves and polyadenylates the nascent mRNA molecule.

(Therapeutic) gene of interest as employed herein means a gene suitable of for generating a pharmacological effect (generally a beneficial pharmacological effect), for example after expression, in particular the activity may enhance the utility or therapy of the DNA construct or the adenovirus of the present disclosure, such as oncolytic virus activity.

A range of different types of transgene (and combinations thereof) are envisaged that encode molecules that themselves act to modulate tumour or immune responses and act therapeutically, or are agents that directly or indirectly inhibit, activate or enhance the activity of therapeutic molecules. Molecules, which may be encoded include protein ligands or active binding fragments of ligands, antibodies (for example, full length or fragments, such as Fv, ScFv, Fab, F(ab)'2 or smaller specific binding fragments), or other target-specific binding proteins or peptides (e.g. as may be selected by techniques such as phage display etc), natural or synthetic binding receptors, ligands or fragments, specific molecules regulating the transcription or translation of genes encoding the targets, such as siRNA or shRNA molecules, transcription factors and the like. Molecules may be in the form of fusion proteins with other peptide sequences, for example to enhance their activity, stability, specificity etc. In one embodiment, ligands may be fused with immunoglobulin Fc regions to form dimers and enhance stability or provide effector function. In one embodiment the transgene may encode a fusion protein, for example with an entity fused to antibodies or antibody fragments having specificity to antigen presenting cells such as dendritic cells e.g. anti-DEC-205, anti-mannose receptor, anti-dectin.

In one embodiment inserts may also encode reporter genes that can be used, for example to detect cells infected with the adenoviruses or adenoviral vectors according to the invention, imaging of tumours or draining lymphatics and lymph nodes etc.

In one embodiment proteins encoded are human, for example human antibodies or natural ligands to molecules listed below, or siRNA molecules targeting them, or tumour antigens.

In one embodiment the transgene or genes encode T-cell costimulatory receptors or their ligands such as, but not limited to: OX40, OX40 ligand, CD27, CD28, CD30, CD40, CD40 Ligand (CD40L), CD137, GITR, 4-1BB, ICOS, ICOS ligand.

In one embodiment the transgene or genes encode T-cell co-inhibitory molecules or their ligands (checkpoint inhibitory receptors and ligands) such as, but not limited to: Cytotoxic T lymphocyte associated antigen-4 (CTLA-4), programmed cell death-1 (PD-1), PD-Ligand-1 (PD-L1, also known as B7-H1), PD-Ligand-2 (PD-L2, also known as B7-DC), other B7 receptor superfamily members such as B7-H3, B7-H4, herpes virus entry mediator (HVEM), inhibitory receptor Ig-like transcript-3 (ILT-2), ILT-3, ILT-4, T-cell immunoglobulin mucin protein-3 (TIM-3), lymphocyte activation gene-3 (LAG-3), B and T lymphocyte attenuator (BTLA), LIGHT (homologous to lymphotoxin, exhibits inducible expression, and competes with HSV glycoprotein D for herpes virus entry mediator, a receptor expressed by T lymphocytes), CD160.

In one embodiment the transgene or genes encode molecules expressed by regulatory T-cells (natural and induced Tregs, Tr1 etc), Myeloid derived suppressor cells (MDSCs) & other Immunosuppressive Immune cells such as, but not limited to: CD16, CD25, CD33, CD332, CD127, CD31, CD43, CD44, CD162, CD301a, CD301b, and Galectin-3.

In one embodiment the transgene or genes encode dendritic cell (and other antigen-presenting cell) receptors or their ligands such as, but not limited to: Fms-related tyrosine kinase 3 (FLT-3), FLT-3 ligand, Toll-like receptors (TLR) and their ligands (e.g. TLR-9, flagellin as ligand for TLR-5), CCR7 (CD197), CD1a, CD1c (BDCA-1), CD11b, CD11c, CD80 (B7-1), CD83, CD86 (B7-2), CD123 (IL-3Rα), CD172a (SIRPα), CD205 (DEC205), CD207 (Langerin), CD209 (DC-SIGN), CD273 (B7-DC), CD281 (TLR1), CD283 (TLR3), CD286 (TLR6), CD289 (TLR9), CD287 (TLR7), CXCR4 (CD184), GITR Ligand, IFN-α2, IL-12, IL-23, ILT1 (CD85h), ILT2 (CD85j), ILT3 (CD85k), ILT4 (CD85d), 27D6 5148, 42D1 5149, ILT5 (CD85a), ILT7 (CD85g), TSLP Receptor, CD141 (BDCA-3), CD303 (CLEC4c, BDCA-2), CADM1 (NECL2), CLEC9a, XCR1, CD304 (Neuropilin-1, BDCA-4).

In one embodiment the transgene or genes encode antigen processing and presentation mediators such as, but not limited to: MHC Class II transactivator (CTIIA), Gamma-IFN-inducible lysosomal thiol reductase (GILT).

In one embodiment the transgene or genes encode cytokines or their receptors such as, but not limited to: Interleukin-1α (IL-1α), IL-1β, IL-6, IL-9, IL-12, IL-13, IL-17, IL-18, IL-22, IL-23, IL-24, IL-25, IL-26, IL-27, IL-33, IL-35. Interleukin-2 (IL-2), IL-4, IL-5, IL-7, IL-10, IL-15, IL-21, IL-25, IL-1 receptor antagonist (IL-1RA), interferon-α (IFNα), interferon-β ( IFNβ), interferon γ (IFNγ), tumour necrosis factor-α (TNFα), transforming growth factor-β (TGFβ - different subtypes), granulocyte macrophage colony stimulating factor (GM-CSF).

In one embodiment the transgene or genes encode chemokines or chemokine receptors such as, but not limited to: Interleukin-8 (IL-8), CCL5 (RANTES), CCL17, CCL22, CCL20, CXCL9, CXCL10, CXCL11, CXCL13, CCR2, CCR4, CCR5, CCR6, CCR7, CCR8, CXCR3, CXCR4, CXCR5, CRTH2.

In one embodiment the transgene or genes encode transcription factors or other regulators of transcription such as, but not limited to: STAT3, STAT1, STAT4, STAT6, CTIIA, MyD88, NFκB family members.

In one embodiment the transgene or genes encode pro-drug converting enzymes or other enzymes such as, but not limited to: cytosine deaminase and tyrosine kinases.

In one embodiment the transgene or genes encode intracellular trafficking molecules or regulators of cell function such as, but not limited to: Heat shock protein-70 (HSp70), regulators of cell survival and death (e.g. survivin).

In one embodiment the transgene or genes encode tumour cell or tumour microenvironmental receptors and products such as, but not limited to: EGF Ligands & receptors: amphiregulin, betacelluin (BTC), neuroregulin-1a (NRG1a), NRG1b, NRG3, transforming growth factor-a (TGFa), LRIG1 (leucine-rich repeat and Ig-like domain-containing-1), LRIG3, EGF, EGF-L6, Epigen, HB-EGF, EGFR (ErbB1), Her2 (ErbB2, Her3 (ErbB3), Her4 (ErbB4).

In one embodiment the transgene or genes are ligands & receptors for families of molecules such as, but not limited to: hedgehog, FGF, IGF, Wnt, VEGF, TNF, TGFb, PDGF, Notch.

In one embodiment the transgene or genes encode intracellular tumour cell enzymes such as but not limited to: Indoleamine 2,3 dioxygenase (IDO).

In one embodiment the transgene or genes encode antigens for recognition by immune cells such as but not limited to: Foreign immunogenic proteins from infectious organisms as antigens (e.g. cytomegalovirus antigens, influenza antigens, hepatitis B surface and core antigens, diphtheria toxoid, Crm197, tetanus toxoid), peptides derived from such antigens which are known T-cell or antibody epitopes, or genetically engineered composites or multimers of such antigens.

In one embodiment the transgene or genes encode tumour-derived proteins as antigens, peptides derived from such antigens which are known T-cell or antibody epitopes, or genetically engineered composites or multimers of such antigens. Such antigens could include, for example, WT1, MUC1, LMP2, Idiotype, HPV E6&E7, EGFRvIII, HER-2/neu, MAGE A3, p53 nonmutant, p53 mutant, NY-ESO-1, GD2, PSMA, PCSA, PSA, gp100, CEA, MelanA/MART1, Ras mutant, Proteinase3 (PR1), bcr-abl, Tyrosinase, Survivin, PSA, hTERT, Sarcoma translocation breakpoints, EphA2 PAP ML-IAP AFP EpCAM, ERG (TMPRSS2 ETS fusion gene), NA17, PAX3, ALK, Androgen receptor, Cyclin B1, Polysialic acid, MYCN, RhoC, TRP-2, GD3, Fucosyl GM1, Mesothelin, PSCA, MAGE A1, sLe(a), CYP1B1, PLAC1, GM3, BORIS, Tn, GloboH, ETV6-AML, NY-BR-1, RGSS, SART3, STn, Carbonic anhydrase IX, PAXS, OY-TES1, Sperm protein 17, LCK, HMWMAA, AKAP-4, SSX2, XAGE 1, B7H3, Legumain, Tie 2, Page4, VEGFR2, MAD-CT-1, FAP, PDGFR-β, MAD-CT-2, Fos-related antigen 1 (Cheever et al 2009).

As employed herein pNG-62 means a plasmid comprising ColoAd2.4 genome and a transgene cassette comprising a gene encoding GFP (see SEQ ID NO: 35, Figure 30).

### Preparing Adenoviruses

The present disclosure also provides a method of generating recombinant adenoviruses wherein the early genes, for example those essential for virus replication, particularly E1 and/or E3 can remain intact or an adenoviral vector where genes essential to viral replication are deleted according to the intended purpose. Thus, the disclosure facilitates the genetic engineering of live oncolytic viruses, for example the ability to arm them with therapeutic proteins in regions that was not previously possible. The final adenovirus according to the present disclosure may be provided excising the genomic DNA of the from the plasmid with appropriate restriction enzyme, followed by, for example a ligation step to complete, circularise the genome. Thus a virus or viral vector may be recovered from the plasmid by cutting the genome with restriction enzymes at the 3' and 5' ends of the sequence to excise the vector fragment DNA comprising the origin of replication and selection marker gene. In one embodiment the same restriction enzyme cuts both sites. The linearised DNA can then be inserted into a suitable host cells, such as a HEK293 cell to generate the final virus or viral vector.

### Shuttle Vectors, Plasmids and Adenoviruses

Shuttle vectors of the present disclosure comprise 3' arm contain the virus fibre gene. Details of the shuttle vector given above in relation the method. Shuttle vectors of the present disclosure contain a relatively large amount of adenovirus genomic DNA, for example in range of about 9Kb to about 11.9Kb. In one embodiment 50% or more, for example 60, 70, 75 or 80% of the shuttle vector is adenovirus genomic DNA. In one embodiment the shuttle vector in total is about 15Kb or less, for example 14, 13 or 12Kb. In contrast, the major part of prior art vectors is the transgene and regulatory elements.

This is because the shuttle vector as employed in the present disclosure does not, for example comprise a transgene and/or any regulatory elements associated with a transgene (for example promoters/ IRES sequences) because it was developed for introducing mechanisms into the viral genome to facilitate flexible genetic engineering of the virus, as opposed to simply introducing one transgene into the virus.

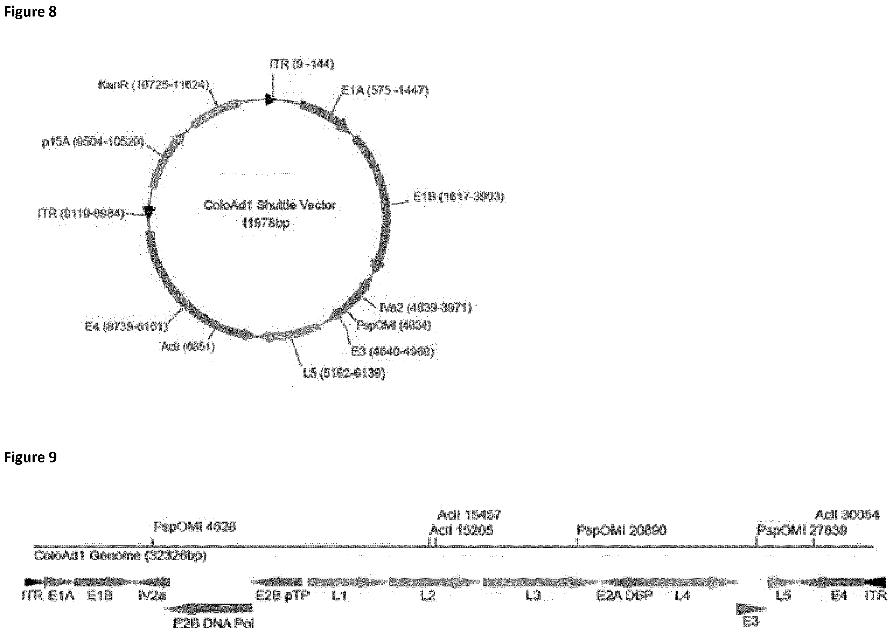

The shuttle vector is, for example a circular DNA shuttle vector comprising a 5' arm of an adenovirus genome linked directly to a 3' arm of the adenovirus genome via a suitable restriction site, (described above as the third restriction enzyme site) such that the "middle sequence" of the adenovirus genome is absent. The 3' arm and the 5' arm together are referred to herein as the major fragment of the shuttle vector The 3' and 5' ends of the major fragment are in turn joined to a vector fragment containing a low copy bacterial origin of replication and a selection marker gene via a linking restriction site (see Figures 6 and 8).

Alternatively, as discussed above the shuttle vector may comprise one or more transgenes, for example in the 3' arm of the shuttle vector.

The shuttle vector may further comprises a 5' arm. The shuttle vector may, for example comprise at least one original restriction site, for example that has been introduced to the genome using standard techniques with which the skilled person is familiar.

Advantageously the new plasmids made according to the present disclosure provide novel, original restrictions sites that are in the vicinity of the late-expressed L5 fibre gene. Insertion of transgenes in this location is less likely to interfere with viral stability and replication.

Adenoviruses or viral vectors may contain a small transgenes, for example capable of being inserted without deleting any of the genome. In one embodiment adenoviruses or adenoviral vectors may contain small transgenes capable of being inserted without deleting any of the genome, for example one or more genes which in total are 4.5kb or less.

The adenovirus or viral vector made by the method or from the plasmid according to the present disclosure may contain larger transgenes. In one embodiment a replication competent adenoviruses has additional space (such as EnAd) in the genome, for example because a gene which non-essential to replication is removed, for example part or all of the E3 and/or E4orf4 region is deleted.

Non-replicating adenovirus (i.e. those that need a packaging cell line to replicate) generally have one or more genes, for example early genes deleted, for example part or all of one or more genes independently selected from E1, E2, E3 and E4 (such as E4 orf 4).

Engineered virus is, for example a live replication competent virus or a replication deficient viral vector. Viral vector as employed herein refers to a DNA molecule used as a vehicle to artificially carry genetic material, for example a transgene, into another cell such as a mammalian cell where it can be replicated and/or expressed.

The engineered adenovirus construct made according to the present disclosure comprises one or more transgenes.

Part or all of the E4 gene is deleted in a virus or viral vector made according to the present disclosure, such as the E4orf4 section of the gene is deleted.

Part or all or the E3 gene is deleted in a virus or viral vector made according to the present disclosure. The E3 gene and part or all of the E4 gene can be deleted in a virus or viral vector made according to the present disclosure.

Part or all of the E2 region can be deleted in a virus or viral vector made according to the present disclosure.

The skilled person will appreciate that adenoviruses or adenoviral vectors generated from the plasmid of the invention may be used either as a therapeutic, for example an oncolytic or as a vaccine, or as a gene delivery vector where the virus cannot replicate without the aid of a packaging cell.

In one embodiment adenoviruses or adenoviral vectors made bythe present disclosure may contain small transgenes capable of being inserted without deleting any of the genome, for example one or more genes which in total are 4.5kb or less.

### Formulations and Methods of Treatment

The skilled person will appreciate that adenoviruses generated from the plasmid according to the invention can be used either as a therapeutic, for example an oncolytic or a vaccine, or as the gene delivery vector where the virus cannot replicate without the aid of a packaging cell, i.e. is a viral vector. Vaccine-based therapy as employed herein means a delivery (e.g. intramuscular, subcutaneous, intradermal, topical, sublingual, intranasal, oral, vaginal or rectal), or a series of such deliveries, of adenoviral vectors of the invention comprising transgenes that encode one or more antigens in order to induce an immune response or quantitatively and/or qualitatively modify an established immune response to the antigens for therapeutic benefit. Whereby each delivery may be formulated with or accompanied by delivery of immunomodulatory agents, for example adjuvants, immunomodulatory peptides, proteins or small molecules as will be understood by the skilled person.

The pharmaceutically acceptable excipient or carrier should not itself induce the production of antibodies harmful to the individual receiving the composition and should not be toxic. Suitable carriers may be large, slowly metabolised macromolecules such as proteins, polypeptides, liposomes, polysaccharides, polylactic acids, polyglycolic acids, polymeric amino acids, amino acid copolymers and inactive virus particles.

Pharmaceutically acceptable salts can be used, for example mineral acid salts, such as hydrochlorides, hydrobromides, phosphates and sulphates, or salts of organic acids, such as acetates, propionates, malonates and benzoates.

Pharmaceutically acceptable carriers in therapeutic compositions may additionally contain liquids such as water, saline, glycerol and ethanol. Additionally, auxiliary substances, such as wetting or emulsifying agents or pH buffering substances, may be present in such compositions. Such carriers enable the pharmaceutical compositions to be formulated as tablets, pills, dragees, capsules, liquids, gels, syrups, slurries and suspensions, for ingestion by the patient.

A thorough discussion of pharmaceutically acceptable carriers is available in Remington's Pharmaceutical Sciences (Mack Publishing Company, N.J. 1991).
the formulation may be provided as a formulation for topical administrations including inhalation.

In the context of this specification "comprising" is to be interpreted as "including".

Aspects of the invention comprising certain elements are also intended to extend to alternative embodiments "consisting" or "consisting essentially" of the relevant elements.

Where technically appropriate, embodiments of the invention may be combined.

Any embodiments specifically and explicitly recited herein may form the basis of a disclaimer either alone or in combination with one or more further embodiments.

Aspects of the present disclosure are described in the sequences and the figures, which may form the basis of an amendment. The disclosure of the figures and sequences have general application to the teaching of the present disclosure and not intended to considered as simply very specific combinations of features.

### EXAMPLES

### Ad11 genome:

| **Region** | **DNA sequence (map units)** | **No. of nucleotides/** % **of total genome** | **ORF** | **Position of ORF (nt)** |
|---|---|---|---|---|
| **ITR** | 1- 137 (0-0.38) | 137/0.38 | - | |
| **E1 A** | 480-1504 (1.4-4.3) | 1025/3.0 | 262R | 568-1147, 1232-1440 |
| | | | 231R | 568-1054, 1232-1440 |
| | | | 58R | 568-639, 1232-1336 |
| **E1 B** | 1556-3930 (4.5-11.3) | 2375/6.8 | 21K | 1610-2152 |
| | | | 55K | 1915-3399 |
| | | | IX | 3483-3902 |
| **E2 A** | 23402-21803 (67.3-62.7) | 1600/4•6 | DNA binding protein (DBP) | 23402-21846 |
| **E2 B** | 10342-3963 (29.7-11.4) | 6380/18•3 | IVa2 | 5588-5576, 5297-3964 |
| | | | Polymerase | 8435-5067 |
| | | | pTP | 10354-8438 |
| **E3** | 26867-30625 (77.2-88) | 3759/10.8 | 12.1K | 27185-27502 |
| | | | 16.1K | 27456-27851 |
| | | | 18.5K | 27836-28336 |
| | | | 20.3K | 28356-28901 |
| | | | 20.6K | 28919-29482 |
| | | | 10.3K | 29526-29801 |
| | | | 15.2K | 29806-30210 |
| | | | 15.3K | 30203-30610 |
| **E4** | 34493-31808 (99.1-91.4) | 2686/7.7 | 125R | 34413-34036 |
| | | | 130R | 33990-33601 |
| | | | 117R | 33604-33251 |
| | | | 122R | 33242-32874 |
| | | | 299R | 32971-32072 |
| **L1** | 10648-13614 (3.6-39.1) | 2967/8.5 | 55K | 10648-11814 |
| | | | pIIIa | 11840-13603 |
| **L2** | 13683-17340 (39.3-49.8) | 3658/10.5 | Penton base | 13683-15368 |
| | | | pVIII | 15380-15958 |
| | | | V | 16001-17056 |
| | | | pX | 17085-17315 |
| **L3** | 17399-21796 (50-62.6) | 4398/12.6 | pVI | 17399-18139 |
| | | | Hexon | 18255-21101 |
| | | | 23K protease | 21138-21767 |
| **L4** | 23433-27496 (67.3-79) | 4064/11.7 | 100K protein | 23433-25871 |
| | | | 33K | 25603-25921, 26090-26452 |
| | | | pVIII | 26502-27185 |
| **L5** | 30812-31797 (88.6-91.4) | 986/2.8 | Fibre | 30812-31789 |
| **L6** | 33097-33915 (95.1-97.5) | 819/2.4 | 169R agnoprotein | 33097-33606 |
| **ITR** | 34658-34794 (99.6-100) | 137/0.38 | - | |

### Example 1 Construction of the ColoAd1 shuttle vector

A 11978bp shuttle vector, referred to as 'ColoAd1 Shuttle Vector', was constructed containing a p15A bacterial origin of replication, a Kanamycin resistance cassette and 5' and 3' arms of ColoAd1 joined by a PspOMI third restriction enzyme site (see Figure 6).

Three DNA fragments were synthesised by PCR:
1) a "5' arm of ColoAd1" corresponding to the 5' 4627bp of ColoAd1 with a 5' Ascl restriction site and 3' PspOMI restriction site,
2) a "3' arm of ColoAd1" corresponding to the 3' 4482bp of the ColoAd1 genome with a 5' PspOMI restriction site and 3' Ascl restriction site, and
3) a vector fragment containing a low copy p15A origin of replication and a kanamycin resistance cassette flanked by Ascl restriction sites

PspOMI third restriction site in the 5' arm and the 3' arm was chosen as a suitable restriction site to allow the shuttle vector to be linearised and recombined with the ColoAd1 genome to form a plasmid, without introducing any undesired alterations to the genome sequence.

The restriction site was designed to be near the 5' end of the ColoAd1 genome and also the 3' end of the genome, in particular about 4-5kb from the ends of the 3' and 5' ITRS such that the shuttle vector had about 4-5kb 3' and 5' arms, as shown in Figure 4.

### Details of the Fragment Synthesis by PCR

The primers used for the PCR amplifications are listed in **Table 1:**

| **Primer ref number** | **Primer name** | **Sequence** |
|---|---|---|
| 0196 (SEQ ID NO: 3) | ColoSFVector Asc1 FWD | TTATAGGCGCGCCCTCTCTTAAGGTAGCATCGGG |
| 0197 (SEQ ID NO: 4) | ColoSFVector Asc1 REV | TTATAGGCGCGCCGCTACCTTAAGAGAGAGGTTGA |
| 0198 (SEQ ID NO: 5) | Colo FWD RVS Asc1 | TTGGCGGCGCGCCTATCTATATAATATACC |
| 0199 (SEQ ID NO: 6) | Colo FWD RVS Asc1 Nest | TTGGCGGCGCGCCTATCTA |
| 0200 (SEQ ID NO: 7) | ColoAd1 5' reverse | AATGCAAATCTGTGAGGGG |
| 0201 (SEQ ID NO: 8) | ColoAd1 3' Forward | CTTAGTGGTGTTGTGGTATTGG |

### 1) Generation of the 5' arm

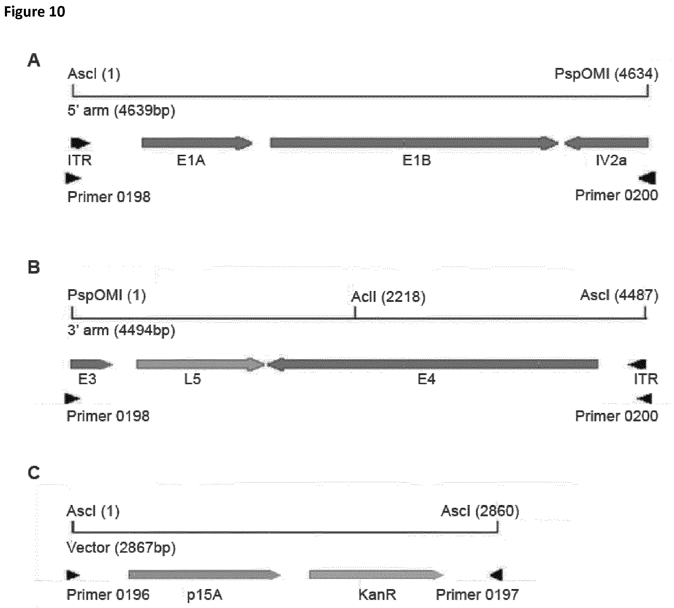

To amplify the E1A, E1B and E2B region and the 5' end of ColoAd1 a PCR was performed on native ColoAd1 using primers 0198 (SEQ ID NO.5) and 0200 (SEQ ID NO.7). A 50µl reaction volume was used for the PCR reaction, according to Table 2 below and a schematic of the PCR product is shown in Figure 10A.

**Table 2**

| **Reagent** | **Volume (µl)** | **Supplier** |
|---|---|---|
| Phusion PCR Mix | 25 | NEB MOS31S |
| Primer 0198 (SEQ ID NO: 5) (10µM) | 2.5 | Sigma |
| Primer 0200 (SEQ ID NO: 7) (10µM) | 2.5 | Sigma |
| DNA (ColoAd1) | 1 | Ark Therapeutics |
| Nuclease Free Water | 19 | Fisher Scientific (BPE 2484-100) |

### 2) Generation of the 3' arm

To amplify the E3, fibre and E4 region and the 3' end of ColoAd1 a PCR was performed on native ColoAd1 using primers 0198 (SEQ ID NO.5) and 0201 (SEQ ID NO.8). A 50µl reaction volume was used for the PCR reaction as detailed in Table 3 below and a schematic of the PCR product is shown in Figure 10B.

**Table 3:**

| **Reagent** | **Volume (µl)** | **Supplier** |
|---|---|---|
| Phusion PCR Mix | 25 | NEB MO531S |
| Primer 0198 (SEQ ID NO: 5) (10µM) | 2.5 | Sigma |
| Primer 0201 (SEQ ID NO: 8) (10µM) | 2.5 | Sigma |
| DNA (ColoAd1) | 1 | Ark Therapeutics |
| Nuclease Free Water | 19 | Fisher Scientific (BPE 2484-100) |

### 3) Generation of the vector fragment

A third PCR was performed on a vector fragment using primers 0196 (SEQ ID NO.3) and 0197 (SEQ ID NO.4) to produce a ~3kb fragment containing a p1SA origin and a Kanamycin resistance gene with 5' and 3' Ascl restriction sites (Figure 10C). A 50µl reaction volume was used for the PCR reaction, as detailed in **Table 4:**

| **Reagent** | **Volume (µl)** | **Supplier** |
|---|---|---|
| Phusion PCR Mix | 25 | NEB MO531S |
| Primer 0197 (SEQ ID NO: 4) (10µM) | 2.5 | Sigma |
| Primer 0196 (SEQ ID NO: 3) (10µM) | 2.5 | Sigma |
| DNA (P15A Vector) | 1 | Oxford Genetics |
| Nuclease Free Water | 19 | Fisher Scientific (BPE 2484-100) |

All PCR amplifications employed the following protocol **Table 5:**

| **Step no.** | **Stage** | **Temp ()** | **Time (Secs)** |
|---|---|---|---|
| Step 1 | Initial Denaturation | 98 | 60 |
| Step 2 | Denaturation | 98 | 8 |
| Step 3 | Annealing | 60 | 20 |
| Step 4 | Extension | 72 | 90 |
| Step 5 | Final Extension | 72 | 300 |
| Step 6 | Hold | 4 | Hold |

30 cycles of amplification were carried out: [Step 1] x 1, [Step 2, Step 3, Step 4] x 30, [Step 5] x 1. 1µl of each PCR product was then run on a 1% agarose gel at 150V for 1hr (Figure 11A). The entire volume of each PCR product was purified by Spin Column Method according to the manufacturer's protocol and eluted into 40µl of Elution Buffer.

The PCRs for the 5' and 3' arms were repeated in order to obtain higher amplification yields. The same programme and mix was used as detailed previously and 1µl of the product was run on a 0.8% gel at 150V for 1 hour (Figure 11B). The entire volume for each PCR product was purified by gel extraction from a 0.8% agarose gel and eluted in 40µl of elution buffer.

Following PCR amplification numerous methods were attempted to 'stick' the three PCR fragments together to form the ColoAd1 shuttle vector. One method attempted to ligate the PCR products using a two-step reaction. This method, which first ligated the 5' arm to the 3' arm followed by a second ligation reaction to ligate the vector, was unsuccessful (see below for details).

A second method attempted to ligate the three PCR products together in a one-step three-way ligation. To determine the conditions for successful ligation using this method the total amount of DNA, the ratio of the DNA fragments, the time and temperature for ligation and the phosphorylation status of the vector were varied. Several combinations were attempted unsuccessfully until a functioning methodology was achieved.

### One-step Three-Way Ligation to Form the ColoAd1 Shuttle Vector

The PCR products of the 5' arm and 3' arm (~4.6kb and ~4.5kb) were double digested using PspOMI & Ascl (see Table 6) and the PCR product of the p15a-KAN vector (~3kb) was digested with Ascl only (see Table 7), for 2hrs at 37°C according to the tables below:

### 5' arm or 3' arm Table 6:

| **Reagent** | **Volume (µl)** | **Supplier** |
|---|---|---|
| DNA (PCR product) | 20 | |
| Buffer 4 | 4 | NEB B7004S |
| PspOM 1 | 2 | NEB R0653S |
| Ascl | 2 | NEB R0558S |
| Nuclease Free Water | 8 | Fisher Scientific (BPE 2484-100) |

### p15A-Kan vector Table 7:

| **Reagent** | **Volume (µl)** | **Supplier** |
|---|---|---|
| DNA (PCR product) | 20 | |
| Buffer 4 | 4 | NEB B7004S |
| Ascl | 2 | NEB R0558S |
| Nuclease Free Water | 8 | Fisher Scientific (BPE 2484-100) |

The 5' and 3' arm digests were heat inactivated at 65°C for 20mins. The p15A-Kan vector fragment was treated with 1µl Alkaline Calf Phosphatase (CIP) for 1hr at 37°C.

1µl of all the treated restriction digests were run on a 0.8% agarose gel (Figure 12). The entire volumes of digest products were separated on a 0.8% agarose gel, gel purified and eluted in 40 µl of elution buffer.

A one-step three way ligation was performed with the purified digest products employing 2µl T4 DNA ligase and 4µl ligase buffer for 1hr at RT: with various ratios of fragments, as per Table 8:

| **Ratio of 5' arm:3' arm: vector fragment** | **Amount of DNA** | **Formation of Shuttle Vector** |
|---|---|---|
| 1:1:6 (2µl:2µl:12µl) | ~20ng:20ng:120ng | No |
| 1:1:3 (2µl:2µl:6µl) | ~20ng:20ng:60ng | No |
| 1:1:1 (2µl of each) | ~20ng:20ng:20ng | Yes |

The entire ligation mixtures were transformed into bacteria by incubation on ice for 30 minutes with XL-10 Gold Ultracompetent cells followed by 30 second heatshock at 42 degrees. 500µ! of each transformed culture was spread on LB + Kan plates and incubated overnight at 37°C. A control using 6µl of dephosphorylated p15A-kan vector with 2µl T4 DNA Ligase was spread on LB + Kan plates. After overnight incubation colonies were present on all plates except the control plate.

### Diagnostic PCR screening and restriction digestion of colonies

4 Colonies were picked from each plate and were cultured overnight in 4ml LB Broth at 250rpm, 37°C. DNA was purified by miniprep, which involved harvesting DNA from bacteria by alkaline lysis and purification of DNA on DNA binding columns according to the manufacturer's miniprep protocol The DNA was eluted in 40µl buffer.

Diagnostic PCRs were performed on the 12 purified DNA samples to determine if a ~12kb ColoAd1 Shuttle Vector product was present and contained both the 5' and 3' arms in the correct orientation (Figure 13). Three separate PCRs were employed for each construct using primers across the junctions where ligations should have taken place. The sequences of the primers used in the reactions are shown in **Table 9** below.

**Table 9**

| **Reference number** | **Primer name** | **Sequence** |
|---|---|---|
| 0202 (SEQ ID NO: 9) | Kanr 5' arm FWD | ATCGCCTTCTATCGCCTTC |
| 0203 (SEQ ID NO: 10) | Kanr 5' arm REV | AGCAGTGCAAATCACAGTC |
| 0204 (SEQ ID NO: 11) | 5' 3' arms FWD | CAAACTGAGTCTGCTGTCG |
| 0205 (SEQ ID NO: 12) | 5' 3' arms REV | ATAAAGGGGTGTTGGGAGG |
| 0206 (SEQ ID NO: 13) | 3' arm p15A FWD | CCCTCGTAAAACCTGTCATC |
| 0207 (SEQ ID NO: 14) | 3' arm p15A REV | CCCATTCGTCTCTCCATTC |

The PCR reactions were set up according to the mixes detailed in Tables 10-12 below:

**Table 10 Mix 1:**

| **Reaction** | **Volume (µl)** |
|---|---|
| Taq PCR mix | 25 |
| Primer 202 (SEQ ID NO: 9) | 1 |
| Primer 203 (SEQ ID NO: 10) | 1 |
| Nuclease free water | 22 |
| DNA | 1 |

**Table 11 Mix 2:**

| **Reaction** | **Volume (µl)** |
|---|---|
| Taq PCR mix | 25 |
| Primer 204 (SEQ ID NO: 11) | 1 |
| Primer 205 (SEQ ID NO: 12) | 1 |
| Nuclease free water | 22 |
| DNA | 1 |

**Table 12 Mix 3:**

| **Reaction** | **Volume (µl)** |
|---|---|
| Taq PCR mix | 25 |
| Primer 206 (SEQ ID NO: 13) | 1 |
| Primer 207 (SEQ ID NO: 14) | 1 |
| Nuclease free water | 22 |
| DNA | 1 |

The PCR programme was set up according to the manufacturer's instructions for TAQ polymerase PCR mix (Qiagen #201443).

1µl of the PCR products were run on a 1% agarose gel for 1hr at 150V (Figure 14)

For correctly sized and orientated constructs 3 PCR products of 1.3kb, 1.4kb and 1.1kb were expected as detailed in the schematic in Figure 13. Only samples 13, 14 and 16 (Figure 14) showed correctly sized products. These constructs were all produced using a 1:1:1 ligation ratio. Constructs using the 1:1:6 and 1:1:12 ratios did not produce correctly sized fragments (Figure 14 #17-24). To confirm the presence of the 12kb ColoAd1 shuttle vector in the 1:1:1 ligation samples restriction digest with PspOMI or double digest with PspOMI and Ascl was carried out on a selection of constructs for 1 hr at 37°C. The digestion reactions used are detailed in Table 13 and Table 14.

**Table 13 Single digest:**

| **Reagent** | **Volume (µl)** | **Supplier** |
|---|---|---|
| DNA (Clones) | 3 | |
| PspOMI | 0.5 | NEB B7004S |
| Buffer 4 | 2 | NEB R0558S |
| Nuclease free water | 14.5 | Fisher Scientific (BPE 2484-100) |

**Table 14 Double digest:**

| **Reagent** | **Volume (µl)** | **Supplier** |
|---|---|---|
| DNA (Clones) | 3 | |
| PspOMI | 0.5 | NEB B7004S |
| AscI | 0.5 | NEB R0653S |
| Buffer 4 | 2 | NEB R0558S |
| Nuclease free water | 14.5 | Fisher Scientific (BPE 2484-100) |

1µl of each digest was run on a 0.8% gel for 1hr at 150V (Figure 15).

For correctly sized and orientated constructs the PspOMI digest would be expected to yield a single ~12kb band and the PspOMI/Ascl digest would be expected to yield a ~3kb band and ~4.7kb band. For the double digest the ~4.7kb band would be expected to have ~Twice the intensity of the ~3kb band due to the presence of both the 5' and 3' arms at this size. Constructs 13 and 16 showed the correct digest banding patterns corresponding to the ColoAd1 Shuttle vector (Figure 8).

The construct number 16 was then sequenced which confirmed successful construction of a ColoAd1 shuttle vector SEQ ID NO: 2.

### Example 2 Construction of the ColoAd2.4 Shuttle Vector

The ColoAd1 shuttle vector generated in Example 1 contained 11 unique (native) restriction sites that occur only once in the genome allowing modification of any region of the ColoAd1 genome present in the shuttle vector (for example, modification of the E1 region). A restriction map showing the location of the sites and the ColoAd1 genes present in the shuttle vector is provided in Figure 6.

Two of the unique (native) restriction sites (PspOMI and AclI) flank the fibre (L5) gene at positions 4634 and 6851 respectively in the ColoAd1 Shuttle Vector. These correspond to the original PspOMI and Acll that flank the fibre (L5) gene in the ColoAd1 genome at positions 27839 and 30065 (Figure 9). In the ColoAd1 shuttle vector these sites permit DNA sequences in this region to be excised and replaced with a modified or different sequence, or have a DNA sequence inserted within them as a simple addition.

The ColoAd2.4 Shuttle vector was generated from the ColoAd1 shuttle vector by replacement of the DNA sequence between the PspOMI and Acll restriction sites with a synthetic DNA fragment. The synthetic DNA fragment sequence was identical to the sequence it was replacing in the ColoAd1 Shuttle Vector except that it contained an additional 19bp downstream of the fibre gene (GCGATCGCTACCCTGCAGG - SEQ ID NO.29, Figure 3). These additional bases included new original Sgfl and Sbfl restriction sites (Figure 17).

The synthetic DNA fragment flanked by PspOMI and Acll sites was ordered from MWG Eurofins (Figure 18, SEQ ID NO. 27)). The synthetic fragment was supplied in a 5.17kb AmpR pBluescript II SK plasmid. The ColoAd1 shuttle vector and the pBluescript plasmid containing the synthetic fragment were digested for 1 hour at 37°C in the reaction detailed in Table 15:

| **Reagent** | **Volume (insert/vector) (µl)** | **Supplier** |
|---|---|---|
| DNA (pBSK plasmid/ColoAd1 shuttle vector) | 4/10 | |
| AclI | 0.4 | NEB R0598S |
| PspOMI | 0.2 | NEB R0653S |
| Buffer 4 | 2 | NEB R0558S |
| BSA | 2 | NEB B9001S |
| Nuclease free water | 5.4/11.3 | Fisher Scientific (BPE 2484-100) |

Both the digested shuttle vector and synthetic fragment were separated on a 0.8% agarose gel and the fragments of appropriate size were gel purified and eluted in 40µl of nuclease free water.

The synthetic fragment was ligated into the ColoAd1 shuttle vector using a 3:1 ligation ratio of insert to shuttle vector at volumes of 3µl:1µl with 1x Ligase Buffer and 1µl T4 DNA ligase in a 10µl reaction for 1 hour at RT.

2µl of the ligation mixture was transformed by heatshock at 42°C into XL-1 Blue cells according to the manufacturer's protocol. Following transformation, 5 colonies were picked from the LB kanamycin plate and were cultured overnight in 3ml LB Broth containing Kanamycin, at 250rpm, 37°C. Minipreps were performed for each culture according to the manufacturer's protocol and the purified DNA was eluted in 40µl buffer.

To confirm the construction of the ColoAd2.4 Shuttle vector containing the original restriction sites, Sbfl and Sgfl, restriction enzyme analysis was performed for each of the 5 samples. Restriction digests with EcoRV and Sbfl were set up as detailed in **Table 16** below:

| **Reagent** | **Volume (µl)** | **Supplier** |
|---|---|---|
| DNA | 3 | |
| EcoRV HF | 0.2 | NEB R319SS |
| SbfI HF | 0.2 | NEB R3642L |
| BSA | 2 | NEB B9001S |
| NEBuffer 4 | 2 | NEB R0558S |
| Nuclease free water | 14.6 | Fisher Scientific (BPE 2484-100) |

All 5 of the digests produced correctly sized fragments: bands of 3kb and 9kb (Figure 19).

The putative ColoAd2.4 shuttle vector #5 was amplified in bacteria from a glycerol stock and the DNA harvested and purified by maxiprep. This construct #5 was sequenced confirming successful construction of the ColoAd2.4 shuttle vector (SEQ ID No.26).

### Example 3 Construction of the ColoAd2.0 Shuttle Vector

The ColoAd2.0 Shuttle Vector (SEQ ID NO: 15, Figure 20) was generated from the ColoAd1 Shuttle Vector constructed in Example 1. The methods employed to generate the ColoAd2.0 Shuttle Vector were identical to those used to generate the ColoAd2.4 Shuttle Vector; using ligation of a synthetic DNA fragment between the PspOMI and Acll restriction sites detailed in Example 2

The synthetic DNA fragment sequence used to construct the ColoAd2.0 shuttle vector was similar to the sequence it was replacing in the ColoAd1 Shuttle Vector except it contained an additional 9bp upstream of the Fibre (L5) gene, comprising a Fsel original restriction site, and an additional 123bp downstream of the fibre gene, comprising two polyadenylation sequences and Sgfl, Notl and Sbfl original restrictions sites (SEQ ID NO: 16, Figure 21)

The construction of the ColoAd2.0 Shuttle Vector was confirmed by a panel of restriction digests with the enzymes Fsel, Ascl, Notl, Sbfl or PspOMI 1hr, 37°C as detailed by the mixes in **Table 17:**

| **Reagent** | **Volume (µl)** | **Supplier** |
|---|---|---|
| DNA | 3 | |
| Enzyme 1(FseI, PspOMI) | 0.2 | NEB R0588S, NEB R0653S |
| Enzyme 2 (SbfI, AscI, NotI) | 0.2 | NEB R3642S, NEB R0558S, NEB R3189S |
| BSA | 2 | NEB B9001S |
| NEBuffer 4 | 2 | NEB R0558S |
| Nuclease free water | 14.6 | Fisher Scientific (BPE 2484-100) |

2ul of each digest was separated on a 1% agarose gel. All the constructs showed the correct banding patterns for each digest (Figure 22). ColoAd2.0 Shuttle Vector construct #4 was selected and sequenced which confirmed successful construction of the ColoAd2.0 Shuttle Vector (SEQ ID NO: 15)

### Example 4. Construction of the ColoAd2.1 Shuttle Vector

The ColoAd2.1 Shuttle vector (SEQ ID NO: 17, Figure 23) was generated from the ColoAd1 shuttle vector (produced in Example 1) by replacement of the DNA sequence between the PspOMI and Acll restriction sites with a DNA fragment generated by two PCR reactions (SEQ ID NO: 18). The DNA fragment produced was identical to the sequence it was replacing in the ColoAd1 Shuttle Vector except it contained an additional 9bp upstream of the fibre gene (AGCGGCCGC- SEQ ID NO: 19, Figure 3). These additional bases include an original Notl restriction site (Figure 24C).

To introduce the Notl restriction site by PCR into the PspOMI - Acll flanked sequence, 2 PCR reactions were required. One PCR (PCR 1) that generated a fragment including the 5' PspOMI restriction site and an introduced 3' Notl site (Figure 24A) and a second (PCR 2) that generated a fragment including an introduced 5' Notl site and the 3' Acll site (Figure 24B). Ligation of these PCR products at the Notl site generated the ColoAd2.0 DNA fragment (Figure 24C) used in ColoAd2.0 Shuttle Vector construction.

The primers used for the two PCR reactions are shown in **Table 18:**

| **Primer ref number** | **Primer name** | **Sequence** |
|---|---|---|
| 0274 (SEQ ID NO.20) | Bam-Not A Fwd | TTCGGATCCGGGCCCATACTAGTCTTGC |
| 0275 (SEQ ID NO.21) | Bam-Not A Rev | CATGCGGCCGCTCTGGGAAGAAAGACATGAAGA |
| 0276 (SEQ ID NO.22) | Not-EcoRl A Fwd | TATGCGGCCGCATGACCAAGAGAGTCCG |
| 0277 (SEQ ID NO.23) | Not-EcoRI A Rev | TGCGAATTCAACGTTGTCCATGGTACAGAC |

**PCR** 1 was performed on the ColoAd1 genome template DNA using primers 0274 (SEQ ID NO: 20) and 0275 (SEQ ID NO: 21). A 50µl reaction volume was used for the PCR 1 reaction, according to Table 19 below and a schematic of the PCR 1 product is shown in Figure 24A (SEQ ID NO: 24).

**Table 19**

| **Reagent** | **Volume (µl)** | **Supplier** |
|---|---|---|
| Phusion PCR Mix | 25 | NEB MO531S |
| Primer 0274 (SEQ ID NO: 20) (10µM) | 2.5 | Sigma |
| Primer 0275 (SEQ ID NO: 21) (10µM) | 2.5 | Sigma |
| DNA (ColoAd1) | 1 | Ark Therapeutics |
| Nuclease Free Water | 19 | Fisher Scientific (BPE 2484-100) |

**PCR 2** was performed on the ColoAd1 genome template DNA using primers 0276 (SEQ ID NO: 22) and 0277 (SEQ ID NO.23). A 50µl reaction volume was used for the PCR 2 reaction, according to Table 20 below and a schematic of the PCR 2 product is shown in Figure 24B (SEQ ID NO: 25)

**Table 20**

| **Reagent** | **Volume (µl)** | **Supplier** |
|---|---|---|
| Phusion PCR Mix | 25 | NEB MO531S |
| Primer 0276 (SEQ ID NO.22) (10µM) | 2.5 | Sigma |
| Primer 0277 (SEQ ID NO.23) (10µM) | 2.5 | Sigma |
| DNA (ColoAd1) | 1 | Ark Therapeutics |
| Nuclease Free Water | 19 | Fisher Scientific (BPE 2484-100) |

The PCR reactions were both carried out according to the programme detailed in **Table 21:**

| **Step no.** | **Stage** | **Temp (°C)** | **Time (Secs)** |
|---|---|---|---|
| Step 1 | Initial Denaturation | 98 | 60 |
| Step 2 | Denaturation | 98 | 8 |
| Step 3 | Annealing | 60 | 20 |
| Step 4 | Extension | 72 | 90 |
| Step 5 | Final Extension | 72 | 300 |
| Step 6 | Hold | 4 | Hold |

30 cycles of amplification were carried out: [Step 1] x 1, [Step 2, Step 3, Step 4] x 30, [Step 5] x 1.

The entire volume of the PCR products were purified by Spin Column Method according to the manufacturer's protocol and eluted into 40µl of Elution Buffer.

The PCR 1 product was then digested with BamHl and Notl (Table 22) and a cloning vector was also digested using Bglll and Notl (see Table 23) for 1hr, 37°C.

**Table 22 PCR product:**

| **Reagent** | **Volume (µl)** | **Supplier** |
|---|---|---|
| DNA (PCR product) | 20 | |
| Buffer 4 | 5 | NEB B7004S |
| BamHI | 0.2 | NEB R0136S |
| NotI | 0.2 | NEB R3189S |
| Nuclease Free Water | 19.6 | Fisher Scientific (BPE 2484-100) |

**Table 23 Vector:**

| **Reagent** | **Volume (µl)** | **Supplier** |
|---|---|---|
| DNA | 5 | |
| Buffer 4 | 2 | NEB R7004S |
| BgIII | 0.2 | NEB R0144S |
| NotI | 0.2 | NEB R3189S |
| Nuclease Free Water | 10.6 | Fisher Scientific (BPE 2484-100) |

The digested vector and PCR 1 product were separated on a 0.8% agarose gel and the fragments of appropriate size were gel purified and eluted in 40µl of nuclease free water.

The PCR 1 product was ligated into the vector using a 3:1 ligation ratio of insert to vector at volumes of 10µl:1µl with 1x Ligase Buffer and 1µl T4 DNA ligase in a 20µl reaction for 1 hour at RT.

6µl of the ligation mixture was transformed by heat shock at 42°C into XL-1 Blue cells according to the manufacturer's protocol. Following transformation, 5 colonies were picked from the LB kanamycin plate and were cultured overnight in 3ml LB Broth containing Kanamycin, at 250rpm, 37°C. Minipreps were performed for each culture according to the manufacturer's protocol and the purified DNA was eluted in 40µl buffer.

To confirm the vector contained the PCR 1 product, restriction enzyme analysis was performed for each of the 5 samples. Restriction digests with Spel and Ascl were set up for 1hr, 37°C as detailed in

**Table 24:**

| **Reagent** | **Volume (µl)** | **Supplier** |
|---|---|---|
| DNA (PCR product) | 20 | |
| Buffer 4 | 5 | NEB B7004S |
| SpeI | 0.2 | NEB R3133S |
| AscI | 0.2 | NEB R0558S |
| Nuclease Free Water | 19.6 | Fisher Scientific (BPE 2484-100) |

All 5 of the digests produced correctly sized fragments: bands of 1.5kb and 4kb.

The PCR 2 product and the newly generated vector containing the PCR 1 product were digested with Notl and EcoRl, 1hr, 37°C, as detailed in **Table 25:**

| **Reagent** | **Volume (µl)** | **Supplier** |
|---|---|---|
| DNA (PCR product) | 20 | |
| Buffer 4 | 5 | NEB B7004S |
| EcoRI | 0.2 | NEB R0101S |
| NotI | 0.2 | NEB 3189S |
| Nuclease Free Water | 19.6 | Fisher Scientific (BPE 2484-100) |

The digested vector containing the PCR 1 fragment and PCR 2 product were separated on a 0.8% agarose gel and the fragments of appropriate size were gel purified and eluted in 40µl of nuclease free water.

The PCR 2 product was ligated into the vector using a 3:1 ligation ratio of insert to vector at volumes of 10µl:1µl with 1x Ligase Buffer and 1µl T4 DNA ligase in a 20µl reaction for 1 hour at RT.

6µl of the ligation mixture was transformed by heat shock at 42°C into XL-1 Blue cells according to the manufacturer's protocol. Following transformation, 5 colonies were picked from the LB kanamycin plate and were cultured overnight in 3ml LB Broth containing Kanamycin, at 250rpm, 37°C. Minipreps were performed for each culture according to the manufacturer's protocol and the purified DNA was eluted in 40µl buffer.

To confirm the vector contained the DNA fragment consisting of both PCR1 and PCR2 products, restriction enzyme analysis was performed for each of the 5 samples. Restriction digests with PspOMI and Acll were set up for 1hr, 37°C as detailed in **Table 26:**

| **Reagent** | **Volume (µl)** | **Supplier** |
|---|---|---|
| DNA (PCR product) | 20 | |
| Buffer 4 | 5 | NEB B7004S |
| PspOMI | 0.2 | NEB R0653S |
| AclI | 0.2 | NEB R0598S |
| Nuclease Free Water | 19.6 | Fisher Scientific (BPE 2484-100) |

All 5 of the digests produced correctly sized fragments: bands of 2.3kb and 3.15kb. To confirm the sequence of the DNA fragment between the PspOMI and Acll sites in the vector was correct the sample #5 was sequenced (SEQ ID NO. 18).

The ColoAd2.1 Shuttle Vector was then generated from the ColoAd1 Shuttle Vector constructed in Example 1. The methods employed to ligate the DNA fragment generated by the 2 PCR reactions into the ColoAd1 Shuttle Vector are identical to those ligate the synthetic DNA fragment to produce the ColoAd2.4 Shuttle Vector in Example 2.

The construction of the ColoAd2.1 Shuttle Vector was confirmed by restriction digest with the enzymes Notl and Ascl for 1hr, 37°C as detailed by the mixes in **Table 27:**

| **Reagent** | **Volume (µl)** | **Supplier** |
|---|---|---|
| DNA (PCR product) | 20 | |
| Buffer 4 | 5 | NEB B7004S |
| AscI | 0.2 | NEB R0558S |
| NotI | 0.2 | NEB R3189S |
| Nuclease Free Water | 19.6 | Fisher Scientific (BPE 2484-100) |

2ul of each digest was separated on a 1% agarose gel, all constructs show the correct banding patterns corresponding to the ColoAd2.1 Shuttle Vector (Figure 25). ColoAd2.1 Shuttle Vector Sample #4 was selected and sequenced, confirming successful construction of the ColoAd2.1 Shuttle Vector (SEQ ID NO. 17).

### Example 5 Construction of the ColoAd2.4, ColoAd2.0 or ColoAd2.1 plasmids by homologous recombination

ColoAd2.0 (SEQ ID NO.30), ColoAd2.4 (SEQ ID NO.28) and ColoAd2.1 (SEQ ID NO.31) plasmids were generated from the ColoAd2.0, ColoAd2.4 and ColoAd2.1 Shuttle Vectors by homologous recombination. A schematic overview of the methods is provided in Figure 5.

The PspOMI site in the ColoAd2.0, ColoAd2.4 and ColoAd2.1 shuttle vectors permitted the shuttle vectors to be linearised for homologous recombination with the ColoAd1 genome in electrocompetent *E. coli*

The ColoAd2.4 shuttle vector (SEQ ID NO.26) (68.6ng/µl) was digested with PspOMI for 1 hour, 37°C using the following reaction:

| **Reagent** | **Volume (µl)** | **Supplier** |
|---|---|---|
| DNA (PCR product) | 25 (x2) | |
| Buffer 4 | 5 | NEB B7004S |
| PspOMI | 1 | NEB R0598S |
| BSA | 5 | B9001S |
| Nuclease Free Water | 19 | Fisher Scientific (BPE 2484-100) |

The 50µl digest was treated with 1µl CIP (Calf Alkaline Phosphatase) for 1 hour at 37°C.

The digests were pooled and purified on a Sigma Genelute Miniprep column, eluting in 40µl NFW.

Recombinations were carried out with 3.5µl (23.4ng/µl) ColoAd2.4 shuttle vector and 1.5µl (36ng/µl) of ColoAd1 in 40µl of electrocompetent BJ5183 cells (Agilent). A negative control was also carried out with 3.5 µl of the linearised vector (ColoAd2.4 vector) in 40µl BJ5183 cells. Recombinations were performed by electroporation according to manufacturer's protocol (#200154 Agilent).

5µl of the cultures were diluted and spread on LB agar + Kanamycin and incubated overnight at 37°C.

The negative control showed few normal sized colonies on the LB+Kan plates, while the recombination plates showed many tiny colonies and a few large or medium colonies (Figure 27A). From the experimental plates 48 colonies were picked and inoculated into 3 ml LB Broth+Kan overnight at 37°C, 250 rpm.

The DNA was purified from the bacteria by miniprep according to the manufacturer's protocol and was eluted in 40µl nuclease free water.

To determine the presence of recombinants in the DNA samples, the candidates were digested with EcoRV and Sbfl using the following reaction **Table 29:**

| **Reagent** | **Volume (µl)** | **Supplier** |
|---|---|---|
| DNA | 12 | |
| Buffer 4 | 2 | NEB B7004S |
| BSA | 2 | NEB B9001S |
| EcoRV | 0.2 | NEB R3195S |
| Sbfl Hi Fi | 0.2 | NEB B7004S |
| Nuclease free water | 3.6 | Fisher Scientific (BPE 2484-100) |

The digests were run on a 0.8% agarose gel for 1 hour at 150V (Figure 27B).

Recombinants #3, #8 and #10 showed the correct bands following EcoRV/Sbfl digestions and 2µl of each were transformed into 50µl XL-1 Blue cells according to manufacturer's protocol. 50µl of cells were plated on LB and Kanamycin plates and incubated overnight at 37°C.

From the #8 plates, 7 colonies were picked and grown in 3ml LB broth with Kanamycin overnight at 37°C, 220rpm. Minipreps were performed on the recombinant clones according to the manufacturer's protocol and DNA was eluted in 40µl NFW. Diagnostic restriction enzyme digests were set up at 37°C for 1 hour using the following digestion reactions **Table 30:**

| **Reagent** | **Volume (µl)** | **Supplier** |
|---|---|---|
| DNA | 2 | |
| Buffer 4 | 2 | NEB B7004S |
| BSA | 2 | NEB B9001S |
| EcoRV | 0.2 | NEB R3195S |
| Sbfl-HF | 0.2 | NEB B7004S |
| Nuclease free water | 13.6 | Fisher Scientific (BPE 2484-100) |

**Table 31**

| **Reagent** | **Volume (µl)** | **Supplier** |
|---|---|---|
| DNA | 2 | |
| PspOMI | 0.2 | NEB R0598S |
| Buffer 4 | 5 | NEB R0558S |
| Nuclease free water | 15.6 | Fisher Scientific (BPE 2484-100) |

The digests were run on a 0.8% agarose gel (Figure 28).

Of the constructs digested Recombinant ColoAd2.4 #4 plasmid had the correct restriction pattern. This plasmid was therefore amplified from a glycerol stock and the DNA was purified by maxiprep and sequenced (SEQ ID NO.28). This confirmed successful production of the ColoAd2.4 plasmid (Figure 26). ColoAd2.0 (SEQ ID NO.30), ColoAd2.4 (SEQ ID NO.28) and ColoAd2.1 (SEQ ID NO.31) plasmids shown in Figure 2 were all successfully constructed using the above method

All plasmids were sequenced to confirm no unwanted changes had occurred in the ColoAd1 genome sequence.

### Example 6 Failed two-step ligation of the shuttle vector

20 µl of the **ColoAd1** 5' arm and 3' arm PCR products described in Example 1 were digested by PspOMI at 37°C for 2 hrs as detailed below **Table 32:**

| **Reagent** | **Volume (µl)** | **Supplier** |
|---|---|---|
| DNA (3' and 5' arm PCR products) | 20 | |
| Buffer 4 | 4 | NEB B7004S |
| PspOMI | 1.5 | NEB R0653S |
| Nuclease Free Water | 14.5 | Fisher Scientific (BPE 2484-100) |

The digests were separated on a 0.8% agarose gel, gel purified and eluted in 30 µl of elution buffer. The entire volume of eluted product was used for ligation reactions.

The 5' arm and 3' arm (~ 4.6 kb and ~4.5 kb) fragments were ligated at a 1:1 ratio using volumes of 10 µl (low) or 25 µl (high) at RT for 2.5 hrs. **Table 33:**

| **10:10 mix** | **25:25 Mix** | |
|---|---|---|
| 10 µl 5' arm DNA | 25 µl 5' arm DNA | |
| 10 µl 3' arm DNA | 25 µl 3' arm DNA | |
| 2 µl T4 DNA ligase | 3 µl T4 DNA ligase | NEB M0202S |
| 4 µl Ligase Buffer | 6 µl Ligase Buffer | NEB B0202S |

The ligation reactions were heat inactivated for 5 mins at 70°C and two PCRs were performed on each set of ligations using Primer 0199 (SEQ ID NO.6) or Primer 0198 (SEQ ID NO.5) to amplify a ~9.1 kb fragment.

1 µl of each PCR product was run on a 0.8% agarose gel (Figure 29A).

The total volume of PCR products following low volume ligation were separated on a 0.8% agarose gel, gel purified and eluted into 40µl elution buffer.

The ligated PCR 9kb product and previously PCR amplified ~3 kb p15A-Kan vector were restriction digested with enzyme Ascl for 2 hrs at 37°C using the following mix:

**Table 34**

| **Reagent** | **Volume (µl)** | **Supplier** |
|---|---|---|
| DNA (9kb ligations/p15a-Kan vector) | 20 | |
| Buffer 4 | 4 | NEB B7004S |
| Ascl | 2 | NEB R0558S |
| Nuclease free water | 10 | Fisher Scientific (BPE 2484-100) |

The Ascl digested ~9kb fragments were heat inactivated at 65°C for 20 mins and the Ascl digested p15a KAN vector was treated with 1µl CIP for 1 hr at 37°C.

1µl of the digested products were run on a 1% agarose gel to assess relative DNA concentrations (Figure 29B).

The entire volume of each product was then purified by Spin Column method and eluted in 40 µl elution buffer.

A ligation between the ~ 9kb fragment (5'arm - 3'arm) and the p15A-Kan vector was performed using 2 µl of T4 DNA Ligase and 4 µl Ligase Buffer at the following ratios for 1.5 hrs at RT:
1:1 (5 µl); 1:2 (2 µl:4 µl); 1:3 (2 µ!:6 µl); 1:3 (4 µl:12 µl); and 1:3 (3 µl:9 µl)

The entire volume of each ligation mix was transformed by heatshock into 50 µl of XL-10 Gold Ultracompetent cells according to the manufacturer's protocol (Stratagene #200314).

500µ! of each culture was spread on LB + kanamycin plates and incubated overnight at 37°C. Colonies were present on the plates and were amplified in LB Broth, 37°C, 250rpm. The DNA was purified from the bacteria by miniprep, according to the manufacturer's protocol.

The purified DNA was analysed for the presence of the ColoAd1 Shuttle Vector by diagnostic PCR and restriction digest methods identical to those detailed in Example 1.

For correctly sized and orientated constructs diagnostic PCR was expected to produce 1.3kb, 1.4kb and 1.1kb bands and restriction digest was expected to yield a single ~12kb band for PspOMI and ~3kb band and ~4.7kb bands for PspOMI/Ascl. None of the samples showed the correct bands by either diagnostic method (Figure 16).

### Example 7 Construction of a ColoAd2.4 plasmid containing a reporter transgene cassette (pNG-62)

The ColoAd2.4 plasmid generated in Example 5 was used to construct a plasmid named pNG-62 (SEQ ID NO. 35, Figure 30), which contained a reporter gene transgene cassette between the ColoAd2.4 plasmid unique restriction sites Sgfl and Sbfl. The transgene cassette consisted of a branched splice acceptor sequence (bSA), a fluorescent reporter gene, green fluorescent protein (GFP) and a SV40 late polyA sequence (PA).

### 1) Construction of the transgene cassette

A cloning vector that contained the GFP sequence with a 3' SV40 late polyA sequence (mpSF-CMV-GFP-PA) was used as a PCR template for construction of the transgene cassette.

A branched splice acceptor (bSA) and KOZAK sequence 5'- TGCTAATCTTCCTTTCTCTCTTCAGGCCGCC-3' (SEQ ID NO. 36) was added to the 5' end of the GFP gene by PCR. The PCR primers also introduced a 5' Sgfl site before the start of the bSA sequence and a 3' Sbfl site after the SV40 polyA sequence to give the transgene cassette PCR product (Figure 31, SEQ ID NO. 37). The PCR primers used to amplify the PCR product are detailed in **Table 35:**

| **Reference number** | **Primer name** | **Sequence** |
|---|---|---|
| 0350 (SEQ ID NO: 39) | GFP Sgf bSA Fwd | TATGCGATCGCTGCTAATCCTTTCTCTCTTCAGGCCA CCATGGTGAGCAAGGGC |
| 0323 (SEQ ID NO.40) | Luc Sbf PA Rev | CTGAAGAGAGAAACTACCTGCAGGACCACATTTGTA GAGGTTTTAC |

A 50µl reaction volume was used for the PCR reaction detailed in **Table 36:**

| **Reagent** | **Volume** | **Supplier** |
|---|---|---|
| DNA | 1 µl | |
| PCR phusion mix | 25 µl | NEB MOS31S |
| Forward primer - 0350 | 2.5 µl | Mwg Eurofins |
| Reverse primer - 0323 | 2.5 µl | Mwg Eurofins |
| Nuclease free water | 19 µl | Fisher Scientific (BPE 2484-100) |

PCR amplification was carried out according to the programme in **Table 37:**

| **Cycles** | **Step number** | **Stage** | **Temp (⁰C)** | **Time (Secs)** |
|---|---|---|---|---|
| 1 | Step 1 | Initial Denaturation | 98 | 60 |
| 10 | Step 2 | Denaturation | 98 | 20 |
| | Step 3 | Annealing | 57 | 30 |
| | Step 4 | Extension | 72 | 60 |
| 20 | Step 5 | Denaturation | 98 | 20 |
| | Step 6 | Annealing | 65 | 30 |
| | Step 7 | Extension | 72 | 60 |
| 1 | Step 8 | Final Extension | 72 | 300 |
| | Step 9 | Hold | 4 | Hold |

The PCR product was purified by spin column eluting in 40 µl of NFW. The PCR product and the subcloning vector containing Sgfl and Sbfl restriction sites were digested with the enzymes Sgfl and Sbfl (Table 38) for 1hr at 37°C.

**Table 38**

| Reagent | **Volume** | **Supplier** |
|---|---|---|
| PCR product/ vector | 20/4 µl | |
| Sgfl | 2 µl | |
| Sbfl | 1 µl | |
| BSA | 5/2 µl | NEB B9001S |
| NEBuffer 4 | 5/2 µl | NEB R0558S |
| Nuclease free water | 17/9 µl | Fisher Scientific (BPE 2484-100) |

The digested products were separated on a 1% agarose gel and the fragments of appropriate size were gel purified and eluted in 40ul of nuclease free water.

The digested, purified PCR product was ligated into the linearised vector using a 3:1 ligation ratio of insert to vector at volumes of 3 µl:1 µl with 1x ligase buffer and 1 µl T4 DNA ligase in a 10 µl reaction for 1hr at RT.

1 µl of the ligation reaction was transformed into 50 µl of XL Gold cells and 100 µl spread on LB + ampicillin (100 µg/ml) plates. After overnight growth >50 colonies were present on the culture plates.

5 colonies were picked and cultured overnight in 3ml LB Broth containing ampicillin. DNA was purified by miniprep and eluted in 40 µl buffer.

To confirm the presence of the transgene cassette in the vector the constructs were diagnostically digested with Sgfl and Sbfl restriction enzymes Table 39:

| **Reagent** | **Volume (µl)** | **Supplier** |
|---|---|---|
| DNA (PCR product) | 20 | |
| Buffer 4 | 5 | NEB B7004S |
| Sgfl | 0.2 | NEB R0598S |
| Sbfl | 0.2 | |
| Nuclease Free Water | 19.6 | Fisher Scientific (BPE 2484-100) |

All 5 constructs produced correctly sized fragments from the digests: bands of 1.1kb and 4.7kb (Figure 32). Construct #1 was therefore sequenced and confirmed successful construction of the GFP transgene cassette (Figure 31, SEQ ID NO. 37).

### 2) Construction of plasmid NG-62

Construct #1 that contained the GFP transgene cassette (576 ng/µl) and ColoAd2.4 plasmid (583ng/µl) were digested with Sgfl and Sbfl for 2 hours at 37°C using the reaction shown in **Table 40:**

| **Reagent** | **Volume (µl)** | **Supplier** |
|---|---|---|
| DNA (construct 1/ColoAd 2.4 plasmid) | 5 (x2) | |
| Sgfl | 2 | NEB R0630S |
| Sbfl | 1.5 | NEB R3642S |
| Buffer 4 | 2 | NEB B7004S |
| BSA | 2 | NEB B9001S |
| Nuclease free water | 7.5 | Fisher Scientific (BPE 2484-100) |

The digests were separated on a 0.8% agarose gel and the fragments of appropriate size were excised from the gel. The ColoAd2.4 plasmid was then purified using the QIAEX II kit (QIAGEN) and the GFP transgene cassette was purified using the quick gel extraction kit (QIAGEN).

The transgene cassette (40 ng/µl) was ligated into the ColoAd2.4 plasmid (18 ng/µl) using either a 1.5:1 ligation ratio of insert to vector (2.25 µl: 3.7 µl) or a 2:1 ligation ratio (3 µl: 4 µl). The reactions were carried out with 1x Ligase Buffer and 1 µl T4 DNA ligase in a 10 µl reaction volume. The ligation was carried out for 16 hrs at 16°C.

4 µl of the ligation mixture was transformed into 50 µl XL-Blue cells according to the manufacturer's protocol (Agilent) and the entire transformation volume was spread on LB agar plates containing kanamycin. After overnight growth all colonies were picked from the plates and were cultured overnight in 3 ml LB Broth containing Kanamycin, at 250 rpm, 37°C.

DNA was purified by miniprep and was eluted in 40 µl buffer.

To determine if pNG-62 plasmid had been generated, the constructs were screened by restriction digest using the enzymes Nhel and EcoRV as detailed in **Table 41:**

| **Reagent** | **Volume (µl)** | **Supplier** |
|---|---|---|
| DNA | 2 | |
| Nhel | 0.6 | NEB R0131S |
| EcoRV-HF | 0.4 | NEB R3159S |
| Buffer 4 | 2 | NEB R0558S |
| BSA | 2 | NEB B9001S |
| Nuclease free water | 13 | Fisher Scientific (BPE 2484-100) |

The digest products were separated on a 0.8% agarose gel. Of the constructs screened numbers 1, 2 & 3 from 1.5:1 ratio ligation and 1, 2 & 4 from the 2:1 ratio ligation showed band sizes that correspond to the predicted pattern of 12.3kb, 9.7kb, 5.5kb and 3.1kb of the plasmid pNG-62 (Figure 33A).

The putative pNG-62 constructs #2 (1.5:1) and #1 (2:1) were further diagnostically restriction digested with the enzyme Bglll or the enzymes Nhel and EcoRV (Figure 33B) and then sequenced. This confirmed successful construction of the plasmid pNG-62 (SEQ ID NO 35, Figure 30).

### Example 8 NG-62 Virus production and transgene expression

The plasmid pNG-62 generated in Example 7 was linearised and used to produce viable ColoAd1 virus particles containing the ColoAd1 genome with a reporter gene (GFP) transgene cassette inserted downstream of the fibre (L5) gene between introduced Sgfl and Sbfl restriction sites.

The plasmid pNG-62 (685ng/µl) was linearised to produce the NG-62 virus genome (SEQ ID NO. 38) by restriction digest with the enzyme AscI. The restriction digest reaction was set up according to table 42 and carried out for 2 hrs, 37°C.

**Table 42**

| **Reagent** | **Volume (µl)** | **Supplier** |
|---|---|---|
| pNG-62 DNA (~7µg) | 10 | |
| Ascl | 2.5 | NEB R0558S |
| Buffer 4 | 5 | NEB B7004S |
| Nuclease free water | 32.5 | Fisher Scientific (BPE 2484-100) |

Digested pNG-62 DNA was diluted with 50µl nuclease-free water and then purified by phenol/chloroform extraction. The extracted NG-62 DNA was then precipitated for 16hrs, -20°C in 300µl >95% molecular biology grade ethanol and 10µl 3M Sodium Acetate.

The precipitated DNA was pelleted by centrifuging at 14000rpm, 5 mins and was washed in 500µl 70% ethanol, before centrifuging again, 14000rpm, 5mins. The clean DNA pellet was air dried, resuspended in 500µl OptiMEM containing 15µl lipofectamine transfection reagent and incubated for 30 mins, RT. The transfection mixture was then added drop wise to a T-25 flask containing Hek293 cells grown to 70% confluency. After incubation of the cells with the transfection mix for 2hrs at 37°C, 5% CO₂ 4mls of cell media (DMEM high glucose with glutamine supplemented with 2% FBS) was added to the cells and the flasks was incubated 37°C, 5% CO₂.

The transfected Hek293 cells were monitored every 24hrs and were supplemented with additional media every 48-72hrs. The production of virus was monitored by observation of a significant cytopathic effect (CPE) in the cell monolayer. Once CPE was observed the virus was harvested from Hek293 cells by three freeze-thaw cycles. The harvested NG-62 virus was used to infect AD293 cells and confirmed viable virus production by observation of significant CPE in the cell monolayer 24hrs and 48hrs post-infection (Figure 34A). The productive expression of GFP transgene from the virus was also confirmed in the infected AD293 cells by immunofluorescence imaging (Figure 34B).

### References

Shenk, (2001) "Adenoviridae: The Viruses and Their Replication," in Fields Virology, Vol.2, Fourth Edition, Knipe, ea., Lippincott, Williams & Wilkins, pp. 2265-2267
Jin et al (2004) "Identification of novel insertion sites in the Ad5 genome that utilize the Ad splicing machinery for therapeutic gene expression" Molecular Therapy Vol.12(6) pp1052-63.
Cheever M.J. et al, The prioritization of cancer antigens: a National Cancer Institute pilot project for the acceleration of translational research. Clin Cancer Res 2009;15:5323-5337

## Claims

1. A method of preparing a shuttle vector comprising a selection marker gene and a low copy bacterial replication of origin, and an adenovirus genome comprising a 5' ITR, a 3' ITR, and an L5 gene said method comprising the steps:
**a)** preparing an adenovirus shuttle vector by performing a one-step three-way ligation to equal proportions of the following three fragments:
i) a vector fragment comprising a selection marker gene and a low copy bacterial replication of origin, wherein the 5' end of the vector fragment starts with a first restriction enzyme site and terminates at the 3' end of the vector fragment in a second restriction enzyme site,
ii) a 5'-arm comprising the 5' end of the adenovirus genome including the 5' ITR, wherein the 5' end of the 5' arm starts with a second restriction enzyme site and terminates at the 3' end of the 5' arm with a third restriction enzyme site,
iii) a 3'-arm comprising the 3' end of the adenovirus genome including the 3' ITR and the L5 gene, wherein the 5' end of the 3' arm starts with a third restriction enzyme site and terminates at the 3' end of the 3' arm with a first restriction enzyme site,
thereby joining:
the 3' end of the 3' arm (fragment iii) to the 5' end of the vector fragment (fragment i) at the first restriction enzyme site,
the 3' end of the vector fragment (fragment i) to the 5' end of the 5' arm (fragment ii) at the second restriction enzyme site, and
the 3' end of the 5' arm (fragment ii) to the 5' end of the 3' arm (fragment iii) at the third restriction enzyme site,
to form a circularised shuttle vector arranged as a first restriction enzyme site followed by a vector fragment followed by a second restriction enzyme site followed by a 5' arm, followed by a third restriction enzyme site followed by a 3' arm,
**b)** introducing at least one unique restriction site and/or transgene into the shuttle vector in a location:
i) between the L5 gene and an E3 site,
ii) between the L5 gene and an E4 site, or
iii) between the L5 gene and an E3 site, and also between the L5 gene and an E4 site.

2. A method according to claim 1 wherein the 5' arm comprises about 2.4 to 4.7 kb of the 5' end of an adenovirus genome, and/or
the 3' arm comprises about 3.3 to 4.8 kb of the 3' end of an adenovirus genome.

3. A method according to any one of claims 1 to 2, wherein the unique restriction site is independently selected from Fsel, Notl, Sbfl and Sgfl.

4. A method according to any one of claims 1 to 3, wherein the vector fragment is dephosphorylated.

5. A method according to any one of claims 1 to 4, wherein the adenovirus genome is from an adenovirus capable of replication.

6. A method of preparing a plasmid, comprising steps a) and b) according to any one of claims 1 to 5, and further comprising a step **c)** performing homologous recombination in electrocompetent cells between the shuttle vector of step a) and b) and an adenovirus genome to form a plasmid.

7. A method according to claim 6, wherein step **c)** is performed at a ratio of 3.5:1.5.

8. A method according to claim 6 or 7, wherein step **c)** is performed in electrocompetent BJ5183 cells.

9. A method according to any one of claims 6 to 8, wherein the adenovirus genome is selected from EnAd, OvAd1, OvAd2, Ad3, Ad5 and Ad11.

10. A method of generating an adenovirus, comprising preparing a plasmid according to the method of any one of claims 6 to 9, and comprising a further step of generating an adenovirus.

## Patentansprüche

1. Verfahren zur Herstellung eines Shuttle-Vektors, umfassend ein Selektionsmarker-Gen und einen low-copy bakteriellen Replikationsursprung und ein Adenovirus-Genom, das ein 5'-ITR, ein 3'-ITR und ein L5-Gen umfasst, wobei das Verfahren die Schritte umfasst:
a) Herstellung eines Adenovirus-Shuttle-Vektors mittels Durchführen einer einstufigen Drei-Wege-Ligation zu gleichen Anteilen der folgenden drei Fragmente:
i) ein Vektorfragment, das ein Selektionsmarker-Gen und einen low-copy bakteriellen Replikationsursprung umfasst, wobei das 5'-Ende des Vektorfragments mit einer ersten Restriktionsenzymstelle beginnt und am 3'-Ende des Vektorfragments mit einer zweiten Restriktionsenzymstelle endet,
ii) einen 5'-Arm, der das 5'-Ende des Adenovirus-Genoms einschließlich des 5'-ITR umfasst, wobei das 5'-Ende des 5'-Arms mit einer zweiten Restriktionsenzymstelle beginnt und am 3'-Ende des 5'-Arms mit einer dritten Restriktionsenzymstelle endet,
iii) einen 3'-Arm, der das 3'-Ende des Adenovirus-Genoms einschließlich des 3'-ITR und des L5-Gens umfasst, wobei das 5'-Ende des 3'-Arms mit einer dritten Restriktionsenzymstelle beginnt und am 3'-Ende des 3'-Arms mit einer ersten Restriktionsenzymstelle endet,
dadurch verbindend:
das 3'-Ende des 3'-Arms (Fragment iii) an das 5'-Ende des Vektorfragments (Fragment i) an der ersten Restriktionsenzymstelle,
das 3'-Ende des Vektorfragments (Fragment i) an das 5'-Ende des 5'-Arms (Fragment ii) an der zweiten Restriktionsenzymstelle, und
das 3'-Ende des 5'-Arms (Fragment ii) mit dem 5'-Ende des 3'-Arms (Fragment iii) an der dritten Restriktionsenzymstelle,
um einen zirkularisierten Shuttle-Vektor zu bilden, angeordnet aus einer ersten Restriktionsenzymstelle, gefolgt von einem Vektorfragment, gefolgt von einer zweiten Restriktionsenzymstelle, gefolgt von einem 5'-Arm, gefolgt von einer dritten Restriktionsenzymstelle, gefolgt von einem 3'-Arm,
b) Einführen mindestens einer eindeutigen Restriktionsstelle und/oder eines Transgens in den Shuttle-Vektor an einer Stelle:
i) zwischen dem L5-Gen und einer E3-Stelle,
ii) zwischen dem L5-Gen und einer E4-Stelle, oder
iii) zwischen dem L5-Gen und einer E3-Stelle und auch zwischen dem L5-Gen und einer E4-Stelle.

2. Verfahren nach Anspruch 1,
wobei der 5'-Arm etwa 2,4 bis 4,7 kb des 5'-Endes eines Adenovirus-Genoms umfasst, und/oder
wobei der 3'-Arm etwa 3,3 bis 4,8 kb des 3'-Endes eines Adenovirus-Genoms umfasst.

3. Verfahren nach einem der Ansprüche 1 bis 2, wobei die eindeutige Restriktionsstelle unabhängig voneinander ausgewählt ist aus Fsel, Notl, Sbfl und Sgfl.

4. Verfahren nach einem der Ansprüche 1 bis 3, wobei das Vektorfragment dephosphoryliert ist.

5. Verfahren nach einem der Ansprüche 1 bis 4, wobei das Adenovirus-Genom von einem replikationsfähigen Adenovirus stammt.

6. Verfahren zur Herstellung eines Plasmids, umfassend die Schritte a) und b) nach einem der Ansprüche 1 bis 5 und ferner umfassend einen Schritt c) Durchführen einer homologen Rekombination in elektrokompetenten Zellen zwischen dem Shuttle-Vektor aus Schritt a) und b) und einem Adenovirus-Genom zur Bildung eines Plasmids.

7. Verfahren nach Anspruch 6, wobei Schritt c) in einem Verhältnis von 3,5:1,5 durchgeführt wird.

8. Verfahren nach Anspruch 6 oder 7, wobei Schritt c) in elektrokompetenten BJ5183-Zellen durchgeführt wird.

9. Verfahren nach einem der Ansprüche 6 bis 8, wobei das Adenovirus-Genom ausgewählt ist aus EnAd, OvAd1, OvAd2, Ad3, Ad5 und Ad11.

10. Verfahren zur Erzeugung eines Adenovirus, umfassend das Herstellen eines Plasmids gemäß dem Verfahren nach einem der Ansprüche 6 bis 9, und umfassend einen weiteren Schritt der Erzeugung eines Adenovirus.

## Revendications

1. Procédé de préparation d'un vecteur navette comprenant un gène marqueur de sélection et une origine de réplication bactérienne à faible copie, et un génome d'adénovirus comprenant un ITR 5', un ITR 3' et un gène L5, ce procédé comprenant les étapes suivantes :
a) préparer un vecteur navette adénovirus en effectuant une ligature à trois voies en une seule étape pour obtenir des proportions égales des trois fragments suivants :
i) un fragment de vecteur comprenant un gène marqueur de sélection et une origine de réplication bactérienne à faible copie, dans lequel l'extrémité 5' du fragment de vecteur commence par un premier site d'enzyme de restriction et se termine à l'extrémité 3' du fragment de vecteur par un second site d'enzyme de restriction,
ii) un bras 5' comprenant l'extrémité 5' du génome de l'adénovirus, y compris l'ITR 5', dans lequel l'extrémité 5' du bras 5' commence par un deuxième site d'enzyme de restriction et se termine à l'extrémité 3' du bras 5' par un troisième site d'enzyme de restriction,
iii) un bras 3' comprenant l'extrémité 3' du génome de l'adénovirus, y compris l'ITR 3' et le gène L5, dans lequel l'extrémité 5' du bras 3' commence par un troisième site d'enzyme de restriction et se termine à l'extrémité 3' du bras 3' par un premier site d'enzyme de restriction,
joignant ainsi :
l'extrémité 3' du bras 3' (fragment iii) à l'extrémité 5' du fragment de vecteur (fragment i) au niveau du premier site de l'enzyme de restriction,
l'extrémité 3' du fragment de vecteur (fragment i) à l'extrémité 5' du bras 5' (fragment ii) au niveau du deuxième site d'enzyme de restriction, et
l'extrémité 3' du bras 5' (fragment ii) à l'extrémité 5' du bras 3' (fragment iii) au niveau du troisième site de l'enzyme de restriction,
pour former un vecteur navette circularisé composé d'un premier site d'enzyme de restriction suivi d'un fragment de vecteur suivi d'un deuxième site d'enzyme de restriction suivi d'un bras 5', suivi d'un troisième site d'enzyme de restriction suivi d'un bras 3',
b) introduire au moins un site de restriction unique et/ou un transgène dans le vecteur navette à un endroit donné :
i) entre le gène L5 et un site E3,
ii) entre le gène L5 et un site E4, ou
iii) entre le gène L5 et un site E3, ainsi qu'entre le gène L5 et un site E4.

2. Procédé selon la revendication 1, dans laquelle le bras 5' comprend environ 2,4 à 4,7 kb de l'extrémité 5' d'un génome d'adénovirus, et/ou
le bras 3' comprend environ 3,3 à 4,8 kb de l'extrémité 3' du génome d'un adénovirus.

3. Procédé selon l'une des revendications 1 à 2, dans laquelle le site de restriction unique est choisi indépendamment parmi Fsel, Notl, Sbfl et Sgfl.

4. Procédé selon l'une des revendications 1 à 3, dans laquelle le fragment de vecteur est déphosphorylé.

5. Procédé selon l'une des revendications 1 à 4, dans laquelle le génome de l'adénovirus provient d'un adénovirus capable de se répliquer.

6. Procédé de préparation d'un plasmide, comprenant les étapes a) et b) selon l'une quelconque des revendications 1 à 5, et comprenant en outre une étape c) réalisant une recombinaison homologue dans des cellules électrocompétentes entre le vecteur navette des étapes a) et b) et un génome d'adénovirus pour former un plasmide.

7. Procédé selon la revendication 6, dans laquelle l'étape c) est réalisée dans un rapport de 3,5:1,5.

8. Procédé selon la revendication 6 ou 7, dans laquelle l'étape c) est réalisée dans des cellules BJ5183 électrocompétentes.

9. Procédé selon l'une des revendications 6 à 8, dans laquelle le génome de l'adénovirus est choisi parmi EnAd, OvAd1, OvAd2, Ad3, Ad5 et Ad11.

10. Procédé de génération d'un adénovirus, comprenant la préparation d'un plasmide selon le procédé de l'une des revendications 6 à 9, et comprenant une étape supplémentaire de génération d'un adénovirus.
